(19) Europäisches Patentamt / European Patent Office / Office européen des brevets

(11) EP 1 845 098 A1

(12) **EUROPEAN PATENT APPLICATION**

(43) Date of publication:
**17.10.2007 Bulletin 2007/42**

(21) Application number: **06111899.8**

(22) Date of filing: **29.03.2006**

(51) Int Cl.:
***C07D 487/04*** *(2006.01)* ***A61K 31/5025*** *(2006.01)*
***A61P 25/22*** *(2006.01)* ***A61P 25/08*** *(2006.01)*
***A61P 25/20*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **FERRER INTERNACIONAL, S.A.**
**08028 Barcelona (ES)**

(72) Inventors:
- **Falcó, José Luis**
**08004, Barcelona (ES)**
- **Palomer, Albert**
**08014, Barcelona (ES)**
- **Guglietta, Antonio**
**08750, Molins de Rei (ES)**

(74) Representative: **Barlocci, Anna**
**ZBM Patents**
**Zea, Barlocci & Markvardsen**
**C/ Balmes, 114 - 4°**
**08008 Barcelona (ES)**

# (54) Imidazo[1,2-b]pyridazines, their processes of preparation and their use as GABA receptor ligands

(57) The invention provides novel imidazo[1,2-b]pyridazines of formula (I) and pharmaceutically acceptable salts, polymorphs, hydrates, tautomers, solvates and stereoisomers thereof.

Compounds of formula (I) are useful for treating or preventing diseases associated with $GABA_A$ receptors modulation, anxiety, epilepsy, sleep disorders including insomnia, and for inducing sedation-hypnosis, anesthesia, sleep and muscle relaxation.

The invention also provides synthetic procedures for preparing said compounds and certain intermediates, as well as intermediates themselves.

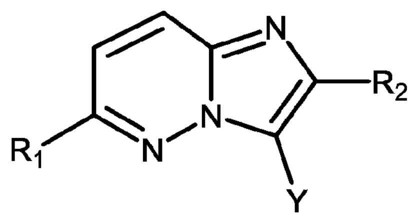

(I)



Printed by Jouve, 75001 PARIS (FR)

## Description

This invention is directed to agents with affinity for $GABA_A$ receptor, specifically to imidazo[1,2-b]pyridazine compounds.

BACKGROUND OF THE INVENTION

$GABA_A$ receptor (y-aminobutyric $acid_A$) is a pentameric protein which forms a membrane ion channel. $GABA_A$ receptor is implicated in the regulation of sedation, anxiety, muscle tone, epileptogenic activity and memory functions. These actions are due to defined subunits of $GABA_A$ receptor, particularly the $\alpha_1$- and $\alpha_2$-subunits.

Sedation is modulated by the $\alpha_1$-subunit. Zolpidem is characterized by a high affinity for the $\alpha_1$-receptors and its sedative and hypnotic action is mediated by these receptors *in vivo.* Similarly, the hypnotic action of zaleplon is also mediated by the $\alpha_1$-receptors.

The anxiolytic action of diazepam is mediated by the enhancement of GABAergic transmission in a population of neurons expressing the $\alpha_2$-receptors. This indicates that the $\alpha_2$-receptors are highly specific targets for the treatment of anxiety.

Muscle relaxation in diazepam is mainly mediated by $\alpha_2$-receptors, since these receptors exhibit a highly specific expression in spinal cord.

The anticonvulsant effect of diazepam is partly due to $\alpha_1$-receptors. In diazepam, a memory-impairing compound, anterograde amnesia is mediated by $\alpha_1$-receptors.

$GABA_A$ receptor and its $\alpha_1$- and $\alpha_2$-subunits have been widely reviewed by H. Möhler et al. (J. Pharmacol. Exp. Ther., 300, 2-8, 2002); H. Möhler et al. (Curr. Opin. Pharmacol., 1, 22-25, 2001); U. Rudolph et al. (Nature, 401, 796-800, 1999); and D.J. Nutt et al. (Br. J. Psychiatry, 179, 390-396, 2001).

Diazepam and other classical benzodiazepines are extensively used as anxiolytic agents, hypnotic agents, anticonvulsants and muscle relaxants.

Their side effects include anterograde amnesia, decrease in motor activity and potentiation of ethanol effects.

In this context, the compounds of this invention are ligands of $\alpha_1$- and $\alpha_2$-$GABA_A$ receptor for their clinical application in sleep disorders, preferably insomnia, anxiety and epilepsy.

Insomnia is a highly prevalent disease. Its chronicity affects 10% of the population and 30% when transitory insomnia is computed as well. Insomnia describes the trouble in getting to sleep or staying asleep and is associated with next-day hangover effects such as weariness, lack of energy, low concentration and irritability. The social and health impact of this complaint is important and results in evident socioeconomic repercussions.

Pharmacological therapy in the management of insomnia firstly included barbiturates and chloral hydrate, but these drugs elicit numerous known adverse effects, for example, overdose toxicity, metabolic induction, and enhanced dependence and tolerance. In addition, they affect the architecture of sleep by decreasing above all the duration and the number of REM sleep stages. Later, benzodiazepines meant an important therapeutic advance because of their lower toxicity, but they still showed serious problems of dependence, muscle relaxation, amnesia and rebound insomnia following discontinuation of medication.

The latest known therapeutic approach has been the introduction of non-benzodiazepine hypnotics, such as pyrrolo[3,4-b]pyrazines (zopiclone), imidazo[1,2-a] pyridines (zolpidem) and, finally, pyrazolo[1,5-a] pyrimidines (zaleplon). Later, two new pyrazolo[1,5-a] pyrimidines, indiplon and ocinaplon, have entered into development, the latter with rather anxiolytic action. All these compounds show a rapid sleep induction and have less next-day hangover effects, lower potential for abuse and lower risk of rebound insomnia than benzodiazepines. The mechanism of action of these compounds is the alosteric activation of $GABA_A$ receptor through its binding to benzodiazepine binding site (C. F. P. George, The Lancet, 358, 1623-1626, 2001). While benzodiazepines are unspecific ligands at $GABA_A$ receptor binding site, zolpidem and zaleplon show a greater selectivity for $\alpha_1$-subunit.

Notwithstanding that, these drugs still affect the architecture of sleep and may induce dependence in long-term treatments.

Some N-imidazo[1,2-b]pyridazin-3-yl-methyl-alkanamides and N-imidazo[1,2-b]pyridazin-3-yl-methyl-benzamides, wherein the phenyl ring from the benzamide group can be optionally substituted, have been disclosed in WO 89/01333,. Related compound N-[[6-chloro-2-(p-tolyl)imidazo[1,2-b]pyridazin-3-yl]methyl]-3-pyridinecarboxamide has been reported in Barlin, G. V. et. al., Australian Journal of Chemistry (1992), 45(4), 731-49.

The compounds of the present invention are structurally related to, but distinct from the compound N,N,6-trimethyl-2-p-tolylimidazo[1,2-a]pyridine-3-acetamide, zolpidem, which is described in US 4,382,938, because of their improved properties as shown in the Detailed Description of the Invention.

Research for new active compounds in the management of insomnia answers an underlying health need, because even recently introduced hypnotics still affect the architecture of sleep and may induce dependence in long-term treatments.

It is therefore desirable to focus on the development of new hypnotic agents with a lower risk of side effects.

SUMMARY OF THE INVENTION

The present invention provides new imidazo[1,2-b]pyridazines which are active versus $GABA_A$ and, particularly, versus its $\alpha_1$- and $\alpha_2$-subunits. Consequently, the compounds of this invention are useful in the treatment and prevention of all those diseases mediated by $GABA_A$ receptor $\alpha_1$- and $\alpha_2$-subunits. Non-limitative examples of such diseases are sleep disorders, preferably insomnia, anxiety and epilepsy. Non-limitative examples of the relevant indications of the compounds of this invention are all those diseases or conditions, such as insomnia or anesthesia, in which an induction of sleep, an induction of sedation or an induction of muscle relaxation are needed.

Thus, the present invention describes a novel class of compounds represented by formula (I):

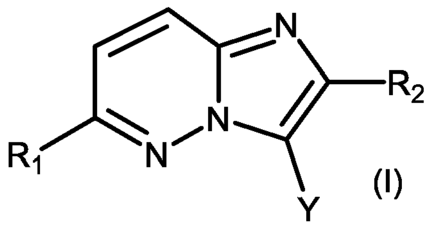

(I)

and pharmaceutically acceptable salts, polymorphs, hydrates, tautomers, solvates and stereoisomers thereof, wherein $R_1$, $R_2$, and Y are defined below, which are ligands of $GABA_A$ receptor.

It is another object of this invention to provide synthetic procedures for preparing the compounds of formula (I) and (II), certain intermediates thereof, as well as intermediates themselves. Novel methods of treating or preventing diseases associated with $GABA_A$ receptors modulation such as anxiety, epilepsy and sleep disorders including insomnia, and for inducing sedation-hypnosis, anesthesia, sleep and muscle relaxation by administering a therapeutically effective amount of said compounds are also within the scope of the invention.

DETAILED DESCRIPTION OF THE INVENTION

The present invention relates to novel imidazo[1,2-b]pyridazine compound of formula (I):

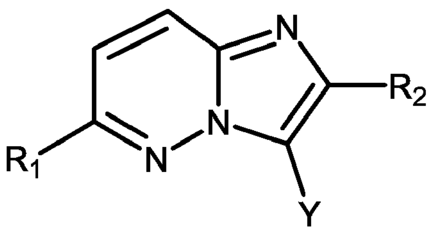

(I)

wherein

$R_1$ and $R_2$ are independently selected from the group consisting of hydrogen, linear or branched alkyl($C_1$-$C_6$), alkenyl ($C_2$-$C_6$), alkynyl($C_2$-$C_6$), cycloalkyl($C_3$-$C_6$), haloalkyl($C_2$-$C_6$), hydroxy, -O-alkyl($C_1$-$C_6$), phenoxy, -S-alkyl($C_1$-$C_6$), phenylthio, halogen, nitro, cyano, amino, alkylamino($C_1$-$C_6$), dialkylamino($C_1$-$C_6$), pyrrolidinyl, morpholinyl, piperidinyl, N-alkyl($C_1$-$C_6$)piperazinyl, phenyl optionally substituted by 1 to 5 Z groups and heteroaryl optionally substituted by 1 to 5 Z groups;

Y is selected from

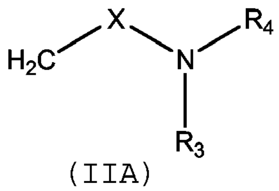
(IIA)

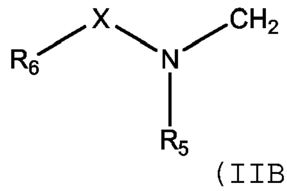
(IIB)

$R_3$ and $R_4$ are independently selected from the group consisting of hydrogen, linear or branched alkyl($C_1$-$C_6$), alkenyl ($C_2$-$C_6$), alkynyl($C_2$-$C_6$), cycloalkyl($C_3$-$C_6$), hydroxyalkyl($C_1$-$C_6$), amino, -NH-alkyl($C_1$-$C_6$), -N-dialkyl($C_1$-$C_6$), pyrrolidinyl, morpholinyl, piperidinyl, -N-alkyl($C_1$-$C_6$)piperazinyl, -N-acyl($C_1$-$C_6$)piperazinyl, phenyl optionally substituted by 1 to 5 Z groups and heteroaryl optionally substituted by 1 to 5 Z groups, or both $R_3$ and $R_4$ can form, together with the nitrogen atom to which they are attached, a 5-6 membered heterocyclic ring optionally substituted by 1 to 5 Z groups, with the proviso that $R_3$ and $R_4$ may not be simultaneously hydrogen;

$R_5$ is selected from the group consisting of hydrogen, linear or branched alkyl($C_1$-$C_6$), cycoalkyl($C_3$-$C_6$), cyloalkyl ($C_3$-$C_6$)alkyl($C_1$-$C_6$), alkenyl($C_2$-$C_6$), alkenyl($C_2$-$C_6$)alkyl($C_1$-$C_6$), alkynyl($C_2$-$C_6$) and alkenyl($C_2$-$C_6$)alkyl($C_1$-$C_6$); $R_6$ is selected from the group consisting of haloalkyl($C_2$-$C_6$), cycloalkyl($C_3$-$C_6$), cycloalkyl($C_3$-$C_6$)alkyl($C_1$-$C_6$), alkynyl ($C_2$-$C_6$)alkyl($C_2$-$C_6$), alkenyl($C_2$-$C_6$)alkyl($C_1$-$C_6$), alkyl($C_1$-$C_6$)-O-alkyl($C_1$-$C_6$), alkyl($C_1$-$C_6$)-NH-alkyl($C_1$-$C_6$), alkyl ($C_1$-$C_6$)-N(dialkyl($C_1$-$C_6$)), -$OR_7$, -$NHR_7$, -$NR_7R_8$, aryl optionally substituted by 1 to 5 Z groups and heteroaryl optionally substituted by 1 to 5 Z groups, with the proviso that $R_6$ may not be 3-pyridyl;

$R_7$ and $R_8$ are independently selected from the group consisting of hydrogen, linear or branched alkyl($C_1$-$C_6$), phenylalkyl($C_1$-$C_6$), haloalkyl($C_2$-$C_6$), cycloalkyl($C_3$-$C_6$), cycloalkyl($C_3$-$C_6$)alkyl($C_1$-$C_6$), alkenyl($C_2$-$C_6$), alkynyl ($C_2$-$C_6$), alkenyl($C_2$-$C_6$)alkyl($C_1$-$C_6$), alkynyl($C_2$-$C_6$)alkyl($C_1$-$C_6$), phenyl optionally substituted by 1 to 5 Z groups and heteroaryl optionally substituted by 1 to 5 Z groups, or both $R_7$ and $R_8$ can form, together with the nitrogen atom to which they are attached, a 5-6 membered heterocyclic ring optionally substituted by 1 to 5 Z groups;

X is selected from CO and $SO_2$;

Z is selected from the group consisting of linear or branched alkyl(C1-C6), alkenyl($C_2$-$C_6$), alkynyl($C_2$-$C_6$), cycloalkyl ($C_3$-$C_6$), haloalkyl($C_2$-$C_6$), hydroxy, - O-alkyl($C_1$-$C_6$), phenoxy, -S-alkyl($C_1$-$C_6$), phenylthio, halogen, nitro, cyano, amino, alkylamino($C_1$-$C_6$) and dialkylamino($C_1$-$C_6$); and pharmaceutically acceptable salts, polymorphs, hydrates, tautomers, solvates and stereoisomers thereof,

with the proviso that when Y is

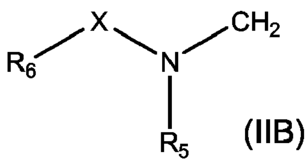
(IIB)

$R_5$ is hydrogen and X is CO, then $R_6$ is different from cycloalkyl ($C_3$-$C_6$) and aryl.

In one embodiment of the invention, the imidazo[1,2-b]pyridazine compound has the following formula:

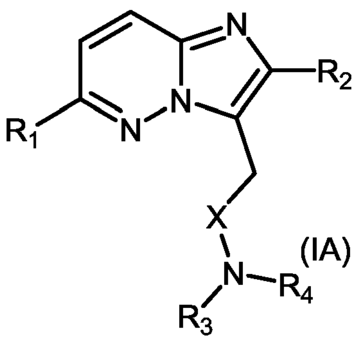
(IA)

In another embodiment of the invention, the imidazo[1,2-b]pyridazine compound has the following formula:

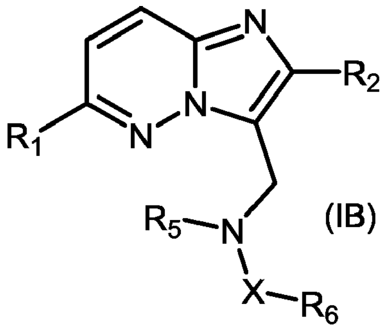

(IB)

Preferably $R_1$ is selected from methyl, chlorine, methoxy, ethoxy, phenylthio or 1-pyrrolidinyl group and $R_2$ is a phenyl group or a phenyl group substituted in *para*- position by methyl, halogen, methoxy, nitro or trifluoromethyl.

Preferably in compounds of formula (IA), X is CO; $R_3$ is selected from the group consisting of hydrogen, linear alkyl($C_1$-$C_6$), phenyl optionally substituted by 1 to 5 Z groups, heteroaryl optionally substituted by 1 to 5 Z groups, amino, -NH-alkyl($C_1$-$C_6$), -N-dialkyl($C_1$-$C_6$), 1-pyrrolidinyl, 4-morpholinyl and 1-piperidinyl; and $R_4$ is selected from the group consisting of hydrogen, linear alkyl($C_1$-$C_6$), phenyl optionally substituted by 1 to 5 Z groups and heteroaryl optionally substituted by 1 to 5 Z groups; or both $R_3$ and $R_4$ can form, together with the nitrogen atom to which they are attached, a 5-6 membered heterocyclic ring optionally substituted by 1 to 5 Z groups; and Z is selected from the group consisting of methyl and methoxy.

Preferably in compounds of formula (IB), X is CO; $R_5$ is selected from the group consisting of hydrogen and linear or branched alkyl ($C_1$-$C_6$); and $R_6$ is 5-isoxazolyl, 2-pyrazinyl, 2-quinoxalyl, 4-pyridyl and 2-thienyl.

Preferably also in compounds of formula (IB), X is CO; $R_5$ is hydrogen; $R_6$ is - $NR_7R_8$; and $R_7$ is selected from the group consisting of is selected from the group consisting of hydrogen, methyl, ethyl, n-propyl, i-propyl, cyclopropyl, cyclopentyl, cyclohexyl, 2-propinyl and phenyl optionally substituted by 1 to 5 Z groups; and Z is selected from the group consisting of methyl, fluorine, chlorine, methoxy, thiomethoxy, acetyl and nitro.

Preferably also in compounds of formula (IB), X is CO; $R_5$ is hydrogen; $R_6$ is - $OR_7$; and $R_7$ is selected from the group consisting of methyl, ethyl, n-propyl, i-propyl, n-butyl,i-butyl and phenyl substituted by 1 to 5 Z groups; and Z is selected from the group consisting of methyl, methoxy and nitro.

Preferably also in compounds of formula (IB), X is $SO_2$; $R_5$ is hydrogen; and $R_6$ is selected from methyl, phenyl substituted by 1 to 5 Z groups, 2-thienyl, 1-methyl-1 H-imidazole-4-yl, 5-bromo-6-chloro-pyridine-3-yl or 3,5-dimethylisoxazole-4-yl; and Z is selected from the group consisting of methyl, chlorine, methoxy and nitro.

The term "pharmaceutically acceptable salt" used herein encompasses any salt formed from organic and inorganic acids, such as hydrobromic, hydrochloric, phosphoric, nitric, sulfuric, acetic, adipic, aspartic, benzenesulfonic, benzoic, citric, ethanesulfonic, formic, fumaric, glutamic, lactic, maleic, malic, malonic, mandelic, methanesulfonic, 1,5-naphthalendisulfonic, oxalic, pivalic, propionic, p-toluenesulfonic, succinic, tartaric acids and the like.

Preferred compounds of formula (I) include:

2-(6-Chloro-2-p-tolyl-imidazo[1,2-b]pyridazin-3-yl)-N,N-diethyl-acetamide;
2-(6-Chloro-2-p-tolyl-imidazo[1,2-b]pyridazin-3-yl)-N,N-dipropyl-acetamide;
N,N-Dibutyl-2-(6-chloro-2-p-tolyl-imidazo[1,2-b]pyridazin-3-yl)-acetamide;
2-(6-Chloro-2-p-tolyl-imidazo[1,2-b]pyridazin-3-yl)-1-piperidin-1-yl-ethanone;
2-(6-Chloro-2-p-tolyl-imidazo[1,2-b]pyridazin-3-yl)-1-morpholin-4-yl-ethanone;
2-(6-Chloro-2-p-tolyl-imidazo[1,2-b]pyridazin-3-yl)-1-pyrrolidin-1-yl-ethanone;
N,N-Diethyl-2-(6-pyrrolidin-1-yl-2-p-tolyl-imidazo[1,2-b]pyridazin-3-yl)-acetamide;
N,N-Diethyl-2-(6-methoxy-2-p-tolyl-imidazo[1,2-b]pyridazin-3-yl)-acetamide;
2-[2-(4-Bromo-phenyl)-6-ethoxy-imidazo[1,2-b]pyridazin-3-yl]-1-morpholin-4-yl-ethanone;
2-[2-(4-Bromo-phenyl)-6-ethoxy-imidazo[1,2-b]pyridazin-3-yl]-1-piperidin-1-yl-ethanone;
2-[2-(4-Bromo-phenyl)-6-ethoxy-imidazo[1,2-b]pyridazin-3-yl]-N,N-dibutylacetamide;
2-[2-(4-Bromo-phenyl)-6-ethoxy-imidazo[1,2-b]pyridazin-3-yl]-N,N-dipropylacetamide;
2-[2-(4-Bromo-phenyl)-6-ethoxy-imidazo[1,2-b]pyridazin-3-yl]-N,N-diethylacetamide;
2-[2-(4-Bromo-phenyl)-6-methyl-imidazo[1,2-b]pyridazin-3-yl]-1-morpholin-4-yl-ethanone;
2-[2-(4-Bromo-phenyl)-6-methyl-imidazo[1,2-b]pyridazin-3-yl]-1-piperidin-1-yl-ethanone;
2-[2-(4-Bromo-phenyl)-6-methyl-imidazo[1,2-b]pyridazin-3-yl]-N,N-dibutylacetamide;
2-[2-(4-Bromo-phenyl)-6-methyl-imidazo[1,2-b]pyridazin-3-yl]-N,N-dipropylacetamide;

2-[2-(4-Bromo-phenyl)-6-methyl-imidazo[1,2-b]pyridazin-3-yl]-N,N-diethylacetamide;
2-[2-(4-Chloro-phenyl)-6-methyl-imidazo[1,2-b]pyridazin-3-yl]-N,N-diethylacetamide;
2-[2-(4-Chloro-phenyl)-6-ethoxy-imidazo[1,2-b]pyridazin-3-yl]-N,N-diethylacetamide;
2-[2-(4-Chloro-phenyl)-6-methyl-imidazo[1,2-b]pyridazin-3-yl]-N,N-dipropylacetamide;
N,N-Dibutyl-2-[2-(4-chloro-phenyl)-6-methyl-imidazo[1,2-b]pyridazin-3-yl]-acetamide;
2-[2-(4-Chloro-phenyl)-6-ethoxy-imidazo[1,2-b]pyridazin-3-yl]-N,N-dipropylacetamide;
N,N-Dibutyl-2-[2-(4-chloro-phenyl)-6-ethoxy-imidazo[1,2-b]pyridazin-3-yl]-acetamide;
N,N-Diethyl-2-(6-methoxy-2-phenyl-imidazo[1,2-b]pyridazin-3-yl)-acetamide;
2-(6-Methoxy-2-phenyl-imidazo[1,2-b]pyridazin-3-yl)-N,N-dipropyl-acetamide;
N,N-Dibutyl-2-(6-methoxy-2-phenyl-imidazo[1,2-b]pyridazin-3-yl)-acetamide;
2-(6-Methoxy-2-phenyl-imidazo[1,2-b]pyridazin-3-yl)-1-morpholin-4-yl-ethanone;
2-(6-Ethoxy-2-phenyl-imidazo[1,2-b]pyridazin-3-yl)-N,N-diethyl-acetamide;
2-(6-Ethoxy-2-phenyl-imidazo[1,2-b]pyridazin-3-yl)-N,N-dipropyl-acetamide;
N,N-Dibutyl-2-(6-ethoxy-2-phenyl-imidazo[1,2-b]pyridazin-3-yl)-acetamide;
2-(6-Ethoxy-2-phenyl-imidazo[1,2-b]pyridazin-3-yl)-1-morpholin-4-yl-ethanone;
2-(6-Ethoxy-2-phenyl-imidazo[1,2-b]pyridazin-3-yl)-1-piperidin-1-yl-ethanone;
N,N-Diethyl-2-(6-methyl-2-phenyl-imidazo[1,2-b]pyridazin-3-yl)-acetamide;
2-(6-Methyl-2-phenyl-imidazo[1,2-b]pyridazin-3-yl)-N,N-dipropyl-acetamide;
N,N-Dibutyl-2-(6-methyl-2-phenyl-imidazo[1,2-b]pyridazin-3-yl)-acetamide;
2-(6-Methyl-2-phenyl-imidazo[1,2-b]pyridazin-3-yl)-1-morpholin-4-yl-ethanone;
2-(6-Methyl-2-phenyl-imidazo[1,2-b]pyridazin-3-yl)-1-piperidin-1-yl-ethanone;
2-[2-(4-Fluoro-phenyl)-6-methyl-imidazo[1,2-b]pyridazin-3-yl]-N,N-dipropylacetamide;
N,N-Dibutyl-2-[2-(4-fluoro-phenyl)-6-methyl-imidazo[1,2-b]pyridazin-3-yl]-acetamide;
2-[2-(4-Fluoro-phenyl)-6-methoxy-imidazo[1,2-b]pyridazin-3-yl]-N,N-dipropylacetamide;
N,N-Dibutyl-2-[2-(4-fluoro-phenyl)-6-methoxy-imidazo[1,2-b]pyridazin-3-yl]-acetamide;
2-[6-Ethoxy-2-(4-fluoro-phenyl)-imidazo[1,2-b]pyridazin-3-yl]-N,N-dipropylacetamide;
N,N-Dibutyl-2-[6-ethoxy-2-(4-fluoro-phenyl)-imidazo[1,2-b]pyridazin-3-yl]-acetamide;
2-[6-Ethoxy-2-(4-fluoro-phenyl)-imidazo[1,2-b]pyridazin-3-yl]-1-morpholin-4-yl-ethanone;
N,N-Diethyl-2-(6-methoxy-2-p-tolyl-imidazo[1,2-b]pyridazin-3-yl)-acetamide;
2-(6-Methoxy-2-p-tolyl-imidazo[1,2-b]pyridazin-3-yl)-N,N-dipropyl-acetamide;
N,N-Dibutyl-2-(6-methoxy-2-p-tolyl-imidazo[1,2-b]pyridazin-3-yl)-acetamide;
2-(6-Methoxy-2-p-tolyl-imidazo[1,2-b]pyridazin-3-yl)-1-piperidin-1-yl-ethanone;
2-(6-Ethoxy-2-p-tolyl-imidazo[1,2-b]pyridazin-3-yl)-N,N-diethyl-acetamide;
2-(6-Ethoxy-2-p-tolyl-imidazo[1,2-b]pyridazin-3-yl)-N,N-dipropyl-acetamide;
N,N-Dibutyl-2-(6-ethoxy-2-p-tolyl-imidazo[1,2-b]pyridazin-3-yl)-acetamide;
2-(6-Ethoxy-2-p-tolyl-imidazo[1,2-b]pyridazin-3-yl)-1-pyrrolidin-1-yl-ethanone;
2-(6-Ethoxy-2-p-tolyl-imidazo[1,2-b]pyridazin-3-yl)-1-morpholin-4-yl-ethanone;
2-(6-Ethoxy-2-p-tolyl-imidazo[1,2-b]pyridazin-3-yl)-1-piperidin-1-yl-ethanone;
N,N-Diethyl-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-N,N-dipropyl-acetamide;
N,N-Dibutyl-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-1-pyrrolidin-1-yl-ethanone;
2-(6-Methyl-2-p-tolyl-imidazo[1,2-b]pyridazin-3-yl)-1-morpholin-4-yl-ethanone;
2-(6-Methyl-2-p-tolyl-imidazo[1,2-b]pyridazin-3-yl)-1-piperidin-1-yl-ethanone;
N,N-Diethyl-2-[2-(4-fluoro-phenyl)-6-methyl-imidazo[1,2-b]pyridazin-3-yl]-acetamide;
2-[2-(4-Fluoro-phenyl)-6-methyl-imidazo[1,2-b]pyridazin-3-yl]-1-piperidin-1-yi-ethanone;
N,N-Diethyl-2-[2-(4-fluoro-phenyl)-6-methoxy-imidazo[1,2-b]pyridazin-3-yl]-acetamide;
2-[2-(4-Fluoro-phenyl)-6-methoxy-imidazo[1 ,2-b]pyridazin-3-yl]-I -piperidin-I - yl-ethanone;
2-[6-Ethoxy-2-(4-fluoro-phenyl)-imidazo[1,2-b]pyridazin-3-yl]-N,N-diethylacetamide;
2-[6-Ethoxy-2-(4-fluoro-phenyl)-imidazo[1,2-b]pyridazin-3-yl]-1-piperidin-1-yl-ethanone;
2-[2-(4-Fluoro-phenyl)-6-methyl-imidazo[1,2-b]pyridazin-3-yl]-1-morpholin-4-yl-ethanone;
2-[2-(4-Fluoro-phenyl)-6-methoxy-imidazo[1,2-b]pyridazin-3-yl]-1-morpholin-4-yl-ethanone;
N,N-Diethyl-2-[2-(4-methoxy-phenyl)-6-methyl-imidazo[1,2-b]pyridazin-3-yl]-acetamide;
2-[2-(4-Methoxy-phenyl)-6-methyl-imidazo[1,2-b]pyridazin-3-yl]-N,N-dipropylacetamide;
N,N-Dibutyl-2-[2-(4-methoxy-phenyl)-6-methyl-imidazo[1,2-b]pyridazin-3-yl]-acetamide;
2-[2-(4-Methoxy-phenyl)-6-methyl-imidazo[1,2-b]pyridazin-3-yl]-1-piperidin-1-yl-ethanone;
2-[2-(4-Methoxy-phenyl)-6-methyl-imidazo[1,2-b]pyridazin-3-yl]-1-morpholin-4-yl-ethanone;
N,N-Diethyl-2-[6-methoxy-2-(4-methoxy-phenyl)-imidazo[1,2-b]pyridazin-3-yl]-acetamide;

2-[6-Methoxy-2-(4-methoxy-phenyl)-imidazo[1,2-b]pyridazin-3-yl]-N,N-dipropyl-acetamide;
N,N-Dibutyl-2-[6-methoxy-2-(4-methoxy-phenyl)-imidazo[1,2-b]pyridazin-3-yl]-acetamide;
2-[6-Methoxy-2-(4-methoxy-phenyl)-imidazo[1,2-b]pyridazin-3-yl]-1-piperidin-1-yl-ethanone;
2-[6-Methoxy-2-(4-methoxy-phenyl)-imidazo[1,2-b]pyridazin-3-yl]-1-morpholin-4-yl-ethanone;
Acetic acid 2-{[2-(6-methyl-2-p-tolyl-imidazo[1,2-b]pyridazin-3-yl)-acetyl]-propyl-amino}-ethyl ester;
1-(3,5-Dimethyl-piperidin-1-yl)-2-(6-methyl-2-p-tolyl-imidazo[1,2-b]pyridazin-3-yl)-ethanone;
N-Cyclopropylmethyl-2-(6-methyl-2-p-tolyl-imidazo[1,2-b]pyridazin-3-yl)-N-propyl-acetamide;
2-(6-Methyl-2-p-tolyl-imidazo[1,2-b]pyridazin-3-yl)-N-thiazol-2-yl-acetamide;
N,N-Diisopropyl-2-(6-methyl-2-p-tolyl-imidazo[1,2-b]pyridazin-3-yl)-acetamide;
N-Cyclohexyl-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-N-phenyl-acetamide;
2-(6-Methyl-2-p-tolyl-imidazo[1,2-b]pyridazin-3-yl)-N-p-tolyl-acetamide;
2-(6-Methyl-2-p-tolyl-imidazo[1,2-b]pyridazin-3-yl)-N-pyridin-2-yl-acetamide;
2-(6-Methyl-2-p-tolyl-imidazo[1,2-b]pyridazin-3-yl)-N-pyridin-2-ylmethylacetamide;
N-(3,5-Dimethyl-isoxazol-4-yl)-2-(6-methyl-2-p-tolyl-imidazo[1,2-b]pyridazin-3-yl)-acetamide;
N-Cyclopentyl-2-(6-methyl-2-p-tolyl-imidazo[1,2-b]pyridazin-3-yl)-acetamide;
N,N-Diallyl-2-(6-methyl-2-p-tolyl-imidazo[1,2-b]pyridazin-3-yl)-acetamide;
N-Cyclopropyl-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-N-quinolin-2-yl-acetamide;
N-(5-Methyl-isoxazol-3-yl)-2-(6-methyl-2-p-tolyl-imidazo[1,2-b]pyridazin-3-yl)-acetamide;
N-(4-Methoxy-phenyl)-2-(6-methyl-2-p-tolyl-imidazo[1,2-b]pyridazin-3-yl)-acetamide;
N-(3-Methyl-isoxazol-5-yl)-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-N-[1,3,4]thiadiazol-2-yl-acetamide;
[2-(4-Fluoro-phenyl)-6-pyrrolidin-1-yl-imidazo[1,2-b]pyridazin-3-yl]-acetic acid hydrazide;
[2-(4-Bromo-phenyl)-6-methyl-imidazo[1,2-b]pyridazin-3-yl]-acetic acid hydrazide;
[2-(4-Methoxy-phenyl)-6-methyl-imidazo[1,2-b]pyridazin-3-yl]-acetic acid hydrazide;
[2-(4-Chloro-phenyl)-6-methyl-imidazo[1,2-b]pyridazin-3-yl]-acetic acid hydrazide;
[2-(4-Fluoro-phenyl)-6-methyl-imidazo[1,2-b]pyridazin-3-yl]-acetic acid hydrazide;
(6-Methyl-2-phenyl-imidazo[1,2-b]pyridazin-3-yl)-acetic acid hydrazide;
2-(6-Methyl-2-p-tolyl-imidazo[1,2-b]pyridazin-3-yl)-N-morpholin-4-yl-acetamide;
2-(6-Methyl-2-p-tolyl-imidazo[1,2-b]pyridazin-3-yl)-N-piperidin-1-yl-acetamide;
(6-Methyl-2-p-tolyl-imidazo[1,2-b]pyridazin-3-yl)-acetic acid N',N'-dimethylhydrazide;
Thiophene-2-carboxylic acid (6-chloro-2-p-tolyl-imidazo[1,2-b]pyridazin-3-ylmethyl)-amide;
N-(6-Chloro-2-p-tolyl-imidazo[1,2-b]pyridazin-3-ylmethyl)-isonicotinamide;
Quinoxaline-2-carboxylic acid (6-chloro-2-p-tolyl-imidazo[1,2-b]pyridazin-3-ylmethyl)-amide;
Pyrazine-2-carboxylic acid (6-chloro-2-p-tolyl-imidazo[1,2-b]pyridazin-3-ylmethyl)-amide;
Isoxazole-5-carboxylic acid (6-chloro-2-p-tolyl-imidazo[1,2-b]pyridazin-3-ylmethyl)-amide;
Thiophene-2-carboxylic acid (6-phenylsulfanyl-2-p-tolyl-imidazo[1,2-b]pyridazin-3-ylmethyl)-amide;
Isoxazole-5-carboxylic acid (6-phenylsulfanyl-2-p-tolyl-imidazo[1,2-b]pyridazin-3-ylmethyl)-amide;
Pyrazine-2-carboxylic acid (6-phenylsulfanyl-2-p-tolyl-imidazo[1,2-b]pyridazin-3-ylmethyl)-amide;
Quinoxaline-2-carboxylic acid (6-phenylsulfanyl-2-p-tolyl-imidazo[1,2-b]pyridazin-3-ylmethyl)-amide;
N-(6-Phenylsulfanyl-2-p-tolyl-imidazo[1,2-b]pyridazin-3-ylmethyl)-isonicotinamide;
N-(2-p-Tolyl-imidazo[1,2-b]pyridazin-3-ylmethyl)-acetamide;
N-(6-Pyrrolidin-1-yl-2-p-tolyl-imidazo[1,2-b]pyridazin-3-ylmethyl)-acetamide;
N-(6-Piperidin-1-yl-2-p-tolyl-imidazo[1,2-b]pyridazin-3-ylmethyl)-acetamide;
N-(6-Morpholin-4-yl-2-p-tolyl-imidazo[1,2-b]pyridazin-3-ylmethyl)-acetamide;
N-[6-(4-Methyl-piperazin-1-yl)-2-p-tolyl-imidazo[1,2-b]pyridazin-3-ylmethyl]-acetamide;
N-[2-(4-Bromo-phenyl)-6-pyrrolidin-1-yl-imidazo[1,2-b]pyridazin-3-ylmethyl]-acetamide;
N-(2-Phenyl-6-pyrrolidin-1-yl-imidazo[1,2-b]pyridazin-3-ylmethyl)-acetamide;
N-[2-(4-Fluoro-phenyl)-6-pyrrolidin-1-yl-imidazo[1,2-b]pyridazin-3-ylmethyl]-acetamide;
N-(2-Furan-2-yl-6-pyrrol idin-1-yl-imidazo[1,2-b]pyridazi n-3-ylmethyl)-acetamide;
N-[2-(4-Methoxy-phenyl)-6-pyrrolidin-1-yl-imidazo[1,2-b]pyridazin-3-ylmethyl]-acetamide;
N-[2-(4-Nitro-phenyl)-6-pyrrolidin-1-yl-imidazo[1,2-b]pyridazin-3-ylmethyl]-acetamide;
N-[6-Pyrrolidin-1-yl-2-(4-trifluoromethyl-phenyl)-imidazo[1,2-b]pyridazin-3-ylmethyl]-acetamide;
Pyrazine-2-carboxylic acid (6-methoxy-2-phenyl-imidazo[1,2-b]pyridazin-3-ylmethyl)-amide;
Isoxazole-5-carboxylic acid (6-methoxy-2-phenyl-imidazo[1,2-b]pyridazin-3-ylmethyl)-amide;
Pyrazine-2-carboxylic acid (2-phenyl-6-pyrrolidin-1-yl-imidazo[1,2-b]pyridazin-3-ylmethyl)-amide;
Isoxazole-5-carboxylic acid (2-phenyl-6-pyrrolidin-1-yl-imidazo[1,2-b]pyridazin-3-ylmethyl)-amide;

N-(6-Phenyl-2-p-tolyl-imidazo[1,2-b]pyridazin-3-ylmethyl)-acetamide;
Pyrazine-2-carboxylic acid [2-(4-fluoro-phenyl)-6-methoxy-imidazo[1,2-b]pyridazin-3-ylmethyl]-amide;
Isoxazole-5-carboxylic acid [2-(4-fluoro-phenyl)-6-methoxy-imidazo[1,2-b]pyridazin-3-ylmethyl]-amide;
Isoxazole-5-carboxylic acid [2-(4-chloro-phenyl)-6-methoxy-imidazo[1,2-b]pyridazin-3-ylmethyl]-amide;
Pyrazine-2-carboxylic acid [2-(4-bromo-phenyl)-6-methoxy-imidazo[1,2-b]pyridazin-3-ylmethyl]-amide;
Pyrazine-2-carboxylic acid [2-(4-bromo-phenyl)-6-ethoxy-imidazo[1,2-b]pyridazin-3-ylmethyl]-amide;
Pyrazine-2-carboxylic acid [2-(4-chloro-phenyl)-6-pyrrolidin-1-yl-imidazo[1,2-b]pyridazin-3-ylmethyl]-amide;
Pyrazine-2-carboxylic acid [2-(4-chloro-phenyl)-6-methoxy-imidazo[1,2-b]pyridazin-3-ylmethyl]-amide;
1-(6-Chloro-2-p-tolyl-imidazo[1,2-b]pyridazin-3-ylmethyl)-3-cyclopentyl-urea;
1-(4-Acetyl-phenyl)-3-(6-chloro-2-p-tolyl-imidazo[1,2-b]pyridazin-3-ylmethyl)-urea;
1-(6-Chloro-2-p-tolyl-imidazo[1,2-b]pyridazin-3-ylmethyl)-3-(4-nitro-phenyl)-urea;
1-(4-Chloro-phenyl)-3-(6-chloro-2-p-tolyl-imidazo[1,2-b]pyridazin-3-ylmethyl)-urea;
1-(6-Chloro-2-p-tolyl-imidazo[1,2-b]pyridazin-3-ylmethyl)-3-(4-fluoro-phenyl)-urea;
1-(6-Chloro-2-p-tolyl-imidazo[1,2-b]pyridazin-3-ylmethyl)-3-p-tolyl-urea;
1-(6-Chloro-2-p-tolyl-imidazo[1,2-b]pyridazin-3-ylmethyl)-3-(4-methylsulfanylphenyl)-urea;
1-(6-Chloro-2-p-tolyl-imidazo[1,2-b]pyridazin-3-ylmethyl)-3-(4-methoxy-phenyl)-urea;
1-(6-Chloro-2-p-tolyl-imidazo[1,2-b]pyridazin-3-ylmethyl)-3-phenyl-urea;
1-(6-Chloro-2-p-tolyl-imidazo[1,2-b]pyridazin-3-ylmethyl)-3-isopropyl-urea;
1-(6-Chloro-2-p-tolyl-imidazo[1,2-b]pyridazin-3-ylmethyl)-3-ethyl-urea;
1-Ethyl-3-(6-phenylsulfanyl-2-p-tolyl-imidazo[1,2-b]pyridazin-3-ylmethyl)-urea;
1-Phenyl-3-(6-phenylsulfanyl-2-p-tolyl-imidazo[1,2-b]pyridazin-3-ylmethyl)-urea;
1-(4-Fluoro-phenyl)-3-(6-phenylsulfanyl-2-p-tolyl-imidazo[1,2-b]pyridazin-3-ylmethyl)-urea;
1-Cyclopentyl-3-(6-phenylsulfanyl-2-p-tolyl-imidazo[1,2-b]pyridazin-3-ylmethyl)-urea;
1-isopropyl-3-(6-phenysulfanyl-2-p-toly-imidazo[1,2-b]pyridazin-3-ylmethyl)-urea;
1-(4-Acetyl-phenyl)-3-(6-phenylsulfanyl-2-p-tolyl-imidazo[1,2-b]pyridazin-3-ylmethyl)-urea;
1-Ethyl-3-(6-methoxy-2-phenyl-imidazo[1,2-b]pyridazin-3-ylmethyl)-urea;
1-Ethyl-3-[2-(4-fluoro-phenyl)-6-methoxy-imidazo[1,2-b]pyridazin-3-ylmethyl]-urea;
1-[2-(4-Chloro-phenyl)-6-methoxy-imidazo[1,2-b]pyridazin-3-ylmethyl]-3-ethylurea;
1-[2-(4-Bromo-phenyl)-6-methoxy-imidazo[1,2-b]pyridazin-3-ylmethyl]-3-ethylurea;
1-[2-(4-Bromo-phenyl)-6-ethoxy-imidazo[1,2-b]pyridazin-3-ylmethyl]-3-ethylurea;
1-[2-(4-Chloro-phenyl)-6-pyrrolidin-1-yl-imidazo[1,2-b]pyridazin-3-ylmethyl]-3-ethyl-urea;
(6-Chloro-2-p-tolyl-imidazo[1,2-b]pyridazin-3-ylmethyl)-carbamic acid ethyl ester;
(6-Chloro-2-p-tolyl-imidazo[1,2-b]pyridazin-3-ylmethyl)-carbamic acid prop-2-ynyl ester;
(6-Chloro-2-p-tolyl-imidazo[1,2-b]pyridazin-3-ylmethyl)-carbamic acid 2,2,2-trichloro-ethyl ester;
(6-Chloro-2-p-tolyl-imidazo[1,2-b]pyridazin-3-ylmethyl)-carbamic acid phenyl ester;
(6-Chloro-2-p-tolyl-imidazo[1,2-b]pyridazin-3-ylmethyl)-carbamic acid p-tolyl ester;
(6-Chloro-2-p-tolyl-imidazo[1,2-b]pyridazin-3-ylmethyl)-carbamic acid 4-methoxy-phenyl ester;
(6-Chloro-2-p-tolyl-imidazo[1,2-b]pyridazin-3-ylmethyl)-carbamic acid 4-nitro-phenyl ester;
(6-Chloro-2-p-tolyl-imidazo[1,2-b]pyridazin-3-ylmethyl)-carbamic acid benzyl ester;
(6-Chloro-2-p-tolyl-imidazo[1,2-b]pyridazin-3-ylmethyl)-carbamic acid methyl ester;
(6-Chloro-2-p-tolyl-imidazo[1,2-b]pyridazin-3-ylmethyl)-carbamic acid isobutyl ester;
(6-Chloro-2-p-tolyl-imidazo[1,2-b]pyridazin-3-ylmethyl)-carbamic acid butyl ester;
(6-Phenylsulfanyl-2-p-tolyl-imidazo[1,2-b]pyridazin-3-ylmethyl)-carbamic acid ethyl ester;
(6-Phenylsulfanyl-2-p-tolyl-imidazo[1,2-b]pyridazin-3-ylmethyl)-carbamic acid methyl ester;
(6-Phenylsulfanyl-2-p-tolyl-imidazo[1,2-b]pyridazin-3-ylmethyl)-carbamic acid phenyl ester;
(6-Phenylsulfanyl-2-p-tolyl-imidazo[1,2-b]pyridazin-3-ylmethyl)-carbamic acid p-tolyl ester;
(6-Phenylsulfanyl-2-p-tolyl-imidazo[1,2-b]pyridazin-3-ylmethyl)-carbamic acid 4-methoxy-phenyl ester;
(6-Phenylsulfanyl-2-p-tolyl-imidazo[1,2-b]pyridazin-3-ylmethyl)-carbamic acid benzyl ester;
(6-Phenylsulfanyl-2-p-tolyl-imidazo[1,2-b]pyridazin-3-ylmethyl)-carbamic acid isopropyl ester;
(6-Phenylsulfanyl-2-p-tolyl-imidazo[1,2-b]pyridazin-3-ylmethyl)-carbamic acid butyl ester;
(6-Phenylsulfanyl-2-p-tolyl-imidazo[1,2-b]pyridazin-3-ylmethyl)-carbamic acid isobutyl ester;
(6-Methoxy-2-phenyl-imidazo[1,2-b]pyridazin-3-ylmethyl)-carbamic acid isobutyl ester;
(6-Methoxy-2-phenyl-imidazo[1,2-b]pyridazin-3-ylmethyl)-carbamic acid ethyl ester;
(6-Methoxy-2-phenyl-imidazo[1,2-b]pyridazin-3-ylmethyl)-carbamic acid methyl ester;
(2-Phenyl-6-pyrrolidin-1-yl-imidazo[1,2-b]pyridazin-3-ylmethyl)-carbamic acid ethyl ester;
[2-(4-Fluoro-phenyl)-6-methoxy-imidazo[1,2-b]pyridazin-3-ylmethyl]-carbamic acid isobutyl ester;
[2-(4-Fluoro-phenyl)-6-methoxy-imidazo[1,2-b]pyridazin-3-ylmethyl]-carbamic acid ethyl ester;
[2-(4-Fluoro-phenyl)-6-methoxy-imidazo[1,2-b]pyridazin-3-ylmethyl]-carbamic acid methyl ester;

[2-(4-Chloro-phenyl)-6-methoxy-imidazo[1,2-b]pyridazin-3-ylmethyl]-carbamic acid methyl ester;
[2-(4-Chloro-phenyl)-6-methoxy-imidazo[1,2-b]pyridazin-3-ylmethyl]-carbamic acid isobutyl ester;
[2-(4-Chloro-phenyl)-6-methoxy-imidazo[1,2-b]pyridazin-3-ylmethyl]-carbamic acid ethyl ester;
[2-(4-Bromo-phenyl)-6-methoxy-imidazo[1,2-b]pyridazin-3-ylmethyl]-carbamic acid isobutyl ester;
[2-(4-Bromo-phenyl)-6-methoxy-imidazo[1,2-b]pyridazin-3-ylmethyl]-carbamic acid ethyl ester;
[2-(4-Bromo-phenyl)-6-methoxy-imidazo[1,2-b]pyridazin-3-ylmethyl]-carbamic acid methyl ester;
[2-(4-Bromo-phenyl)-6-ethoxy-imidazo[1,2-b]pyridazin-3-ylmethyl]-carbamic acid isobutyl ester;
[2-(4-Bromo-phenyl)-6-ethoxy-imidazo[1,2-b]pyridazin-3-ylmethyl]-carbamic acid ethyl ester;
[2-(4-Bromo-phenyl)-6-ethoxy-imidazo[1,2-b]pyridazin-3-ylmethyl]-carbamic acid methyl ester;
[2-(4-Chloro-phenyl)-6-pyrrolidin-1-yl-imidazo[1,2-b]pyridazin-3-ylmethyl]-carbamic acid methyl ester;
[2-(4-Chloro-phenyl)-6-pyrrolidin-1-yl-imidazo[1,2-b]pyridazin-3-ylmethyl]-carbamic acid ethyl ester;
[2-(4-Chloro-phenyl)-6-pyrrolidin-1-yl-imidazo[1,2-b]pyridazin-3-ylmethyl]-carbamic acid isobutyl ester;
N-(6-Phenylsulfanyl-2-p-tolyl-imidazo[1,2-b]pyridazin-3-ylmethyl)-methanesulfonamide;
N-(6-Phenylsulfanyl-2-p-tolyl-imidazo[1,2-b]pyridazin-3-ylmethyl)-benzenesulfonamide;
4-Methyl-N-(6-phenylsulfanyl-2-p-tolyl-imidazo[1,2-b]pyridazin-3-ylmethyl)-benzenesulfonamide;
4-Methoxy-N-(6-phenylsulfanyl-2-p-tolyl-imidazo[1,2-b]pyridazin-3-ylmethyl)-benzenesulfonamide;
4-Chloro-N-(6-phenylsulfanyl-2-p-tolyl-imidazo[1,2-b]pyridazin-3-ylmethyl)-benzenesulfonamide;
4-Nitro-N-(6-phenylsulfanyl-2-p-tolyl-imidazo[1,2-b]pyridazin-3-ylmethyl)-benzenesulfonamide;
Thiophene-2-sulfonic acid (6-phenylsulfanyl-2-p-tolyl-imidazo[1,2-b]pyridazin-3-ylmethyl)-amide;
1-Methyl-1 H-imidazole-4-sulfonic acid (6-phenylsulfanyl-2-p-tolyl-imidazo[1,2-b]pyridazin-3-ylmethyl)-amide;
5-Bromo-6-chloro-pyridine-3-sulfonic acid (6-phenylsulfanyl-2-p-tolylimidazo[1,2-b]pyridazin-3-ylmethyl)-amide; and
3,5-Dimethyl-isoxazole-4-sulfonic acid (6-phenylsulfanyl-2-p-tolyl-imidazo[1,2-b]pyridazin-3-ylmethyl)-amide.

Another aspect of the present invention is to provide a process for preparing the compounds of formulas (IA) and (IB), as well as certain imidazo[1,2-b]pyridazine intermediates themselves such as the compounds of formulas (IV) and (V):

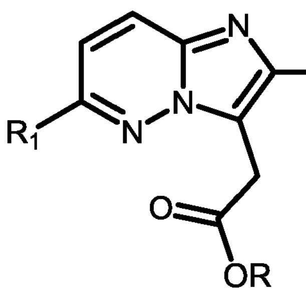

(IV)

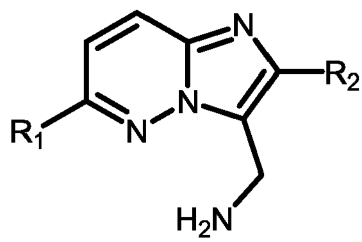

(V)

wherein R is a linear or branched alkyl($C_1$-$C_6$), and $R_1$ and $R_2$ are as defined above, as well as pharmaceutically acceptable salts thereof, with the proviso that in (V) $R_1$ may not be chlorine when $R_2$ is hydrogen.

Preferably, in the intermediates of formula (IV), R is methyl, $R_1$ is a methyl, chlorine, methoxy, ethoxy, thiophenoxy or 1-pyrrolidinyl group and $R_2$ is a phenyl group or a phenyl group substituted in *para-* position by methyl, halogen, methoxy, nitro or trifluoromethyl.

Preferably, in the intermediates of formula (V), $R_1$ is a methyl, chlorine, methoxy, ethoxy, thiophenoxy or 1-pyrrolidinyl group and $R_2$ is a phenyl group or a phenyl group substituted in *para-* position by methyl, halogen, methoxy, nitro or trifluoromethyl.

Another aspect of the present invention is to provide a process for preparing the intermediates of formula (V).

The compounds of general formula (I), when X is CO, can be obtained following the synthetic strategy showed in Scheme 1.

Scheme 1

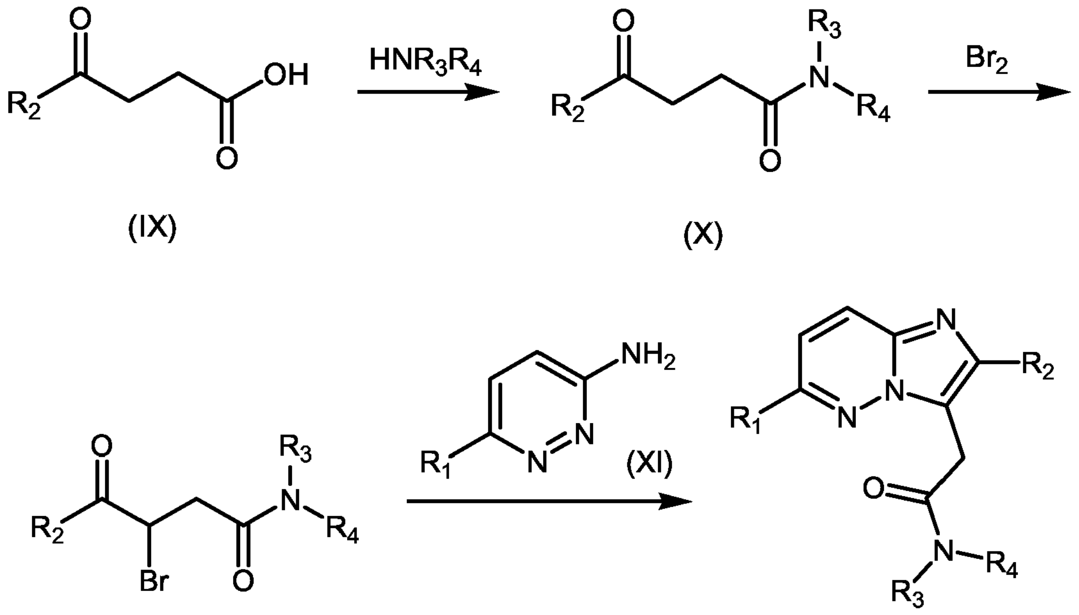


(IX) (X) (XI) (III, X = CO) (I, X = CO)

Starting from ketoacids (IX), ketoamides (X) can be obtained by using conventional coupling conditions. These ketoamides (X) can be brominated in α-position of the reacting carbonyl group with bromine in acetic acid, to yield the bromoketoamides (III). Finally, cyclization of aminopyridazines (XI) in acetonitrile at reflux affords the imidazopyridazines (I, X = CO).

On the other hand, if $R_3$ or $R_4$ are optionally substituted amino groups, then the molecule obtained is not an amide but an hydrazide. The synthetic pathway has to be slightly modified for that proposal (Scheme 2).

Scheme 2

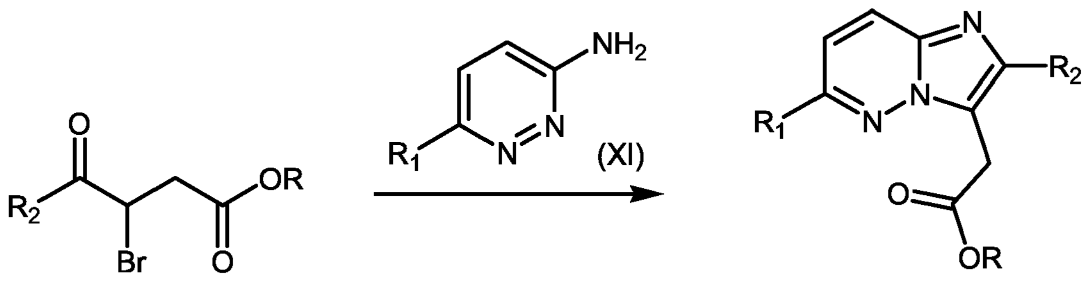

(XI) (XIII) (IV)

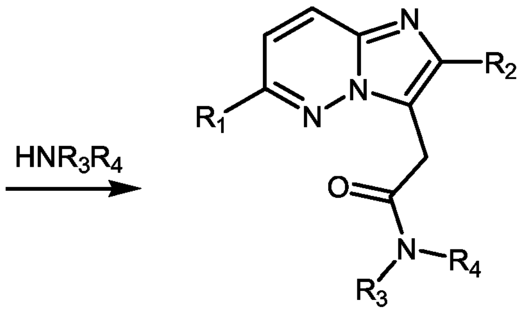


(I, X = CO)

A Fischer esterification of the same ketoacid (IX) is carried out with an alcohol ROH to afford the corresponding ester (XII). This ester is brominated under similar conditions as the amide (X) described above, to yield bromoketoesters (XIII). A cyclization with aminopyridazines (XI) allows the preparation of the imidazopyridazines (IV) substituted with an ester group. Finally, acylic substitution by using a substituted hydrazine in a suitable solvent at reflux provides the corresponding hydrazides (I, X = CO, $R_3$ or $R_4$ are optionally substituted amino groups). Suitable solvents to be used in this reaction are selected preferably from linear or branched alkanols ($C_1$-$C_6$), more preferably methanol, or mixtures thereof.

The compounds of general formula (II) can be obtained following the synthetic strategy showed in Scheme 3.

Scheme 3

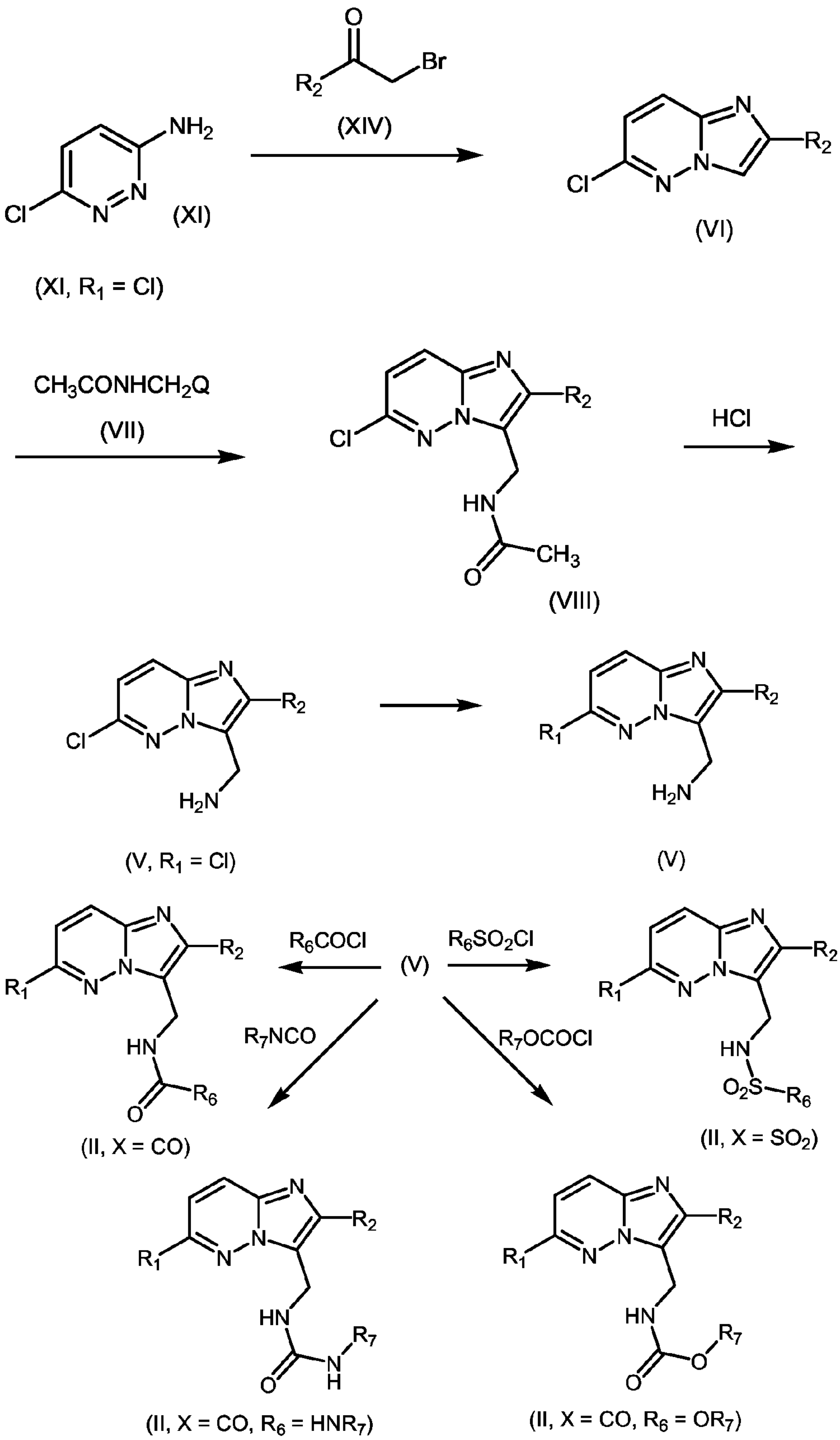


(XI)

(XI, $R_1$ = Cl)

(VI)

(VIII)

(V, $R_1$ = Cl)

(V)

(II, X = CO)

(II, X = $SO_2$)

(II, X = CO, $R_6$ = $HNR_7$)

(II, X = CO, $R_6$ = $OR_7$)

In the acetamide intermediate compounds (VII), Q is selected from the group consisting of-OH, -Oalkyl($C_1$-$C_3$), -$N^+$(alkyl($C_1$-$C_3$))$_3Cl^-$, -$N^+$(alkyl($C_1$-$C_3$))$_3Br^-$ and -$N^+$(alkyl($C_1$-$C_3$))$_3I^-$. Preferably Q is -OH.

The preparation of imidazopyridazine scaffold is made by reacting a 2-bromoacetophenone (XIV) and an aminopyridazine, at reflux in a suitable solvent selected preferably from linear or branched alkanols ($C_1$-$C_6$), more preferably ethanol, or mixtures thereof. Then, the synthetic strategy is based in the utility of the position 3 of the ring. The reaction between (VI) and N-methanolacetamide in acetic acid, using $H_2SO_4$ as catalyst, at reflux, yields the acetamide (VIII). This amide is hydrolized in a medium constituted by HCl and a suitable solvent selected preferably from linear or branched alkanols ($C_1$-$C_6$), more preferably ethanol, or mixtures thereof, to the corresponding amine (V, $R_1$ = Cl). Finally, chlorine displacement in amine (V, $R_1$ = Cl) by an $R_1$ group through aromatic substitution affords the substituted amine (V).

The amine compounds (V) offer the versatility to be converted into amides (II, X = CO), ureas (II, X = CO, $R_6$ = $HNR_7$), carbamates (II, X = CO, $R_6$ = $OR_7$) or sulfonamides (II, X = $SO_2$) as shown in Scheme 3. These reactions are performed under conventional coupling conditions.

The compounds of the present invention or their pharmaceutically acceptable salts, polymorphs, hydrates, tautomers, solvates and stereoisomers can be used for the preparation of a medicament for treating or preventing diseases associated with $GABA_A$ receptor modulation in a human or non-human mammal. More specifically, diseases associated with $GABA_A$ receptor modulation comprise diseases associated with $\alpha_1$-$GABA_A$ receptor modulation and/or $\alpha_2$-$GABA_A$ receptor modulation. A non-limitative list of such diseases comprises anxiety, epilepsy, sleep disorders, including insomnia, and the like.

Another embodiment of the present invention is to provide the use of a compound of formula (I) or a pharmaceutically acceptable salt, polymorph, hydrate, tautomer, solvate and stereoisomer thereof for the preparation of a medicament for treating or preventing anxiety in a human or non-human mammal.

Another embodiment of the present invention is to provide the use of a compound of formula (I) or a pharmaceutically acceptable salt, polymorph, hydrate, tautomer, solvate or stereoisomers thereof for the preparation of a medicament for treating or preventing epilepsy in a human or non-human mammal in need thereof.

Another embodiment of the present invention is to provide the use of a compound of formula (I) or a pharmaceutically acceptable salt, polymorph, hydrate, tautomer, solvate or stereoisomer thereof for the preparation of a medicament for treating or preventing sleep disorders in a human or non-human mammal in need thereof.

Another embodiment of the present invention is to provide the use of a compound of formula (I) or a pharmaceutically acceptable salt, polymorph, hydrate, tautomer, solvate or stereoisomer thereof for the preparation of a medicament for treating or preventing insomnia in a human or non-human mammal in need thereof.

Another embodiment of the present invention is to provide the use of a compound of formula (I) or a pharmaceutically acceptable salt, polymorph, hydrate, tautomer, solvate or stereoisomer thereof for the preparation of a medicament for inducing sedation-hypnosis in a human or non-human mammal in need thereof.

Another embodiment of the present invention is to provide the use of a compound of formula (I) or a pharmaceutically acceptable salt, polymorph, hydrate, tautomer, solvate or stereoisomer thereof for the preparation of a medicament for inducing anesthesia in a human or non-human mammal in need thereof.

Another embodiment of the present invention is to provide the use of a compound of formula (I) or a pharmaceutically acceptable salt, polymorph, hydrate, tautomer, solvate or stereoisomer thereof for the preparation of a medicament for modulating the necessary time to induce sleep and its duration in a human or non-human mammal in need thereof.

Another embodiment of the present invention is to provide the use of a compound of formula (I) or a pharmaceutically acceptable salt, polymorph, hydrate, tautomer, solvate or stereoisomer thereof for the preparation of a medicament for inducing muscle relaxation in a human or non-human mammal in need thereof.

The present invention also relates to a method of treatment or prevention of a human or non-human mammal suffering from diseases associated with $GABA_A$ receptor modulation in a human or non-human mammal, which comprises administering to said human or non-human mammal in need thereof a therapeutically effective amount of a compound of formula (I) or formula (II) or pharmaceutically acceptable salts, polymorphs, hydrates, tautomers, solvates and stereoisomers thereof, together with pharmaceutically acceptable diluents or carriers. More specifically, diseases associated with $GABA_A$ receptor modulation comprise diseases associated with $\alpha_1$-$GABA_A$ receptor modulation and/or $\alpha_2$-$GABA_A$ receptor modulation. A non-limitative list of such diseases comprises anxiety, epilepsy, sleep disorders, including insomnia, and the like.

As used herein, the term "mammal" shall refer to the Mammalian class of higher vertebrates. The term "mammal" includes, but is not limited to, a human.

Another embodiment of the present invention is to provide a pharmaceutical composition containing a compound of formula (I) or pharmaceutically acceptable salts, polymorphs, hydrates, tautomers, solvates and stereoisomers thereof, in association with therapeutically inert carriers.

The compositions include those suitable for oral, rectal and parenteral (including subcutaneous, intramuscular,

and intravenous) administration, although the most suitable route will depend on the nature and severity of the condition being treated. The most preferred route of the present invention is the oral route. The compositions may be conveniently presented in unit dosage form, and prepared by any of the methods well known in the art of pharmacy.

The active compound can be combined with a pharmaceutical carrier according to conventional pharmaceutical compounding techniques. The carrier may take a wide variety of forms depending on the form of the preparation desired for administration, e.g. oral or parenteral (including intravenous injections or infusions). In preparing the compositions for oral dosage form any of the usual pharmaceutical media may be employed. Usual pharmaceutical media include, for example, water, glycols, oils, alcohols, flavoring agents, preservatives, coloring agents, and the like in the case of oral liquid preparations (such as for example, suspensions, solutions, emulsions and elixirs); aerosols; or carriers such as starches, sugars, microcrystalline cellulose, diluents, granulating agents, lubricants, binders, disintegrating agents and the like, in the case of oral solid preparations (such as for example, powders, capsules, and tablets) with the oral solid preparations being preferred over the oral liquid preparations.

Because of their ease of administration, tablets and capsules represent the most advantageous oral dosage unit form, in which case solid pharmaceutical carriers are employed. If desired, tablets may be coated by standard aqueous or non-aqueous techniques.

A suitable dosage range for use is from about 0.01 mg to about 100.00 mg total daily dose, given as a once daily administration or in divided doses if required.

The compounds of the present invention have a high affinity for $\alpha_1$- and $\alpha_2$-$GABA_A$ receptors. These *in vitro* results are consistent with those *in vivo* results obtained in sedation-hypnosis tests.

In accordance with the results obtained, certain compounds of the present invention have evidenced pharmacological activity both *in vitro* and *in vivo,* which has been similar to or higher than that of prior-art compound zolpidem. All these results support their use in diseases or conditions modulated by $\alpha_1$- and $\alpha_2$-$GABA_A$ receptors, such as insomnia or anesthesia, in which an induction of sleep, an induction of sedation or an induction of muscle relaxation are needed.

The pharmacological activity of the compounds of the present invention has been determined as shown below.

a) Ligand-binding assays. Determination of the affinity of test compounds for $\alpha_1$- and $\alpha_2$-$GABA_A$ receptor

Male Sprague-Dawley rats weighing 200-250 g at the time of experiment were used. After decapitation of the animal, the cerebellum (tissue that mostly contains $\alpha_1$-$GABA_A$ receptor) and spinal cord (tissue that mostly contains $\alpha_2$-$GABA_A$ receptor) were removed. The membranes were prepared according to the method by J. Lameh et al. (Prog. Neuro-Psychopharmacol. Biol. Psychiatry, 24, 979-991, 2000) and H. Noguchi et al. (Eur J Pharm, 434, 21-28, 2002) with slight modifications. Once the tissues weighed, they were suspended in 50 mM Tris·HCl (pH 7.4), 1:40 (v/v), or sucrose 0.32 M in the case of spinal cord, homogenized and then centrifuged at 20000 g for 10 minutes at 7°C. The resulting pellet was resuspended under the same conditions and centrifuged again. The pellet was finally resuspended on a minimum volume and kept at -80°C overnight. On the next day, the process was repeated until the final pellet was resuspended at a ratio of 1:10 (v/v)in the case of cerebellum and at a ratio of 1:5 (v/v) in the case of spinal cord.

Affinity was determined by competitive tests using radiolabeled flumazenil as ligand. The tests were performed according to the methods described by S. Arbilla et al. (Eur. J. Pharmacol., 130, 257-263, 1986); and Y. Wu et al. (Eur. J. Pharmacol., 278, 125-132, 1995) using 96-well microtiter plates. The membranes containing the study receptors, flumazenil (radiolabeling at a final concentration of 1 nM) and ascending concentrations of test compounds (in a total volume of 230 μl in 50 mM [pH 7.4] Tris·HCl buffer) were incubated. Simultaneously, the membranes were only incubated with the radiolabeled flumazenil (total binding, 100%) and in the presence of an elevated concentration of unradiolabeled flumazenil (non-specific binding, % estimation of radiolabeled ligand). The reactions started on adding the radiolabeled ligand followed by incubation for 60 minutes at 4°C. At the end of the incubation period, 200 μl of reaction were transferred to a multiscreen plate (Millipore) and filtered using a vacuum manifold and then washed three times with cold test buffer. The multiscreen plates were equipped with a GF/B filter that retained the membranes containing the receptors and the radiolabeled ligand which had been bound to the receptors. After washing, the plates were left till dry. Once dried, scintillation liquid was added and left under stirring overnight. The next day the plates were counted using a Perkin-Elmer Microbeta scintillation counter.

For analysis of the results the percentage of specific binding for every concentration of test compound was calculated as follows:

$$\% \text{ specific binding} = (X\text{-}N/T\text{-}N) \times 100$$

where,

X: amount of bound ligand for every concentration of compound.
T: total binding, maximum amount bound to the radiolabeled ligand.
N: non-specific binding, amount of radiolabeled ligand bound in a non-specific way irrespective of the receptor used.

Every concentrations of compound were tested in triplicate and their mean values were used to determine the experimental values of % specific binding versus the concentration of compound. Affinity data are expressed as % inhibition at $10^{-5}$M and $10^{-7}$M concentrations. The results of these tests are given in Tables 1 and 2.

Table 1. Affinity for the $\alpha_1$-subunit of the $GABA_A$ receptor

| Compound | % inhib $10^{-5}$M | % inhib $10^{-7}$M |
|---|---|---|
| Example 1 | 100.1 | 98.2 |
| Example 2 | 100.3 | 99.6 |
| Example 3 | 100.2 | 99.4 |
| Example 4 | 99.9 | 98.3 |
| Example 5 | 100.3 | 97.5 |
| Example 6 | 100.0 | 97.4 |
| Example 9 | 96.8 | 11.4 |
| Example 10 | 99.1 | 39.3 |
| Example 11 | 96.0 | 22.0 |
| Example 12 | 96.9 | 34.6 |
| Example 13 | 99.7 | 58.3 |
| Example 14 | 99.5 | 80.6 |
| Example 17 | 99.5 | 97.1 |
| Example 20 | 99.6 | 73.7 |
| Example 22 | 100.0 | 97.6 |
| Example 23 | 99.1 | 61.7 |
| Example 24 | 98.4 | 57.7 |
| Example 26 | 100.0 | 73.5 |
| Example 35 | 100.2 | 97.5 |
| Example 40 | 100.3 | 98.0 |
| Example 41 | 99.8 | 75.4 |
| Example 42 | 99.7 | 62.2 |
| Example 43 | 99.5 | 49.9 |
| Example 65 | 99.5 | 58.5 |
| Example 66 | 98.3 | 50.4 |
| Example 67 | 98.9 | 42.2 |
| Example 70 | 99.9 | 84.5 |
| Example 71 | 100.2 | 95.0 |
| Example 72 | 100.4 | 91.5 |
| Example 73 | 99.9 | 76.5 |
| Example 75 | 99.4 | 52.1 |
| Example 77 | 100.1 | 42.2 |
| Example 78 | 98.3 | 26.5 |
| Example 80 | 99.7 | 85.2 |
| Example 81 | 100.1 | 98.5 |
| Example 82 | 100.1 | 99.2 |
| Example 83 | 99.6 | 97.6 |
| Example 84 | 100.0 | 91.7 |
| Example 86 | 99.5 | 75.9 |
| Example 87 | 99.3 | 57.2 |
| Example 88 | 100.2 | 83.9 |
| Example 91 | 99.1 | 45.8 |

(continued)

| Compound | % inhib $10^{-5}$M | % inhib $10^{-7}$M |
|---|---|---|
| Example 93 | 100.0 | 78.2 |
| Example 94 | 100.1 | 65.1 |
| Example 100 | 99.5 | 62.4 |
| Example 102 | 99.1 | 66.7 |
| Example 105 | 98.8 | 56.0 |
| Example 106 | 99.5 | 53.7 |
| Example 135 | 99.2 | 61.7 |
| Example 160 | 97.6 | 33.5 |
| Example 190 | 99.3 | 43.2 |
| Example 195 | 97.0 | 20.6 |
| Example 200 | 98.8 | 10.2 |
| zolpidem | 99.4 | 73.6 |

Table 2. Affinity for the $\alpha_2$-subunit of the $GABA_A$ receptor

| Compound | % inhib $10^{-5}$M | % inhib $10^{-7}$M |
|---|---|---|
| Example 1 | 94.3 | 49.0 |
| Example 2 | 98.6 | 66.1 |
| Example 3 | 91.3 | 60.8 |
| Example 4 | 97.6 | 53.2 |
| Example 5 | 97.7 | 45.0 |
| Example 6 | 97.5 | 49.4 |
| Example 9 | 61.7 | 5.9 |
| Example 10 | 72.0 | 19.1 |
| Example 11 | 47.0 | 1.7 |
| Example 12 | 71.9 | 2.2 |
| Example 13 | 76.7 | 15.5 |
| Example 14 | 76.9 | 10.8 |
| Example 17 | 83.0 | 36.8 |
| Example 20 | 82.0 | 20.8 |
| Example 22 | 86.6 | 61.2 |
| Example 23 | 80.0 | 0.0 |
| Example 24 | 77.4 | 13.6 |
| Example 26 | 84.5 | 27.4 |
| Example 35 | 92.5 | 57.9 |
| Example 40 | 91.5 | 60.7 |
| Example 41 | 85.3 | 17.0 |
| Example 42 | 79.4 | 16.3 |
| Example 43 | 80.3 | 20.1 |
| Example 65 | 78.5 | 16.3 |
| Example 66 | 79.4 | 7.8 |
| Example 67 | 83.7 | 8.8 |
| Example 70 | 80.3 | 23.6 |
| Example 71 | 88.5 | 50.6 |
| Example 72 | 85.6 | 51.7 |
| Example 73 | 77.0 | 20.6 |
| Example 75 | 68.8 | 9.3 |
| Example 77 | 83.4 | 23.6 |
| Example 80 | 84.3 | 37.0 |

(continued)

| Compound | % inhib $10^{-5}$M | % inhib $10^{-7}$M |
|---|---|---|
| Example 83 | 93.1 | 57.7 |
| Example 84 | 63.9 | 3.1 |
| Example 86 | 80.9 | 23.0 |
| Example 100 | 73.0 | 2.3 |
| Example 102 | 77.9 | 13.7 |
| Example 160 | 81.3 | 9.7 |
| Example 190 | 90.5 | 18.7 |
| Example 195 | 68.5 | 11.3 |
| Example 200 | 76.4 | 2.5 |
| zolpidem | 74.1 | 19.9 |

b) *In vivo* determination of predictive sedative-hypnotic action

The *in vivo* effects of these compounds were assessed by a predictive sedation-hypnosis test in mice (D. J. Sanger et al., Eur. J. Pharmacol., 313, 35-42, 1996; and G. Griebel et al., Psychopharmacology, 146, 205-213, 1999).

Groups of 5-8 male CD1 mice, weighing 22-26 g at the time of test, were used. The test compounds were administered at 98.0 μmol/kg by mean of intraperitoneal injection. Compounds were suspended in 0.25% agar with one drop of Tween in a volume of 10 mL/kg. Control animals were given the vehicle alone. Using a Smart System (Panlab, S.L., Spain) the traveled distance in cm is recorded for each mouse at 5-minutes intervals during a period of 30 minutes after dosing. The inhibition percentage of traveled distance of treated animals versus control animals (the first 5 minutes were discarded) was calculated. Some compounds were also tested in a lower dose - 0.98 μmol/kg - to further discriminate the potency of sedation induction. The results of this test are given in Table 3 and Table 4.

Table 3. Determination of in vivo sedative-hypnotic activity in mice at 98.0 μmol/kg

| Compound | % inhib motor activity |
|---|---|
| Example 1 | 94.66 |
| Example 2 | 96.71 |
| Example 3 | 90.94 |
| Example 4 | 94.65 |
| Example 5 | 93.65 |
| Example 6 | 96.86 |
| Example 9 | 93.85 |
| Example 10 | 93.47 |
| Example 11 | 79.82 |
| Example 12 | 83.44 |
| Example 13 | 92.08 |
| Example 14 | 95.56 |
| Example 17 | 93.08 |
| Example 20 | 91.51 |
| Example 22 | 87.97 |
| Example 23 | 91.74 |
| Example 24 | 86.54 |
| Example 26 | 91.55 |
| Example 35 | 80.60 |
| Example 40 | 91.79 |
| Example 41 | 91.18 |
| Example 42 | 91.01 |
| Example 43 | 95.72 |
| Example 65 | 95.46 |
| Example 66 | 95.95 |

(continued)

| Compound | % inhib motor activity |
|---|---|
| Example 67 | 90.81 |
| Example 70 | 86.98 |
| Example 71 | 95.96 |
| Example 72 | 93.35 |
| Example 73 | 94.07 |
| Example 75 | 92.56 |
| Example 77 | 89.35 |
| Example 78 | 91.14 |
| Example 80 | 94.41 |
| Example 81 | 90.83 |
| Example 82 | 94.22 |
| Example 83 | 88.93 |
| Example 84 | 90.78 |
| Example 86 | 92.62 |
| Example 87 | 90.70 |
| Example 88 | 88.51 |
| Example 91 | 93.05 |
| Example 93 | 93.20 |
| Example 94 | 93.49 |
| Example 100 | 93.71 |
| Example 102 | 85.50 |
| Example 105 | 94.02 |
| Example 106 | 94.05 |
| Example 135 | 82.47 |
| Example 160 | 81.87 |
| Example 190 | 89.00 |
| Example 195 | 90.63 |
| Example 200 | 93.23 |
| zolpidem | 91.70 |

Table 4. Determination of in vivo sedative-hypnotic activity in mice at 0.98 μmol/kg

| Compound | % inhib motor activity |
|---|---|
| Example 1 | 27.56 |
| Example 2 | 38.39 |
| Example 3 | 14.61 |
| Example 4 | 38.38 |
| Example 5 | 41.15 |
| Example 6 | 51.90 |
| Example 9 | 19.94 |
| Example 10 | 29.54 |
| Example 11 | 15.29 |
| Example 12 | 15.05 |
| Example 13 | 15.11 |
| Example 14 | 4.77 |
| Example 17 | 9.91 |
| Example 22 | 22.21 |
| Example 23 | 12.37 |
| Example 24 | 3.65 |

(continued)

| Compound | % inhib motor activity |
|---|---|
| Example 26 | 19.19 |
| Example 35 | 6.76 |
| Example 41 | 15.73 |
| Example 135 | 38.46 |
| Example 160 | 10.49 |
| Example 190 | 5.11 |
| Example 195 | 7.70 |
| Example 200 | 2.27 |
| zolpidem | 18.30 |

c) *In vivo* determination of predictive anesthetic activity

The *in vivo* effects of these compounds were assessed by a predictive anesthetic test in mice as the loss of righting reflex (Kralic et al., Neuropharmacology, 43(4), 685-689 2002; Belelli et al., Neuropharmacology, 45, 57-71, 2003).

Groups of 5-8 male CD1 mice, weighing 22-26 g at the time of test, were used. The test compounds were administered at 98.0 μmol/kg by mean of intraperitoneal injection. Compounds were suspended in 0.25% agar with one drop of Tween in a volume of 10 mL/kg. Percentage of treated mice which showed loss of righting reflex was calculated.

Interestingly, compounds of examples 2, 3, and 82 exhibited a 90%, 100% and 30% of animals with loss of righting reflex respectively. On the contrary, zolpidem, the prior art compound, exhibited lower anesthetic potential, being necessary to administer double dose than compounds of the present invention to achieve 80% of animals with loss of righting reflex.

The following non-limiting examples illustrate the scope of the present invention.

Example A: General method for the preparation of amides (X)

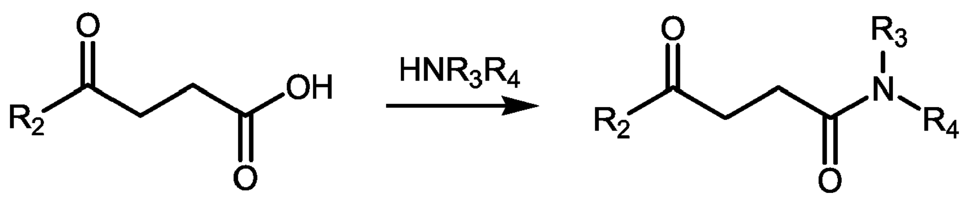


(IX) (X)

To a solution of the acid (IX) (1 eq) in dichloromethane was added a solution of water-soluble carbodiimide (1.5 eq) in dichloromethane. The mixture was stirred at room temperature for 30 minutes. After this period, a solution of 0.5 eq of 4-dimethylamino-pyridine and 1.5 eq of the corresponding amine in dichloromethane was added, and the mixture was stirred for 6 h. The crude was washed with HCl 1 N, the organic layer was dried over $Na_2SO_4$ and filtered off, and the solvent was removed *in vacuo,* to afford the ketoamide (X).

E.g. for

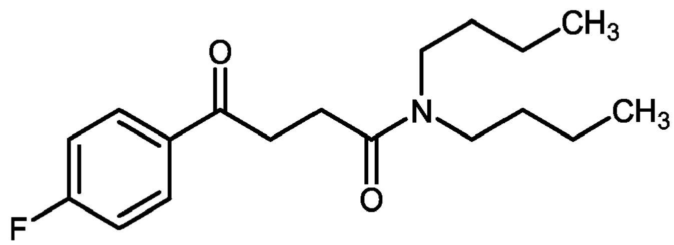

$^1H$ NMR (400 MHz, DMSO-$d_6$): δ 7.80-7.15 (m, 4H, Ar), 3.30 (t, 4H, $CH_2N$), 2.87 (t, 2H, $CH_2CO$), 2.47 (t, 2H, $H_2CON$), 1.58-0.93 (m, 14H, $CH_2CH_2CH_3$).
MS (ES) m/z = 308 ($MH^+$)
HPLC = 100%
Yield = 80%

Example B: General method for the preparation of bromoamides (III, X = CO)

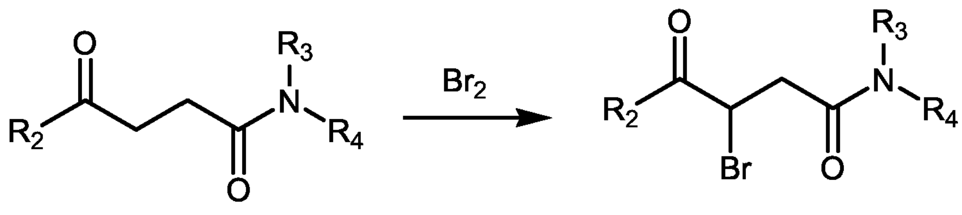


(X) (II, X = CO)

To a solution of (X) (1 eq) in acetic acid was added dropwise a solution of bromine (2.2 eq) in acetic acid. The mixture was stirred at room temperature for 24 h. The solvent was removed *in vacuo* and the residue was extracted with dichloromethane / NaOH 1 N and with dichloromethane / water. The organic layer was dried over $Na_2SO_4$ and filtered off, and the solvent was removed *in vacuo,* thus affording the bromoketoamide (III).

E.g. for

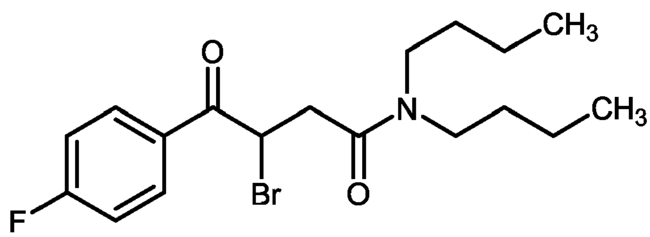

$^1H$ NMR (400 MHz, DMSO-$d_6$): δ 7.97-7.23 (m, 4H, Ar), 5.20 (t, 2H, CHBr), 3.24 (t, 4H, $CH_2N$), 2.87 (d, 2H, $CH_2CON$), 1.75-0.76 (m, 14H, $CH_2CH_2CH_3$).
MS (ES) m/z = 380 (M), 382 ($M^+2H$)
HPLC = 95%
Yield = 34%

Example C: General method for the preparation of imidazopyridazines (I, X = CO)

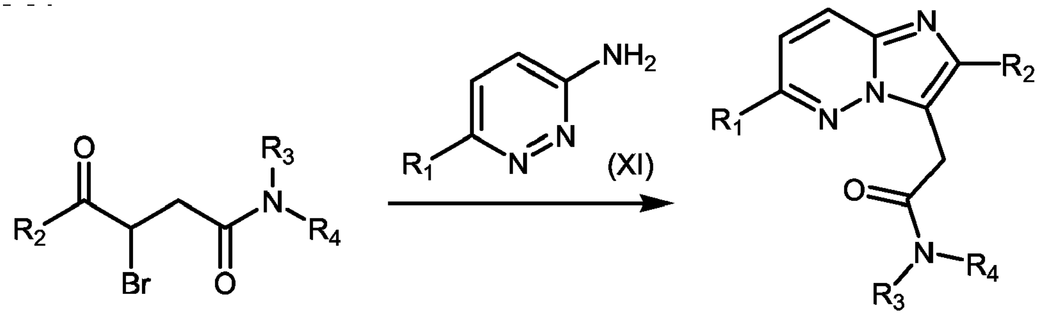


(III, X = CO) (I, X = CO)

To a solution of (III) (1 eq) in acetonitrile was added a solution of (XI) (1.2 eq) in acetonitrile. The mixture was stirred at reflux for 2 h. The solvent was removed *in vacuo* and the residue was extracted with dichloromethane / HCl 1 N and with DCM / water. The organic layer was dried over $Na_2SO_4$ and filtered off, and the solvent was removed *in vacuo,* thus affording the imidazopyridazine (I).

E.g. for

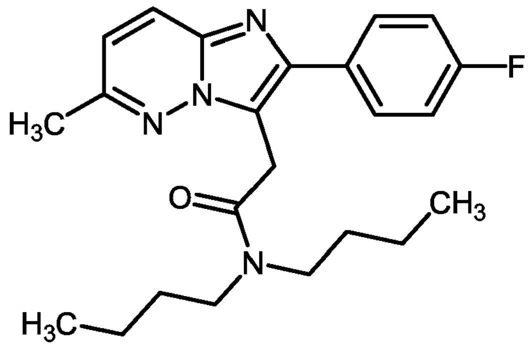

$^1$H NMR (400 MHz, DMSO-$d_6$): δ 7.30-7.03 (m, 6H, Ar), 3.48 (s, 2H, $CH_2$), 2.32 (s, 3H, $CH_3$), 3.21-0.96 (m, 18H, $CH_2CH_2CH_2CH_3$).
MS (ES) m/z = 397 ($MH^+$)
HPLC = 89%
Yield = 60%

Compounds 1-98 were prepared following this methodology.

| Example Number | MH+ (LCMS) | Purity (UV) | IUPAC NAME |
|---|---|---|---|
| 1 | 358 | 99 | 2-(6-Chloro-2-p-tolyl-imidazo[1,2-b]pyridazin-3-yl)-N,N-diethyl-acetamide |
| 2 | 386 | 99 | 2-(6-Chloro-2-p-tolyl-imidazo[1,2-b]pyridazin-3-yl)-N,N-dipropyl-acetamide |
| 3 | 414 | 97 | N,N-Dibutyl-2-(6-chloro-2-p-tolyl-imidazo[1,2-b]pyridazin-3-yl)-acetamide |
| 4 | 370 | 94 | 2-(6-Chloro-2-p-tolyl-imidazo[1,2-b]pyridazin-3-yl)-1-piperidin-1-yl-ethanone |
| 5 | 372 | 97 | 2-(6-Chloro-2-p-tolyl-imidazo[1,2-b]pyridazin-3-yl)-1-morpholin-4-yl-ethanone |
| 6 | 356 | 83 | 2-(6-Chloro-2-p-tolyl-imidazo[1,2-b]pyridazin-3-yl)-1-pyrrolidin-1-yl-ethanone |

(continued)

| Example Number | $MH^+$ (LCMS) | Purity (UV) | IUPAC NAME |
|---|---|---|---|
| 7 | 393 | 100 | N,N-Diethyl-2-(6-pyrrolidin-1-yl-2-p-tolyl-imidazo[1,2-b] pyridazin-3-yl)-acetamide |
| 8 | 353 | 84 | N,N-Diethyl-2-(6-methoxy-2-p-tolyl-imidazo[1,2-b]pyridazin-3-yl)-acetamide |
| 9 | 446 | 84 | 2-[2-(4-Bromo-phenyl)-6-ethoxy-imidazo[1,2-b]pyridazin-3-yl]-1-morpholin-4-yl-ethanone |
| 10 | 444 | 100 | 2-[2-(4-Bromo-phenyl)-6-ethoxy-imidazo[1,2-b]pyridazin-3-yl]-1-piperidin-1-yl-ethanone |
| 11 | 488 | 100 | 2-[2-(4-Bromo-phenyl)-6-ethoxy-imidazo[1,2-b]pyridazin-3-yl]-N,N-dibutyl-acetamide |
| 12 | 460 | 100 | 2-[2-(4-Bromo-phenyl)-6-ethoxy-imidazo[1,2-b]pyridazin-3-yl]-N,N-dipropyl-acetamide |
| 13 | 432 | 80 | 2-[2-(4-Bromo-phenyl)-6-ethoxy-imidazo[1,2-b]pyridazin-3-yl]-N,N-diethyl-acetamide |
| 14 | 416 | 86 | 2-[2-(4-Bromo-phenyl)-6-methyl-imidazo[1,2-b]pyridazin-3-yl]-1-morpholin-4-yl-ethanone |
| 15 | 414 | 96 | 2-[2-(4-Bromo-phenyl)-6-methyl-imidazo[1,2-b]pyridazin-3-yl]-1-piperidin-1-yl-ethanone |
| 16 | 458 | 99 | 2-[2-(4-Bromo-phenyl)-6-methyl-imidazo[1,2-b]pyridazin-3-yl]-N,N-dibutyl-acetamide |
| 17 | 430 | 99 | 2-[2-(4-Bromo-phenyl)-6-methyl-imidazo[1,2-b]pyridazin-3-yl]-N,N-dipropyl-acetamide |
| 18 | 402 | 98 | 2-[2-(4-Bromo-phenyl)-6-methyl-imidazo[1,2-b]pyridazin-3-yl]-N,N-diethyl-acetamide |
| 19 | 358 | 94 | 2-[2-(4-Chloro-phenyl)-6-methyl-imidazo[1,2b]pyridazin-3-yl]-N,N-diethyl-acetamide |
| 20 | 388 | 87 | 2-[2-(4-Chloro-phenyl)-6-ethoxy-imidazo[1,2-b]pyridazin-3-yl]-N,N-diethyl-acetamide |
| 21 | 386 | 100 | 2-[2-(4-Chloro-phenyl)-6-methyl-imidazo[1,2-b]pyridazin-3-yl]-N,N-dipropyl-acetamide |
| 22 | 414 | 99 | N,N-Dibutyl-2-[2-(4-chloro-phenyl)-6-methyl-imidazo[1,2-b] pyridazin-3-yl]-acetamide |
| 23 | 416 | 98 | 2-[2-(4-Chloro-phenyl)-6-ethoxy-imidazo[1,2-b]pyridazin-3-yl]-N,N-dipropyl-acetamide |
| 24 | 444 | 100 | N,N-Dibutyl-2-[2-(4-chloro-phenyl)-6-ethoxy-imidazo[1,2-b] pyridazin-3-yl]-acetamide |
| 25 | 339 | 80 | N,N-Diethyl-2-(6-methoxy-2-phenyl-imidazo[1,2-b]pyridazin-3-yl)-acetamide |
| 26 | 367 | 96 | 2-(6-Methoxy-2-phenyl-imidazo[1,2-b]pyridazin-3-yl)-N,N-dipropyl-acetamide |
| 27 | 396 | 87 | N,N-Dibutyl-2-(6-methoxy-2-phenyl-imidazo[1,2-b]pyridazin-3-yl)-acetamide |
| 28 | 353 | 100 | 2-(6-Methoxy-2-phenyl-imidazo[1,2-b]pyridazin-3-yl)-1-morpholin-4-yl-ethanone |

(continued)

| Example Number | MH+ (LCMS) | Purity (UV) | IUPAC NAME |
|---|---|---|---|
| 29 | 353 | 83 | 2-(6-Ethoxy-2-phenyl-imidazo[1,2-b]pyridazin-3-yl)-N,N-diethyl-acetamide |
| 30 | 381 | 100 | 2-(6-Ethoxy-2-phenyl-imidazo[1,2-b]pyridazin-3-yl)-N,N-dipropyl-acetamide |
| 31 | 410 | 100 | N,N-Dibutyl-2-(6-ethoxy-2-phenyl-imidazo[1,2-b]pyridazin-3-yl)-acetamide |
| 32 | 367 | 88 | 2-(6-Ethoxy-2-phenyl-imidazo[1,2-b]pyridazin-3-yl)-1-morpholin-4-yl-ethanone |
| 33 | 365 | 80 | 2-(6-Ethoxy-2-phenyl-imidazo[1,2-b]pyridazin-3-yl)-1-piperidin-1-yl-ethanone |
| 34 | 323 | 80 | N,N-Diethyl-2-(6-methyl-2-phenyl-imidazo[1,2-b]pyridazin-3-yl)-acetamide |
| 35 | 351 | 100 | 2-(6-Methyl-2-phenyl-imidazo[1,2-b]pyridazin-3-yl)-N,N-dipropyl-acetamide |
| 36 | 380 | 88 | N,N-Dibutyl-2-(6-methyl-2-phenyl-imidazo[1,2-b]pyridazin-3-yl)-acetamide |
| 37 | 337 | 100 | 2-(6-Methyl-2-phenyl-imidazo[1,2-b]pyridazin-3-yl)-1-morpholin-4-yl-ethanone |
| 38 | 335 | 81 | 2-(6-Methyl-2-phenyl-imidazo[1,2-b]pyridazin-3-yl)-1-piperidin-1-yl-ethanone |
| 39 | 369 | 89 | 2-[2-(4-Fluoro-phenyl)-6-methyl-imidazo[1,2-b]pyridazin-3-yl]-N,N-dipropyl-acetamide |
| 40 | 398 | 89 | N,N-Dibutyl-2-[2-(4-fluoro-phenyl)-6-methyl-imidazo[1,2-b] pyridazin-3-yl]-acetamide |
| 41 | 385 | 90 | 2-[2-(4-Fluoro-phenyl)-6-methoxy-imidazo[1,2-b]pyridazin-3-yl]-N,N-dipropyl-acetamide |
| 42 | 414 | 92 | N,N-Dibutyl-2-[2-(4-fluoro-phenyl)-6-methoxy-imidazo[1,2-b] pyridazin-3-yl]-acetamide |
| 43 | 399 | 85 | 2-[6-Ethoxy-2-(4-fluoro-phenyl)-imidazo[1,2-b]pyridazin-3-yl]-N,N-dipropyl-acetamide |
| 44 | 428 | 93 | N,N-Dibutyl-2-[6-ethoxy-2-(4-fluoro-phenyl)-imidazo[1,2-b] pyridazin-3-yl]-acetamide |
| 45 | 385 | 80 | 2-[6-Ethoxy-2-(4-fluoro-phenyl)-imidazo[1,2-b]pyridazin-3-yl]-1-morpholin-4-yl-ethanone |
| 46 | 353 | 97 | N,N-Diethyl-2-(6-methoxy-2-p-tolyl-imidazo[1,2-b]pyridazin-3-yl)-acetamide |
| 47 | 381 | 82 | 2-(6-Methoxy-2-p-tolyl-imidazo[1,2-b]pyridazin-3-yl)-N,N-dipropyl-acetamide |
| 48 | 410 | 90 | N,N-Dibutyl-2-(6-methoxy-2-p-tolyl-imidazo[1,2-b]pyridazin-3-yl)-acetamide |
| 49 | 365 | 80 | 2-(6-Methoxy-2-p-tolyl-imidazo[1,2-b]pyridazin-3-yl)-1-piperidin-1-yl-ethanone |
| 50 | 367 | 85 | 2-(6-Ethoxy-2-p-tolyl-imidazo[1,2-b]pyridazin-3-yl)-N,N-diethyl-acetamide |

(continued)

| Example Number | MH+ (LCMS) | Purity (UV) | IUPAC NAME |
|---|---|---|---|
| 51 | 396 | 80 | 2-(6-Ethoxy-2-p-tolyl-imidazo[1,2-b]pyridazin-3-yl)-N,N-dipropyl-acetamide |
| 52 | 424 | 89 | N,N-Dibutyl-2-(6-ethoxy-2-p-tolyl-imidazo[1,2-b]pyridazin-3-yl)-acetamide |
| 53 | 365 | 81 | 2-(6-Ethoxy-2-p-tolyl-imidazo[1,2-b]pyridazin-3-yl)-1-pyrrolidin-1-yl-ethanone |
| 54 | 381 | 85 | 2-(6-Ethoxy-2-p-tolyl-imidazo[1,2-b]pyridazin-3-yl)-1-morpholin-4-yl-ethanone |
| 55 | 379 | 80 | 2-(6-Ethoxy-2-p-tolyl-imidazo[1,2-b]pyridazin-3-yl)-1-piperidin-1-yl-ethanone |
| 56 | 337 | 90 | N,N-Diethyl-2-(6-methyl-2-p-tolyl-imidazo[1,2-b]pyridazin-3-yl)-acetamide |
| 57 | 365 | 90 | 2-(6-Methyl-2-p-tolyl-imidazo[1,2-b]pyridazin-3-yl)-N,N-dipropyl-acetamide |
| 58 | 394 | 90 | N,N-Dibutyl-2-(6-methyl-2-p-tolyl-imidazo[1,2-b]pyridazin-3-yl)-acetamide |
| 59 | 335 | 80 | 2-(6-Methyl-2-p-tolyl-imidazo[1,2-b]pyridazin-3-yl)-1-pyrrolidin-1-yl-ethanone |
| 60 | 351 | 85 | 2-(6-Methyl-2-p-tolyl-imidazo[1,2-b]pyridazin-3-yl)-1-morpholin-4-yl-ethanone |
| 61 | 349 | 85 | 2-(6-Methyl-2-p-tolyl-imidazo[1,2-b]pyridazin-3-yl)-1-piperidin-1-yl-ethanone |
| 62 | 341 | 87 | N,N-Diethyl-2-[2-(4-fluoro-phenyl)-6-methyl-imidazo[1,2-b] pyridazin-3-yl]-acetamide |
| 63 | 353 | 81 | 2-[2-(4-Fluoro-phenyl)-6-methyl-imidazo[1,2-b]pyridazin-3-yl]-1-piperidin-1-yl-ethanone |
| 64 | 357 | 90 | N,N-Diethyl-2-[2-(4-fluoro-phenyl)-6-methoxy-imidazo[1,2-b] pyridazin-3-yl]-acetamide |
| 65 | 369 | 94 | 2-[2-(4-Fluoro-phenyl)-6-methoxy-imidazo[1,2-b]pyridazin-3-yl]-1-piperidin-1-yl-ethanone |
| 66 | 371 | 91 | 2-[6-Ethoxy-2-(4-fluoro-phenyl)-imidazo[1,2-b]pyridazin-3-yl]-N,N-diethyl-acetamide |
| 67 | 383 | 91 | 2-[6-Ethoxy-2-(4-fluoro-phenyl)-imidazo[1,2-b]pyridazin-3-yl]-1-piperidin-1-yl-ethanone |
| 68 | 355 | 92 | 2-[2-(4-Fluoro-phenyl)-6-methyl-imidazo[1,2-b]pyridazin-3-yl]-1-morpholin-4-yl-ethanone |
| 69 | 371 | 95 | 2-[2-(4-Fluoro-phenyl)-6-methoxy-imidazo[1,2-b]pyridazin-3-yl]-1-morpholin-4-yl-ethanone |
| 70 | 353 | 99 | N,N-Diethyl-2-[2-(4-methoxy-phenyl)-6-methyl-imidazo[1,2-b] pyridazin-3-yl]-acetamide |
| 71 | 381 | 99 | 2-[2-(4-Methoxy-phenyl)-6-methyl-imidazo[1,2-b]pyridazin-3-yl]-N,N-dipropyl-acetamide |
| 72 | 410 | 100 | N,N-Dibutyl-2-[2-(4-methoxy-phenyl)-6-methyl-imidazo[1,2-b] pyridazin-3-yl]-acetamide |

(continued)

| Example Number | MH+ (LCMS) | Purity (UV) | IUPAC NAME |
|---|---|---|---|
| 73 | 365 | 100 | 2-[2-(4-Methoxy-phenyl)-6-methyl-imidazo[1,2-b]pyridazin-3-yl]-1-piperidin-1-yl-ethanone |
| 74 | 367 | 92 | 2-[2-(4-Methoxy-phenyl)-6-methyl-imidazo[1,2-b]pyridazin-3-yl]-1-morpholin-4-yl-ethanone |
| 75 | 369 | 100 | N,N-Diethyl-2-[6-methoxy-2-(4-methoxy-phenyl)-imidazo[1,2-b] pyridazin-3-yl]-acetamide |
| 76 | 397 | 98 | 2-[6-Methoxy-2-(4-methoxy-phenyl)-imidazo[1,2-b]pyridazin-3-yl]-N,N-dipropyl-acetamide |
| 77 | 426 | 100 | N,N-Dibutyl-2-[6-methoxy-2-(4-methoxy-phenyl)-imidazo[1,2-b] pyridazin-3-yl]-acetamide |
| 78 | 381 | 100 | 2-[6-Methoxy-2-(4-methoxy-phenyl)-imidazo[1,2-b]pyridazin-3-yl]-1-piperidin-1-yl-ethanone |
| 79 | 383 | 97 | 2-[6-Methoxy-2-(4-methoxy-phenyl)-imidazo[1,2-b]pyridazin-3-yl]-1-morpholin-4-yl-ethanone |
| 80 | 410 | 94 | Acetic acid 2-{[2-(6-methyl-2-p-tolyl-imidazo[1,2-b]pyridazin-3-yl)-acetyl]-propyl-amino}-ethyl ester |
| 81 | 378 | 89 | 1 -(3,5-Dimethyl-piperidin-1-yl)-2-(6-methyl-2-p-tolyl-imidazo [1,2-b]pyridazin-3-yl)-ethanone |
| 82 | 378 | 93 | N-Cyclopropylmethyl-2-(6-methyl-2-p-tolyl-imidazo[1,2-b] pyridazin-3-yl)-N-propyl-acetamide |
| 83 | 364 | 98 | 2-(6-Methyl-2-p-tolyl-imidazo[1,2-b]pyridazin-3-yl)-N-thiazol-2-yl-acetamide |
| 84 | 365 | 97 | N,N-Diisopropyl-2-(6-methyl-2-p-tolyl-imidazo[1,2-b]pyridazin-3-yl)-acetamide |
| 85 | 363 | 96 | N-Cyclohexyl-2-(6-methyl-2-p-tolyl-imidazo[1,2-b]pyridazin-3-yl)-acetamide |
| 86 | 357 | 95 | 2-(6-Methyl-2-p-tolyl-imidazo[1,2-b]pyridazin-3-yl)-N-phenyl-acetamide |
| 87 | 371 | 99 | 2-(6-Methyl-2-p-tolyl-imidazo[1,2-b]pyridazin-3-yl)-N-p-tolyl-acetamide |
| 88 | 358 | 96 | 2-(6-Methyl-2-p-tolyl-imidazo[1,2-b]pyridazin-3-yl)-N-pyridin-2-yl-acetamide |
| 89 | 372 | 94 | 2-(6-Methyl-2-p-tolyl-imidazo[1,2-b]pyridazin-3-yl)-N-pyridin-2-ylmethyl-acetamide |
| 90 | 376 | 99 | N-(3,5-Dimethyl-isoxazol-4-yl)-2-(6-methyl-2-p-tolyl-imidazo [1,2-b]pyridazin-3-yl)-acetamide |
| 91 | 349 | 93 | N-Cyclopentyl-2-(6-methyl-2-p-tolyl-imidazo[1,2-b]pyridazin-3-yl)-acetamide |
| 92 | 361 | 82 | N,N-Diallyl-2-(6-methyl-2-p-tolyl-imidazo[1,2-b]pyridazin-3-yl)-acetamide |
| 93 | 321 | 97 | N-Cyclopropyl-2-(6-methyl-2-p-tolyl-imidazo[1,2-b]pyridazin-3-yl)-acetamide |
| 94 | 408 | 95 | 2-(6-Methyl-2-p-tolyl-imidazo[1,2-b]pyridazin-3-yl)-N-quinolin-2-yl-acetamide |

(continued)

| Example Number | MH+ (LCMS) | Purity (UV) | IUPAC NAME |
|---|---|---|---|
| 95 | 362 | 95 | N-(5-Methyl-isoxazol-3-yl)-2-(6-methyl-2-p-tolyl-imidazo[1,2-b] pyridazin-3-yl)-acetamide |
| 96 | 387 | 99 | N-(4-Methoxy-phenyl)-2-(6-methyl-2-p-tolyl-imidazo[1,2-b] pyridazin-3-yl)-acetamide |
| 97 | 362 | 99 | N-(3-Methyl-isoxazol-5-yl)-2-(6-methyl-2-p-tolyl-imidazo[1,2-b] pyridazin-3-yl)-acetamide |
| 98 | 365 | 99 | 2-(6-Methyl-2-p-tolyl-imidazo[1,2-b]pyridazin-3-yl)-N-[1,3,4] thiadiazol-2-yl-acetamide |

Example D: General method for the preparation of ketoesters (XII)

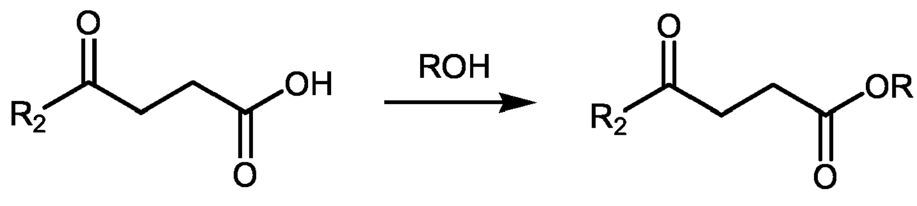


(IX) (XII)

To a solution of (IX) (1 eq) in methanol (ROH, R = $CH_3$) was added dropwise a solution of concentrated $H_2SO_4$ (0.5 eq) in methanol. The mixture was stirred at reflux for 30 minutes. The solvent was removed *in vacuo* and the residue was extracted with dichloromethane / NaOH 1 N and with dichloromethane / water. The organic layer was dried over $Na_2SO_4$ and filtered off, and the solvent was removed *in vacuo,* thus affording the ketoester (XII).

E.g. for

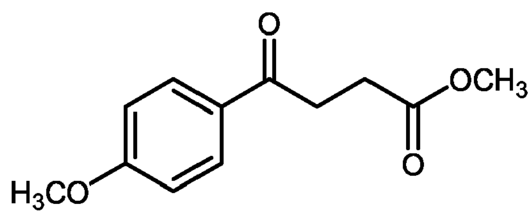

$^1H$ NMR (400 MHz, DMSO-$d_6$): δ 7.89-6.88 (m, 4H, Ar), 3.75 (s, 3H, $OCH_3$), 3.77 (s, 3H, $OCH_3$), 2.65 (t, 2H, $CH_2CO$), 2.25 (t, 2H, $CH_2COO$).
MS (ES) m/z = 223 ($MH^+$)
HPLC = 95%
Yield = 93%

Example E: General method for the preparation of bromoketoesters (XIII)

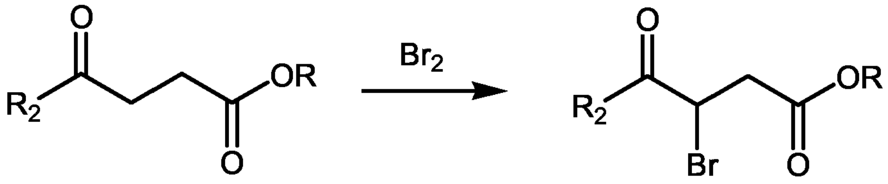


(XII) (XIII)

To a solution of (XII) (1 eq) in acetic acid was added dropwise a solution of bromine (2.2 eq) in acetic acid. The mixture was stirred at room temperature for 24 h. The solvent was removed *in vacuo* and the residue was extracted with dichloromethane / NaOH 1 N and with dichloromethane / water. The organic layer was dried over $Na_2SO_4$ and filtered off, and the solvent was removed *in vacuo,* thus affording the bromoketoester (XIII).

E.g. for

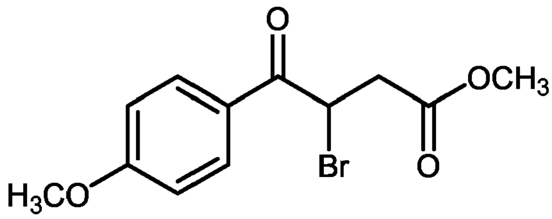

$^1H$ NMR (400 MHz, DMSO-$d_6$): δ 7.98-6.82 (m, 4H, Ar), 5.38 (t, 1 H, CHBr), 3.98 (s, 3H, $OCH_3$), 3.54 (s, 3H, $OCH_3$), 2.75 (t, 2H, $CH_2COO$).
MS (ES) m/z = 301 (M), 303 ($M^+$2H)
HPLC = 95%
Yield = 35%

Example F: General method for the preparation of imidazopyridazines (IV)

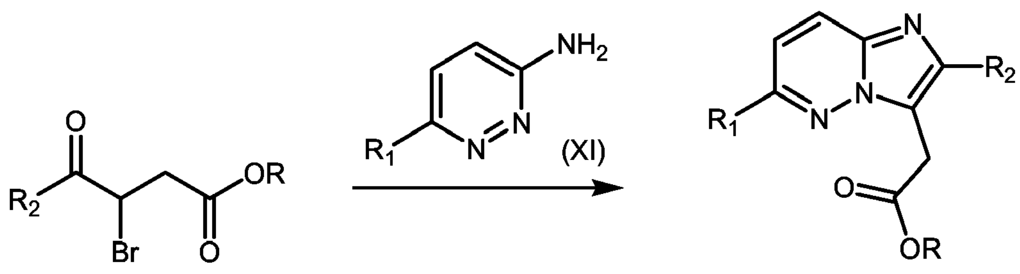


(XIII) (IV)

To a solution of (XIII) (1 eq) in acetonitrile was added a solution of (XI) (1.2 eq) in acetonitrile. The mixture was stirred at reflux for 2 h. The solvent was removed *in vacuo* and the residue was extracted with dichloromethane / HCl 1 N and with dichloromethane / water. The organic layer was dried over $Na_2SO_4$ and filtered off, and the solvent was removed *in vacuo,* thus affording the imidazopyridazine (IV).

E.g. for

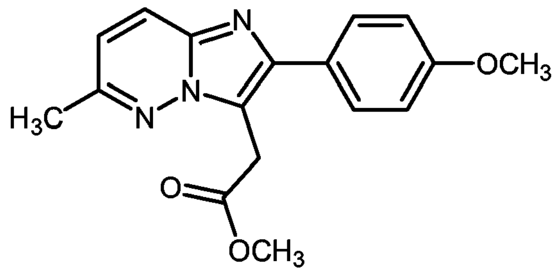

$^1$H NMR (400 MHz, DMSO-$d_6$): δ 7.69-6.79 (m, 6H, Ar), 3.75 (s, 3H, $OCH_3$), 3.67 (s, 3H, $OCH_3$), 3.35 (s, 2H, $CH_2$), 2.17 (s, 3H, $CH_3$).
MS (ES) m/z = 312 ($MH^+$)
HPLC = 90%
Yield = 60%

Example G: General method for the preparation of imidazopyridazines (I, X = CO, $R_3$ or $R_4$ are substitued amino groups)

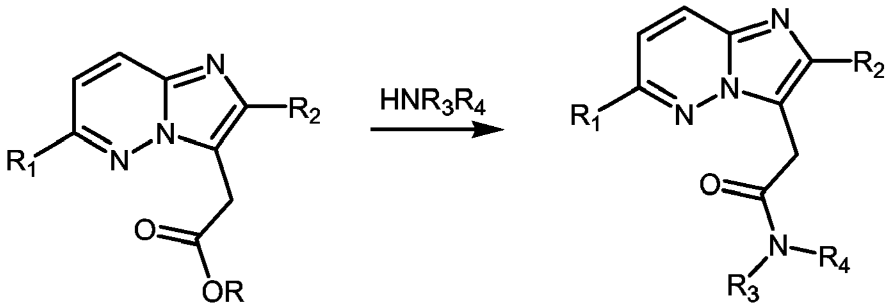


(IV) (I, X = CO)

To a solution of (IV) (1 eq) in methanol was added a solution of (substituted) hydrazine (5 eq) in methanol. The mixture was stirred at reflux for 24 h. The solvent was removed *in vacuo* and the residue was extracted with dichloromethane / HCl 1 N and with dichloromethane / water. The organic layer was dried over $Na_2SO_4$ and filtered off, and the solvent was removed in *vacuo,* thus affording the imidazopyridazine (I).

E.g. for

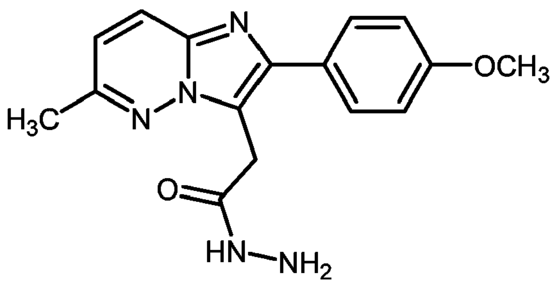

$^1$H NMR (400 MHz, DMSO-$d_6$): δ 8.00(bs, 1 H, NH), 7.50-6.93 (m, 6H, Ar), 3.78 (s, 3H, $OCH_3$), 3.96 (s, 3H, $OCH_3$), 3.28 (s, 2H, $CH_2$), 2.12 (bs, 2H, $NH_2$).
MS (ES) m/z = 312 ($MH^+$)
HPLC = 93%

Yield = 65%

Compounds 99-107 were prepared following this methodology.

| Example Number | $MH^+$ (LCMS) | Purity (UV) | IUPAC NAME |
|---|---|---|---|
| 99 | 355 | 100 | [2-(4-Fluoro-phenyl)-6-pyrrolidin-1-yl-imidazo[1,2-b]pyridazin-3-yl]-acetic acid hydrazide |
| 100 | 361 | 100 | [2-(4-Bromo-phenyl)-6-methyl-imidazo[1,2-b]pyridazin-3-yl]-acetic acid hydrazide |
| 101 | 312 | 93 | [2-(4-Methoxy-phenyl)-6-methyl-imidazo[1,2-b]pyridazin-3-yl]-acetic acid hydrazide |
| 102 | 317 | 99 | [2-(4-Chloro-phenyl)-6-methyl-imidazo[1,2-b]pyridazin-3-yl]-acetic acid hydrazide |
| 103 | 300 | 92 | [2-(4-Fluoro-phenyl)-6-methyl-imidazo[1,2-b]pyridazin-3-yl]-acetic acid hydrazide |
| 104 | 282 | 99 | (6-Methyl-2-phenyl-imidazo[1,2-b]pyridazin-3-yl)-acetic acid hydrazide |
| 105 | 366 | 91 | 2-(6-Methyl-2-p-tolyl-imidazo[1,2-b]pyridazin-3-yl)-N-morpholin-4-yl-acetamide |
| 106 | 364 | 90 | 2-(6-Methyl-2-p-tolyl-imidazo[1,2-b]pyridazin-3-yl)-N-piperidin-1-yl-acetamide |
| 107 | 324 | 91 | (6-Methyl-2-p-tolyl-imidazo[1,2-b]pyridazin-3-yl)-acetic acid N',N'-dimethyl-hydrazide |

Example H: General method for the preparation of imidazopyridazines (VI)

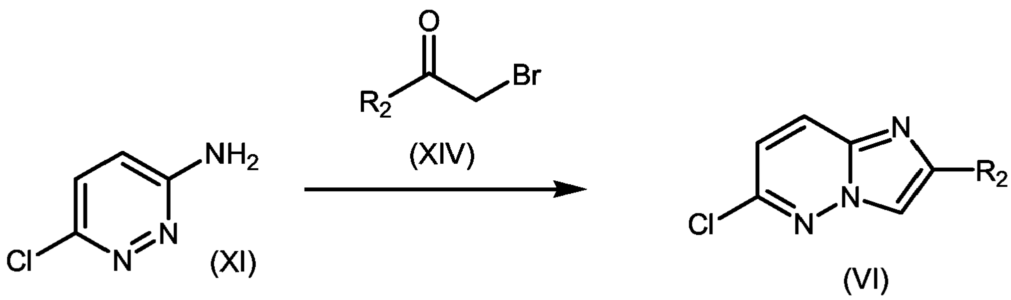
(XIV)
(XI)
(VI)

(XI, $R_1$ = Cl)

To a solution of (XI) (1.2 eq) in ethanol was added a solution of (XIV) (1 eq) in ethanol. The mixture was stirred at reflux for 2 h. The solvent was removed in *vacuo*. Acetone was added to the residue and the mixture was stirred at room temperature for 30 minutes, and the solid thus obtained was filtered off and dried over $CaCl_2$, thus affording the imidazopyridazine (VI).

E.g. for

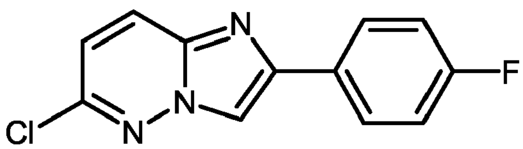

$^1H$ NMR (400 MHz, DMSO-$d_6$): δ 7.79-7.15 (m, 6H, Ar), 7.12 (s, 1 H, H-3).
MS (ES) m/z = 248 ($MH^+$)
HPLC=100%
Yield = 60%

Example I: General method for the preparation of acetamides (VIII)

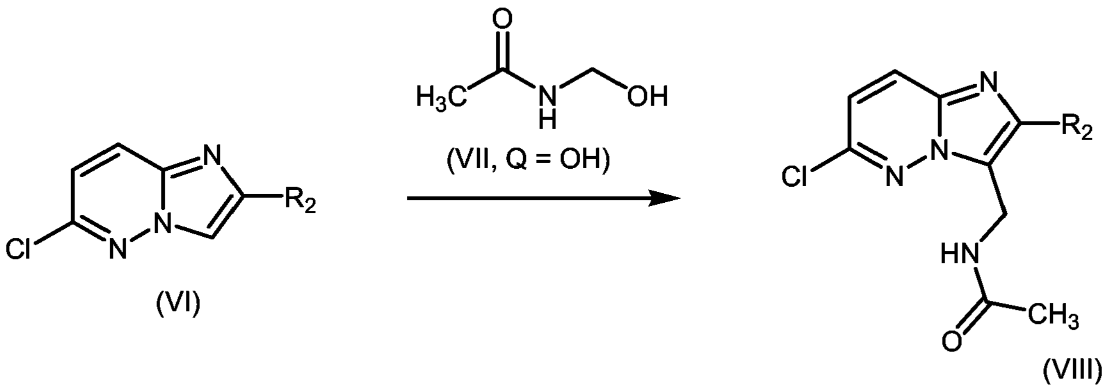


(VI)

(VIII)

To a solution of (VI) (1 eq) in acetic acid was added a solution of (VII) (1 eq) in acetic acid. To this mixture was added dropwise a solution of 2.5 eq of $H_2SO_4$. The mixture was stirred at reflux for 6 h. The solvent was removed *in vacuo* and the residue was extracted with dichloromethane / NaOH 1 N and with dichloromethane / water. The organic layer was dried over $Na_2SO_4$ and filtered off, and the solvent was removed *in vacuo,* thus affording the acetamide (VIII).

E.g. for

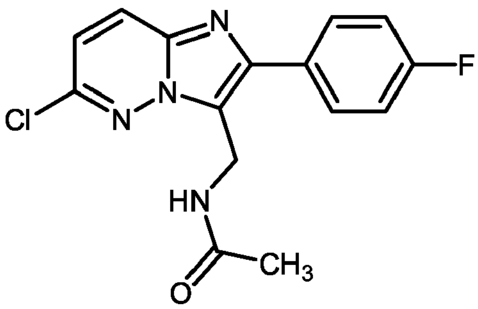

$^1H$ NMR (400 MHz, DMSO-$d_6$): δ 7.99-6.98 (m, 6H, Ar), 4.45 (s, 2H, $CH_2$), 2.09 (s, 3H, $CH_3$).
MS (ES) m/z = 319 ($MH^+$)
HPLC = 85%
Yield = 50%

Example J: General method for the preparation of amines (V)

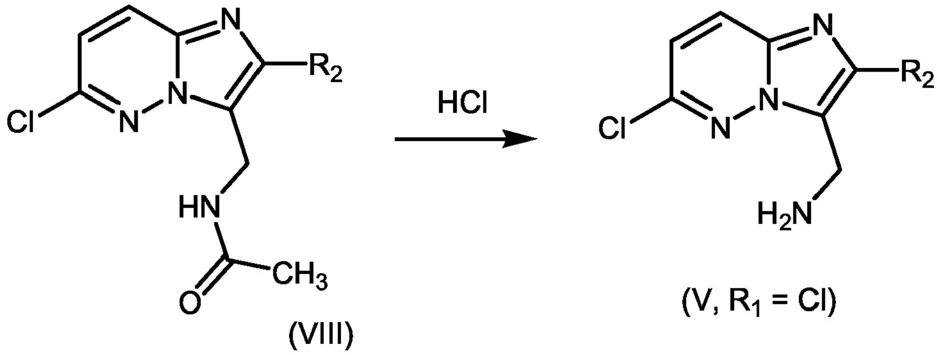


(VIII)

(V, $R_1$ = Cl)

To a solution of (VIII) (1 eq) in ethanol was added a solution of concentrated hydrochloric acid. The mixture was stirred at reflux for 24 h. The solvent was removed *in vacuo* and the residue was extracted with dichloromethane / NaOH 1 N and with dichloromethane / water. The organic layer was dried over $Na_2SO_4$ and filtered off, and the solvent was removed *in vacuo,* thus affording the amine (V).

E.g. for

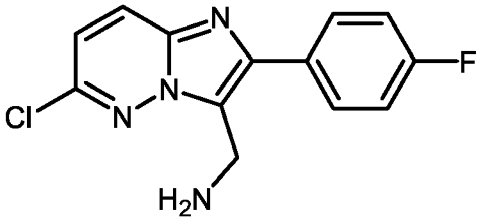

$^1H$ NMR (400 MHz, DMSO-$d_6$): δ 7.89-7.05 (m, 6H, Ar), 3.82 (s, 2H, $CH_2$), 2.09 (bs, 2H, $NH_2$).
MS (ES) m/z = 277 ($MH^+$)
HPLC = 85%
Yield = 87%

Example K: General method for the substitution of amines (V), e.g. substitution by $CH_3O$ group

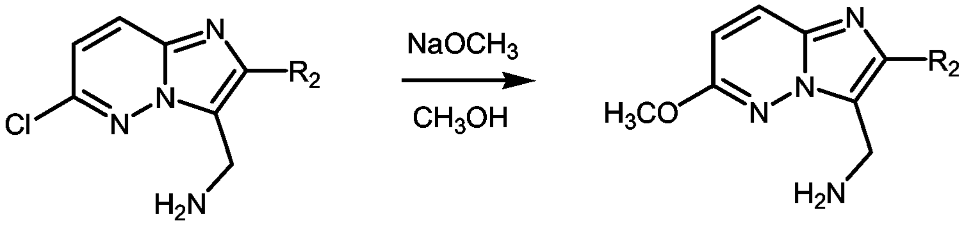


(V, $R_1$ = Cl) (V, $R_1$ = $OCH_3$)

To a solution of (V) (1 eq) in methanol was added a solution of sodium methoxide (10 eq) in methanol. The mixture was stirred at reflux for 24 h. The solvent was removed *in vacuo* and the residue was extracted with dichloromethane / water. The organic layer was dried over $Na_2SO_4$ and filtered off, and the solvent was removed *in vacuo,* thus affording the amine (V).

E.g. for

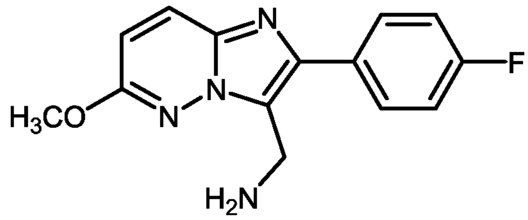

$^1H$ NMR (400 MHz, DMSO-$d_6$): δ 7.97-7.09 (m, 6H, Ar), 3.96 (s, 3H, $OCH_3$), 3.65 (s, 2H, $CH_2$), 2.03 (bs, 2H, $NH_2$).
MS (ES) m/z = 273 ($MH^+$)
HPLC = 100%
Yield = 68%

Example L: General method for the preparation of amides (II, X = CO)

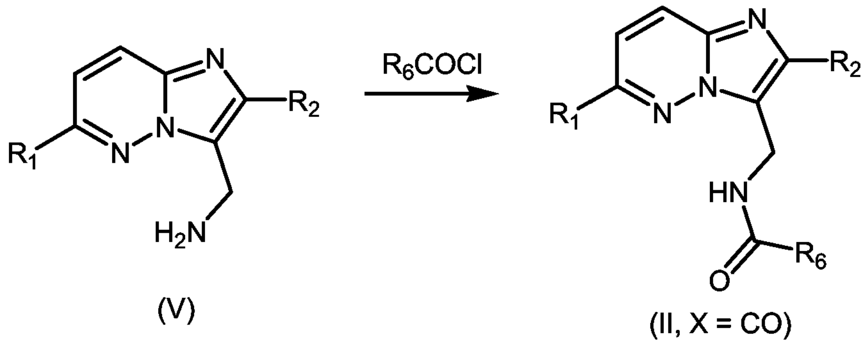


(V)

(II, X = CO)

To a solution of (V) (1 eq) in dichloromethane was added a solution of triethylamine (2 eq) in dichloromethane. To this mixture was added dropwise a solution of $R_6COCl$ (1.2 eq) in dichloromethane. The mixture was stirred at room temperature for 24 h. The solvent was removed *in vacuo* and the residue was extracted with dichloromethane / NaOH 1 N , with dichloromethane / HCl 1 N and with dichloromethane / water. The organic layer was dried over $Na_2SO_4$ and filtered off, and the solvent was removed in *vacuo,* thus affording the amides (II).

E.g. for

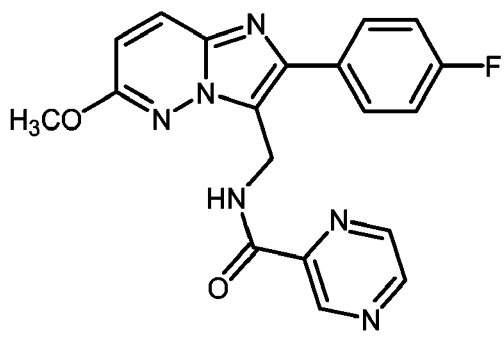

$^1H$ NMR (400 MHz, DMSO-$d_6$): δ 9.03-7.16 (m, 9H, Ar), 4.02 (s, 2H, $CH_2$), 3.96 (s, 3H, $OCH_3$).
MS (ES) m/z = 379 ($MH^+$)
HPLC = 99%
Yield = 38%

Compounds 108-141 were prepared following this methodology.

| Example Number | MH+ (LCMS) | Purity (UV) | IUPAC NAME |
|---|---|---|---|
| 108 | 384 | 100 | Thiophene-2-carboxylic acid (6-chloro-2-p-tolyl-imidazo[1,2-b] pyridazin-3-ylmethyl)-amide |
| 109 | 379 | 83 | N-(6-Chloro-2-p-tolyl-imidazo[1,2-b]pyridazin-3-ylmethyl)-isonicotinamide |
| 110 | 430 | 80 | Quinoxaline-2-carboxylic acid (6-chloro-2-p-tolyl-imidazo[1,2-b] pyridazin-3-ylmethyl)-amide |
| 111 | 380 | 86 | Pyrazine-2-carboxylic acid (6-chloro-2-p-tolyl-imidazo[1,2-b] pyridazin-3-ylmethyl)-amide |
| 112 | 369 | 88 | Isoxazole-5-carboxylic acid (6-chloro-2-p-tolyl-imidazo[1,2-b] pyridazin-3-ylmethyl)-amide |
| 113 | 458 | 80 | Thiophene-2-carboxylic acid (6-phenylsulfanyl-2-p-tolyl-imidazo [1,2-b]pyridazin-3-ylmethyl)-amide |
| 114 | 443 | 91 | Isoxazole-5-carboxylic acid (6-phenylsulfanyl-2-p-tolyl-imidazo [1,2-b]pyridazin-3-ylmethyl)-amide |
| 115 | 454 | 96 | Pyrazine-2-carboxylic acid (6-phenylsulfanyl-2-p-tolyl-imidazo [1,2-b]pyridazin-3-ylmethyl)-amide |
| 116 | 504 | 89 | Quinoxaline-2-carboxylic acid (6-phenylsulfanyl-2-p-tolyl-imidazo[1,2-b]pyridazin-3-ylmethyl)-amide |
| 117 | 453 | 100 | N-(6-Phenylsulfanyl-2-p-tolyl-imidazo[1,2-b]pyridazin-3-ylmethyl)-isonicotinamide |
| 118 | 281 | 100 | N-(2-p-Tolyl-imidazo[1,2-b]pyridazin-3-ylmethyl)-acetamide |
| 119 | 350 | 100 | N-(6-Pyrrolidin-1-yl-2-p-tolyl-imidazo[1,2-b]pyridazin-3-ylmethyl)-acetamide |
| 120 | 364 | 100 | N-(6-Piperidin-1-yl-2-p-tolyl-imidazo[1,2-b]pyridazin-3-ylmethyl)-acetamide |
| 121 | 366 | 80 | N-(6-Morpholin-4-yl-2-p-tolyl-imidazo[1,2-b]pyridazin-3-ylmethyl)-acetamide |
| 122 | 379 | 95 | N-[6-(4-Methyl-piperazin-1-yl)-2-p-tolyl-imidazo[1,2-b] pyridazin-3-ylmethyl]-acetamide |
| 123 | 415 | 83 | N-[2-(4-Bromo-phenyl)-6-pyrrolidin-1-yl-imidazo[1,2-b] pyridazin-3-ylmethyl]-acetamide |
| 124 | 336 | 87 | N-(2-Phenyl-6-pyrrolidin-1-yl-imidazo[1,2-b]pyridazin-3-ylmethyl)-acetamide |
| 125 | 354 | 94 | N-[2-(4-Fluoro-phenyl)-6-pyrrolidin-1-yl-imidazo[1,2-b] pyridazin-3-ylmethyl]-acetamide |
| 126 | 326 | 89 | N-(2-Furan-2-yl-6-pyrrolidin-1-yl-imidazo[1,2-b]pyridazin-3-ylmethyl)-acetamide |
| 127 | 366 | 100 | N-[2-(4-Methoxy-phenyl)-6-pyrrolidin-1-yl-imidazo[1,2-b] pyridazin-3-ylmethyl]-acetamide |
| 128 | 381 | 100 | N-[2-(4-Nitro-phenyl)-6-pyrrolidin-1-yl-imidazo[1,2-b]pyridazin-3-ylmethyl]-acetamide |
| 129 | 404 | 95 | N-[6-Pyrrolidin-1-yl-2-(4-trifluoromethyl-phenyl)-imidazo[1,2-b] pyridazin-3-ylmethyl]-acetamide |
| 130 | 361 | 87 | Pyrazine-2-carboxylic acid (6-methoxy-2-phenyl-imidazo[1,2-b] pyridazin-3-ylmethyl)-amide |

(continued)

| Example Number | MH+ (LCMS) | Purity (UV) | IUPAC NAME |
|---|---|---|---|
| 131 | 350 | 80 | Isoxazole-5-carboxylic acid (6-methoxy-2-phenyl-imidazo[1,2-b] pyridazin-3-ylmethyl)-amide |
| 132 | 400 | 80 | Pyrazine-2-carboxylic acid (2-phenyl-6-pyrrolidin-1-yl-imidazo [1,2-b]pyridazin-3-ylmethyl)-amide |
| 133 | 389 | 86 | Isoxazole-5-carboxylic acid (2-phenyl-6-pyrrolidin-1-yl-imidazo [1,2-b]pyridazin-3-ylmethyl)-amide |
| 134 | 357 | 96 | N-(6-Phenyl-2-p-tolyl-imidazo[1,2-b]pyridazin-3-ylmethyl)-acetamide |
| 135 | 379 | 99 | Pyrazine-2-carboxylic acid [2-(4-fluoro-phenyl)-6-methoxy-imidazo[1,2-b]pyridazin-3-ylmethyl]-amide |
| 136 | 368 | 100 | Isoxazole-5-carboxylic acid [2-(4-fluoro-phenyl)-6-methoxy-imidazo[1,2-b]pyridazin-3-ylmethyl]-amide |
| 137 | 385 | 83 | Isoxazole-5-carboxylic acid [2-(4-chloro-phenyl)-6-methoxy-imidazo[1,2-b]pyridazin-3-ylmethyl]-amide |
| 138 | 440 | 100 | Pyrazine-2-carboxylic acid [2-(4-bromo-phenyl)-6-methoxy-imidazo[1,2-b]pyridazin-3-ylmethyl]-amide |
| 139 | 454 | 98 | Pyrazine-2-carboxylic acid [2-(4-bromo-phenyl)-6-ethoxy-imidazo[1,2-b]pyridazin-3-ylmethyl]-amide |
| 140 | 435 | 97 | Pyrazine-2-carboxylic acid [2-(4-chloro-phenyl)-6-pyrrolidin-1-yl-imidazo[1,2-b]pyridazin-3-ylmethyl]-amide |
| 141 | 396 | 83 | Pyrazine-2-carboxylic acid [2-(4-chloro-phenyl)-6-methoxy-imidazo[1,2-b]pyridazin-3-ylmethyl]-amide |

Example M: General method for the preparation of ureas (II, X = CO, $R_6$= $HNR_7$)

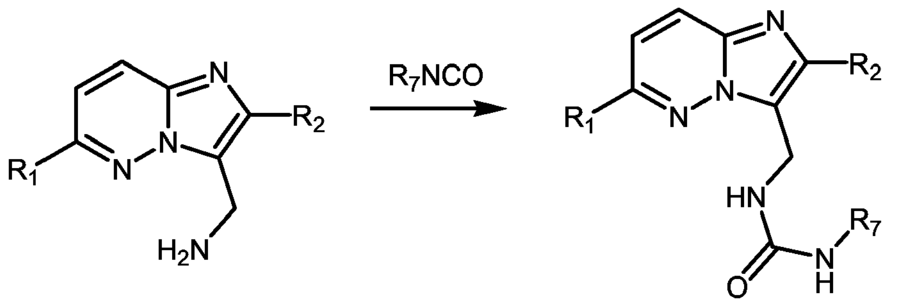


(V)

(II, X = CO, $R_6$ = $HNR_7$)

To a solution of (V) (1 eq) in dichloromethane was added a solution of triethylamine (2 eq) in dichloromethane. To this mixture was added dropwise a solution of $R_7NCO$ (1.2 eq) in dichloromethane. The mixture was stirred at room temperature for 24 h. The solvent was removed *in vacuo* and the residue was extracted with dichloromethane / NaOH 1 N , with dichloromethane / HCl 1 N and with dichloromethane / water. The organic layer was dried over $Na_2SO_4$ and filtered off, and the solvent was removed *in vacuo,* thus affording the ureas (II).

E.g. for

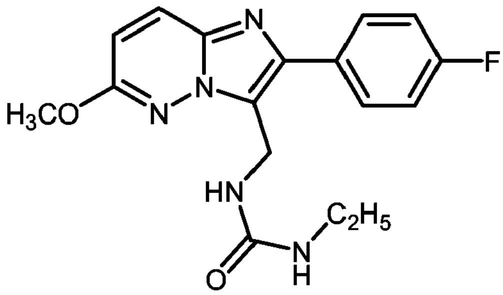

$^1H$ NMR (400 MHz, DMSO-$d_6$): δ 7.58-7.02 (m, 6H, Ar), 6.10 (bs, 2H, NH), 4.32 (s, 2H, $CH_2$), 3.85 (s, 3H, $OCH_3$), 3.15 (q, 2H, $CH_2N$), 1.22 (t, 3H, $CH_3$).
MS (ES) m/z = 343 ($MH^+$)
HPLC = 100%
Yield = 41 %

Compounds 142-164 were prepared following this methodology.

| Example Number | $MH^+$ (LCMS) | Purity (UV) | IUPAC NAME |
|---|---|---|---|
| 142 | 385 | 100 | 1-(6-Chloro-2-p-tolyl-imidazo[1,2-b]pyridazin-3-ylmethyl)-3-cyclopentyl-urea |
| 143 | 435 | 85 | 1-(4-Acetyl-phenyl)-3-(6-chloro-2-p-tolyl-imidazo[1,2-b] pyridazin-3-ylmethyl)-urea |
| 144 | 438 | 80 | 1-(6-Chloro-2-p-tolyl-imidazo[1,2-b]pyridazin-3-ylmethyl)-3-(4-nitro-phenyl)-urea |
| 145 | 427 | 93 | 1-(4-Chloro-phenyl)-3-(6-chloro-2-p-tolyl-imidazo[1,2-b] pyridazin-3-ylmethyl)-urea |
| 146 | 411 | 95 | 1-(6-Chloro-2-p-tolyl-imidazo[1,2-b]pyridazin-3-ylmethyl)-3-(4-fluoro-phenyl)-urea |
| 147 | 407 | 93 | 1-(6-Chloro-2-p-tolyl-imidazo[1,2-b]pyridazin-3-ylmethyl)-3-p-tolyl-urea |
| 148 | 439 | 85 | 1-(6-Chloro-2-p-tolyl-imidazo[1,2-b]pyridazin-3-ylmethyl)-3-(4-methylsulfanyl-phenyl)-urea |
| 149 | 423 | 80 | 1-(6-Chloro-2-p-tolyl-imidazo[1,2-b]pyridazin-3-ylmethyl)-3-(4-methoxy-phenyl)-urea |
| 150 | 393 | 93 | 1-(6-Chloro-2-p-tolyl-imidazo[1,2-b]pyridazin-3-ylmethyl)-3-phenyl-urea |
| 151 | 359 | 86 | 1-(6-Chloro-2-p-tolyl-imidazo[1,2-b]pyridazin-3-ylmethyl)-3-isopropyl-urea |
| 152 | 345 | 80 | 1-(6-Chloro-2-p-tolyl-imidazo[1,2-b]pyridazin-3-ylmethyl)-3-ethyl-urea |
| 153 | 419 | 89 | 1-Ethyl-3-(6-phenylsulfanyl-2-p-tolyl-imidazo[1,2-b]pyridazin-3-ylmethyl)-urea |
| 154 | 467 | 89 | 1-Phenyl-3-(6-phenylsulfanyl-2-p-tolyl-imidazo[1,2-b]pyridazin-3-ylmethyl)-urea |

(continued)

| Example Number | $MH^+$ (LCMS) | Purity (UV) | IUPAC NAME |
|---|---|---|---|
| 155 | 485 | 90 | 1-(4-Fluoro-phenyl)-3-(6-phenylsulfanyl-2-p-tolyl-imidazo[1,2-b]pyridazin-3-ylmethyl)-urea |
| 156 | 459 | 91 | 1-Cyclopentyl-3-(6-phenylsulfanyl-2-p-tolyl-imidazo[1,2-b]pyridazin-3-ylmethyl)-urea |
| 157 | 433 | 87 | 1-Isopropyl-3-(6-phenylsulfanyl-2-p-tolyl-imidazo[1,2-b]pyridazin-3-ylmethyl)-urea |
| 158 | 509 | 84 | 1-(4-Acetyl-phenyl)-3-(6-phenylsulfanyl-2-p-tolyl-imidazo[1,2-b]pyridazin-3-ylmethyl)-urea |
| 159 | 326 | 86 | 1-Ethyl-3-(6-methoxy-2-phenyl-imidazo[1,2-b]pyridazin-3-ylmethyl)-urea |
| 160 | 344 | 100 | 1-Ethyl-3-[2-(4-fluoro-phenyl)-6-methoxy-imidazo[1,2-b]pyridazin-3-ylmethyl]-urea |
| 161 | 361 | 82 | 1-[2-(4-Chloro-phenyl)-6-methoxy-imidazo[1,2-b]pyridazin-3-ylmethyl]-3-ethyl-urea |
| 162 | 405 | 99 | 1-[2-(4-Bromo-phenyl)-6-methoxy-imidazo[1,2-b]pyridazin-3-ylmethyl]-3-ethyl-urea |
| 163 | 419 | 98 | 1-[2-(4-Bromo-phenyl)-6-ethoxy-imidazo[1,2-b]pyridazin-3-ylmethyl]-3-ethyl-urea |
| 164 | 400 | 87 | 1-[2-(4-Chloro-phenyl)-6-pyrrolidin-1-yl-imidazo[1,2-b]pyridazin-3-ylmethyl]-3-ethyl-urea |

Example N: General method for the preparation of carbamates (II, X = CO, $R_6 = OR_7$)

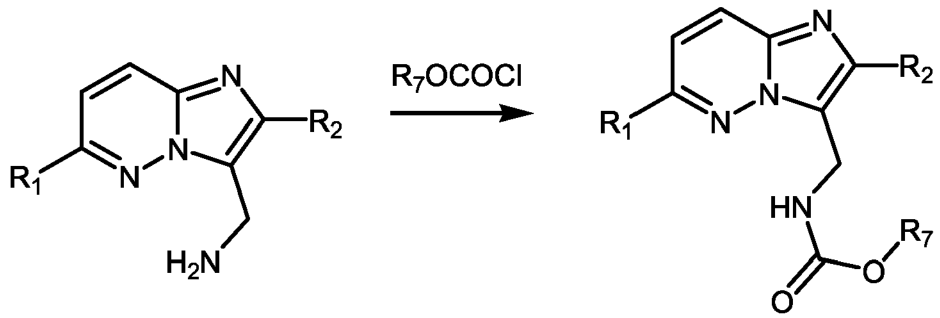


(V) (II, X = CO, $R_6 = OR_7$)

To a solution of (V) (1 eq) in dichloromethane was added a solution of triethylamine (2 eq) in dichloromethane. To this mixture was added dropwise a solution of $R_7OCOCl$ (1.2 eq) in dichloromethane. The mixture was stirred at room temperature for 24 h. The solvent was removed *in vacuo* and the residue was extracted with dichloromethane / NaOH 1 N , with dichloromethane / HCl 1 N and with dichloromethane / water. The organic layer was dried over $Na_2SO_4$ and filtered off, and the solvent was removed in *vacuo,* thus affording the carbamates (II).

E.g. for

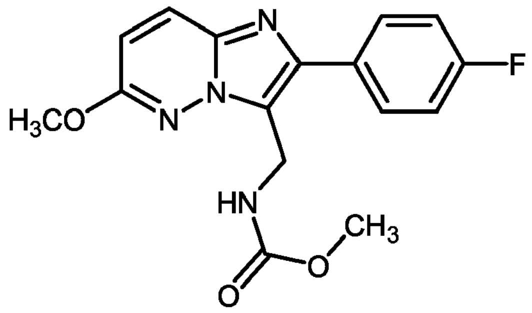

$^1H$ NMR (400 MHz, DMSO-$d_6$): δ 8.07 (bs, 1 H, NH), 7.45-7.06 (m, 6H, Ar), 4.17 (s, 2H, $CH_2$), 3.78 (s, 3H, $OCH_3$), 3.62 (s, 3H, $CH_3$).
MS (ES) m/z = 331 ($MH^+$)
HPLC = 87%
Yield = 35%

Compounds 165-203 were prepared following this methodology.

| Example Number | $MH^+$ (LCMS) | Purity (UV) | IUPAC NAME |
|---|---|---|---|
| 165 | 346 | 86 | (6-Chloro-2-p-tolyl-imidazo[1,2-b]pyridazin-3-ylmethyl)-carbamic acid ethyl ester |
| 166 | 356 | 85 | (6-Chloro-2-p-tolyl-imidazo[1,2-b]pyridazin-3-ylmethyl)-carbamic acid prop-2-ynyl ester |
| 167 | 449 | 86 | (6-Chloro-2-p-tolyl-imidazo[1,2-b]pyridazin-3-ylmethyl)-carbamic acid 2,2,2-trichloro-ethyl ester |
| 168 | 394 | 83 | (6-Chloro-2-p-tolyl-imidazo[1,2-b]pyridazin-3-ylmethyl)-carbamic acid phenyl ester |
| 169 | 408 | 85 | (6-Chloro-2-p-tolyl-imidazo[1,2-b]pyridazin-3-ylmethyl)-carbamic acid p-tolyl ester |
| 170 | 424 | 80 | (6-Chloro-2-p-tolyl-imidazo[1,2-b]pyridazin-3-ylmethyl)-carbamic acid 4-methoxy-phenyl ester |
| 171 | 439 | 80 | (6-Chloro-2-p-tolyl-imidazo[1,2-b]pyridazin-3-ylmethyl)-carbamic acid 4-nitro-phenyl ester |
| 172 | 408 | 88 | (6-Chloro-2-p-tolyl-imidazo[1,2-b]pyridazin-3-ylmethyl)-carbamic acid benzyl ester |
| 173 | 332 | 89 | (6-Chloro-2-p-tolyl-imidazo[1,2-b]pyridazin-3-ylmethyl)-carbamic acid methyl ester |
| 174 | 374 | 92 | (6-Chloro-2-p-tolyl-imidazo[1,2-b]pyridazin-3-ylmethyl)-carbamic acid isobutyl ester |
| 175 | 374 | 94 | (6-Chloro-2-p-tolyl-imidazo[1,2-b]pyridazin-3-ylmethyl)-carbamic acid butyl ester |
| 176 | 420 | 100 | (6-Phenylsulfanyl-2-p-tolyl-imidazo[1,2-b]pyridazin-3-ylmethyl)-carbamic acid ethyl ester |
| 177 | 405 | 80 | (6-Phenylsulfanyl-2-p-tolyl-imidazo[1,2-b]pyridazin-3-ylmethyl)-carbamic acid methyl ester |
| 178 | 468 | 89 | (6-Phenylsulfanyl-2-p-tolyl-imidazo[1,2-b]pyridazin-3-ylmethyl)-carbamic acid phenyl ester |

(continued)

| Example Number | MH+ (LCMS) | Purity (UV) | IUPAC NAME |
|---|---|---|---|
| 179 | 482 | 85 | (6-Phenylsulfanyl-2-p-tolyl-imidazo[1,2-b]pyridazin-3-ylmethyl)-carbamic acid p-tolyl ester |
| 180 | 498 | 80 | (6-Phenylsulfanyl-2-p-tolyl-imidazo[1,2-b]pyridazin-3-ylmethyl)-carbamic acid 4-methoxy-phenyl ester |
| 181 | 482 | 85 | (6-Phenylsulfanyl-2-p-tolyl-imidazo[1,2b]pyridazin-3-ylmethyl)-carbamic acid benzyl ester |
| 182 | 434 | 87 | (6-Phenylsulfanyl-2-p-tolyl-imidazo[1,2-b]pyridazin-3-ylmethyl)-carbamic acid isopropyl ester |
| 183 | 448 | 86 | (6-Phenylsulfanyl-2-p-tolyl-imidazo[1,2-b]pyridazin-3-ylmethyl)-carbamic acid butyl ester |
| 184 | 448 | 85 | (6-Phenylsulfanyl-2-p-tolyl-imidazo[1,2-b]pyridazin-3-ylmethyl)-carbamic acid isobutyl ester |
| 185 | 355 | 100 | (6-Methoxy-2-phenyl-imidazo[1,2-b]pyridazin-3-ylmethyl)-carbamic acid isobutyl ester |
| 186 | 327 | 100 | (6-Methoxy-2-phenyl-imidazo[1,2-b]pyridazin-3-ylmethyl)-carbamic acid ethyl ester |
| 187 | 313 | 83 | (6-Methoxy-2-phenyl-imidazo[1,2-b]pyridazin-3-ylmethyl)-carbamic acid methyl ester |
| 188 | 366 | 100 | (2-Phenyl-6-pyrrolidin-1-yl-imidazo[1,2-b]pyridazin-3-ylmethyl)-carbamic acid ethyl ester |
| 189 | 373 | 85 | [2-(4-Fluoro-phenyl)-6-methoxy-imidazo[1,2-b]pyridazin-3-ylmethyl]-carbamic acid isobutyl ester |
| 190 | 345 | 89 | [2-(4-Fluoro-phenyl)-6-methoxy-imidazo[1,2-b]pyridazin-3-ylmethyl]-carbamic acid ethyl ester |
| 191 | 331 | 87 | [2-(4-Fluoro-phenyl)-6-methoxy-imidazo[1,2-b]pyridazin-3-ylmethyl]-carbamic acid methyl ester |
| 192 | 348 | 81 | [2-(4-Chloro-phenyl)-6-methoxy-imidazo[1,2-b]pyridazin-3-ylmethyl]-carbamic acid methyl ester |
| 193 | 390 | 90 | [2-(4-Chloro-phenyl)-6-methoxy-imidazo[1,2-b]pyridazin-3-ylmethyl]-carbamic acid isobutyl ester |
| 194 | 362 | 85 | [2-(4-Chloro-phenyl)-6-methoxy-imidazo[1,2-b]pyridazin-3-ylmethyl]-carbamic acid ethyl ester |
| 195 | 434 | 97 | [2-(4-Bromo-phenyl)-6-methoxy-imidazo[1,2-b]pyridazin-3-ylmethyl]-carbamic acid isobutyl ester |
| 196 | 406 | 95 | [2-(4-Bromo-phenyl)-6-methoxy-imidazo[1,2-b]pyridazin-3-ylmethyl]-carbamic acid ethyl ester |
| 197 | 392 | 93 | [2-(4-Bromo-phenyl)-6-methoxy-imidazo[1,2-b]pyridazin-3-ylmethyl]-carbamic acid methyl ester |
| 198 | 448 | 100 | [2-(4-Bromo-phenyl)-6-ethoxy-imidazo[1,2-b]pyridazin-3-ylmethyl]-carbamic acid isobutyl ester |
| 199 | 420 | 100 | [2-(4-Bromo-phenyl)-6-ethoxy-imidazo[1,2-b]pyridazin-3-ylmethyl]-carbamic acid ethyl ester |
| 200 | 406 | 93 | [2-(4-Bromo-phenyl)-6-ethoxy-imidazo[1,2-b]pyridazin-3-ylmethyl]-carbamic acid methyl ester |

(continued)

| Example Number | $MH^+$ (LCMS) | Purity (UV) | IUPAC NAME |
|---|---|---|---|
| 201 | 387 | 80 | [2-(4-Chloro-phenyl)-6-pyrrolidin-1-yl-imidazo[1,2-b]pyridazin-3-ylmethyl]-carbamic acid methyl ester |
| 202 | 401 | 88 | [2-(4-Chloro-phenyl)-6-pyrrolidin-1-yl-imidazo[1,2-b]pyridazin-3-ylmethyl]-carbamic acid ethyl ester |
| 203 | 429 | 96 | [2-(4-Chloro-phenyl)-6-pyrrolidin-1-yl-imidazo[1,2-b]pyridazin-3-ylmethyl]-carbamic acid isobutyl ester |

Example O: General method for the preparation of sulfonamides (II, X = $SO_2$

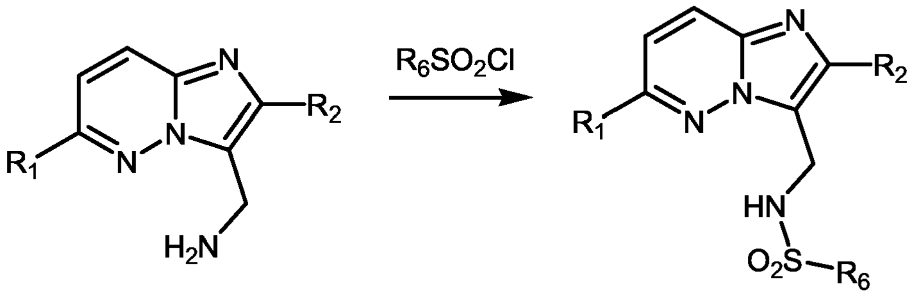


(V)

(II, X = $SO_2$)

To a solution of (V) (1 eq) in dichloromethane was added a solution of triethylamine (2 eq) in dichloromethane. To this mixture was added dropwise a solution of $R_6SO_2Cl$ (1.2 eq) in dichloromethane. The mixture was stirred at room temperature for 24 h. The solvent was removed *in vacuo* and the residue was extracted with dichloromethane / NaOH 1 N , with dichloromethane / HCl 1 N and with dichloromethane / water. The organic layer was dried over $Na_2SO_4$ and filtered off, and the solvent was removed in *vacuo,* thus affording the amides (II).

E.g. for

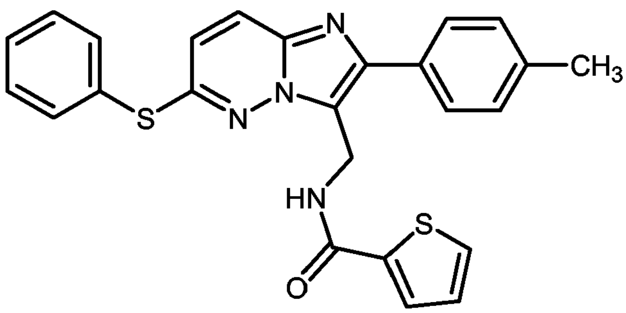

$^1H$ NMR (400 MHz, DMSO-$d_6$): δ 8.10 (bs, 1 H, NH), 7.69-7.14 (m, 14H, Ar), 4.26 (s, 2H, $CH_2$), 2.25 (s, 3H, $CH_3$).
MS (ES) m/z = 424 ($MH^+$)
HPLC = 89%
Yield = 32%

Compounds 204-213 were prepared following this methodology.

| Example Number | MH+ (LCMS) | Purity (UV) | IUPAC NAME |
|---|---|---|---|
| 204 | 426 | 89 | N-(6-Phenylsulfanyl-2-p-tolyl-imidazo[1,2-b]pyridazin-3-ylmethyl)-methanesulfonamide |
| 205 | 488 | 80 | N-(6-Phenylsulfanyl-2-p-tolyl-imidazo[1,2-b]pyridazin-3-ylmethyl)-benzenesulfonamide |
| 206 | 502 | 85 | 4-Methyl-N-(6-phenylsulfanyl-2-p-tolyl-imidazo[1,2-b]pyridazin-3-ylmethyl)-benzenesulfonamide |
| 207 | 518 | 85 | 4-Methoxy-N-(6-phenylsulfanyl-2-p-tolyl-imidazo[1,2-b] pyridazin-3-ylmethyl)-benzenesulfonamide |
| 208 | 522 | 84 | 4-Chloro-N-(6-phenylsulfanyl-2-p-tolyl-imidazo[1,2-b]pyridazin-3-ylmethyl)-benzenesulfonamide |
| 209 | 533 | 83 | 4-Nitro-N-(6-phenylsulfanyl-2-p-tolyl-imidazo[1,2-b]pyridazin-3-ylmethyl)-benzenesulfonamide |
| 210 | 494 | 88 | Thiophene-2-sulfonic acid (6-phenylsulfanyl-2-p-tolyl-imidazo [1,2-b]pyridazin-3-ylmethyl)-amide |
| 211 | 492 | 91 | 1-Methyl-1 H-imidazole-4-sulfonic acid (6-phenylsulfanyl-2-p-tolyl-imidazo[1,2-b]pyridazin-3-ylmethyl)-amide |
| 212 | 602 | 90 | 5-Bromo-6-chloro-pyridine-3-sulfonic acid (6-phenylsulfanyl-2-p-tolyl-imidazo[1,2-b]pyridazin-3-ylmethyl)-amide |
| 213 | 507 | 90 | 3,5-Dimethyl-isoxazole-4-sulfonic acid (6-phenylsulfanyl-2-p-tolyl-imidazo[1,2-b]pyridazin-3-ylmethyl)-amide |

Composition example 1: 5 mg tablets

| Active ingredient | 5.0 mg |
|---|---|
| Colloidal silicon dioxide | 0.6 mg |
| Croscarmellose sodium | 12.0 mg |
| Talc | 4.0 mg |
| Magnesium stearate | 1.5 mg |
| Polysorbate 80 | 1.0 mg |
| Lactose | 75.0 mg |
| Hydroxypropyl methylcellulose | 3.0 mg |
| Polyethylene glycol 4000 | 0.5 mg |
| Titanium dioxide E171 | 1.5 mg |
| Microcrystalline cellulose q.s. to | 125.0 mg |

Composition example 2: 10 mg capsules

| Active ingredient | 10.0 mg |
|---|---|
| Colloidal silicon dioxide | 0.6 mg |
| Crospovidone | 12.0 mg |
| Talc | 4.0 mg |
| Magnesium stearate | 1.5 mg |
| Lauryl sulfate sodium | 1.5 mg |

(continued)

| | |
|---|---|
| Lactose | 77.0 mg |
| Gelatin | 28.5 mg |
| Titanium dioxide E171 | 1.5 mg |
| Indigotin E132 | 0.02 mg |
| Microcrystalline cellulose q.s. to | 155.0 mg |

Composition example 3: oral drops

| Active ingredient | 0.5 g |
|---|---|
| Propylene glycol | 10.0 g |
| Glycerin | 5.0 g |
| Saccharin sodium | 0.1 g |
| Polysorbate 80 | 1.0 g |
| Lemon flavor | 0.2 g |
| Ethanol | 25.0 mL |
| Purified water q.s. to | 100.0 mL |

Composition example 4: 2.5 mg tablets

| Active ingredient | 2.5 mg |
|---|---|
| Colloidal silicon dioxide | 0.6 mg |
| Croscaramellose sodium | 12.0 mg |
| Talc | 4.0 mg |
| Magnesium stearate | 1.5 mg |
| Polysorbate 80 | 1.0 mg |
| Lactose | 75.0 mg |
| Hydroxypropyl methylcellulose | 3.0 mg |
| Polyethylene glycol 4000 | 0.5 mg |
| Titanium dioxide E171 | 1.5 mg |
| Microcrystalline cellulose q.s. to | 125.0 mg |

Composition example 5: 5 mg capsules

| Active ingredient | 5.0 mg |
|---|---|
| Colloidal silicon dioxide | 0.6 mg |
| Crospovidone | 12.0 mg |
| Talc | 4.0 mg |
| Magnesium stearate | 1.5 mg |
| Lauryl sulfate sodium | 1.5 mg |
| Lactose | 77.0 mg |
| Gelatin | 28.5 mg |
| Titanium dioxide E171 | 1.5 mg |
| Indigotin E132 | 0.02 mg |

(continued)

| | |
|---|---|
| Microcrystalline q.s.to | 155.0 mg |

Composition example 6: Oral drops

| Active ingredient | 0.25 g |
|---|---|
| Propylene glycol | 10.0 g |
| Glycerin | 5.0 g |
| Saccharin sodium | 0.1 g |
| Polysorbate 80 | 1.0 g |
| Lemon flavor | 0.2 g |
| Ethanol | 25.0 mL |
| Purified q.s. to | 100.0 mL |

## Claims

**1.** Imidazo[1,2-b]pyridazine compounds of formula (I)

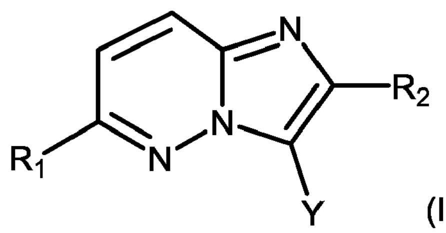

(I)

wherein

$R_1$ and $R_2$ are independently selected from the group consisting of hydrogen, linear or branched alkyl($C_1$-$C_6$), alkenyl($C_2$-$C_6$), alkynyl($C_2$-$C_6$), cycloalkyl($C_3$-$C_6$), haloalkyl($C_2$-$C_6$), hydroxy, -O-alkyl($C_1$-$C_6$), phenoxy, -S-alkyl ($C_1$-$C_6$), phenylthio, halogen, nitro, cyano, amino, alkylamino($C_1$-$C_6$), dialkylamino($C_1$-$C_6$), pyrrolidinyl, morpholinyl, piperidinyl, N-alkyl($C_1$-$C_6$)piperazinyl, phenyl optionally substituted by 1 to 5 Z groups and heteroaryl optionally substituted by 1 to 5 Z groups;
Y is selected from

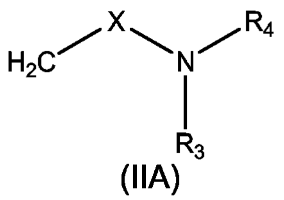

(IIA)

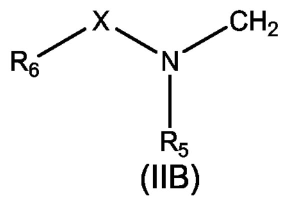

(IIB)

$R_3$ and $R_4$ are independently selected from the group consisting of hydrogen, linear or branched alkyl($C_1$-$C_6$), alkenyl($C_2$-$C_6$), alkynyl($C_2$-$C_6$), cycloalkyl($C_3$-$C_6$), hydroxyalkyl($C_1$-$C_6$), amino, -NH-alkyl($C_1$-$C_6$), -N-dialkyl ($C_1$-$C_6$), pyrrolidinyl, morpholinyl, piperidinyl, -N-alkyl($C_1$-$C_6$)piperazinyl, -N-acyl($C_1$-$C_6$)piperazinyl, phenyl optionally substituted by 1 to 5 Z groups and heteroaryl optionally substituted by 1 to 5 Z groups, or both $R_3$ and $R_4$ can form, together with the nitrogen atom to which they are attached, a 5-6 membered heterocyclic ring optionally substituted by 1 to 5 Z groups, with the proviso that

$R_3$ and $R_4$ may not be simultaneously hydrogen;
$R_5$ is selected from the group consisting of hydrogen, linear or branched alkyl($C_1$-$C_6$), cycloalkyl($C_3$-$C_6$), cycloalkyl($C_3$-$C_6$)alkyl($C_1$-$C_6$), alkenyl($C_2$-$C_6$), alkenyl($C_2$-$C_6$)alkyl($C_1$-$C_6$), alkynyl($C_2$-$C_6$) and alkenyl($C_2$-$C_6$)alkyl ($C_1$-$C_6$);
$R_6$ is selected from the group consisting of haloalkyl($C_2$-$C_6$), cycloalkyl($C_3$-$C_6$), cycloalkyl($C_3$-$C_6$)alkyl($C_1$-$C_6$), alkynyl($C_2$-$C_6$)alkyl($C_1$-$C_6$), alkenyl($C_2$-$C_6$)alkyl($C_1$-$C_6$), alkyl($C_1$-$C_6$)-O-alkyl($C_1$-$C_6$), alkyl($C_1$-$C_6$)-NH-alkyl ($C_1$-$C_6$), alkyl($C_1$-$C_6$)-N(dialkyl($C_1$-$C_6$)), -$OR_7$, -$NHR_7$, -$NR_7R_8$, aryl optionally substituted by 1 to 5 Z groups and heteroaryl optionally substituted by 1 to 5 Z groups, with the proviso that $R_6$ may not be 3-pyridyl;
$R_7$ and $R_8$ are independently selected from the group consisting of hydrogen, linear or branched alkyl($C_1$-$C_6$), phenylalkyl($C_1$-$C_6$), haloalkyl($C_2$-$C_6$), cycloalkyl($C_3$-$C_6$), cycloalkyl($C_3$-$C_6$)alkyl($C_1$-$C_6$), alkenyl($C_2$-$C_6$), alkynyl ($C_2$-$C_6$), alkenyl($C_2$-$C_6$)alkyl($C_1$-$C_6$), alkynyl($C_2$-$C_6$)alkyl($C_1$-$C_6$), phenyl optionally substituted by 1 to 5 Z groups and heteroaryl optionally substituted by 1 to 5 Z groups, or both $R_7$ and $R_8$ can form, together with the nitrogen atom to which they are attached, a 5-6 membered heterocyclic ring optionally substituted by 1 to 5 Z groups;
X is selected from CO and $SO_2$;
Z is selected from the group consisting of linear or branched alkyl(C1-C6), alkenyl($C_2$-$C_6$), alkynyl($C_2$-$C_6$), cycloalkyl($C_3$-$C_6$), haloalkyl($C_2$-$C_6$), hydroxy, - O-alkyl($C_1$-$C_6$), phenoxy, -S-alkyl($C_1$-$C_6$), phenylthio, halogen, nitro, cyano, amino, alkylamino($C_1$-$C_6$) and dialkylamino($C_1$-$C_6$); and
pharmaceutically acceptable salts, polymorphs, hydrates, tautomers, solvates and stereoisomers thereof,
with the proviso that when Y is

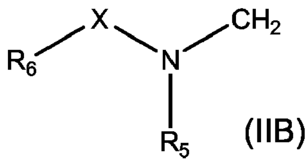
(IIB)

$R_5$ is hydrogen and X is CO, then $R_6$ is different from cycloalkyl ($C_3$-$C_6$) and aryl.

**2.** A compound according to claim 1 which has the formula:

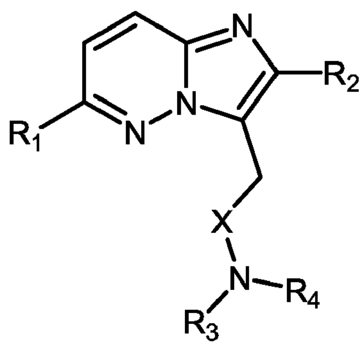
(IA)

wherein $R_1$, $R_2$, $R_3$, $R_4$ and X are as defined in claim 1.

**3.** A compound according to claim 1 which has the formula

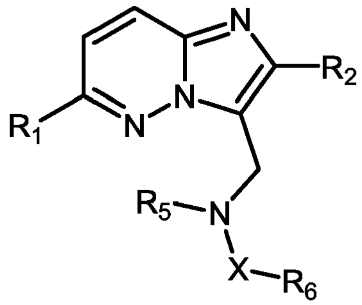

(IB)

wherein $R_1$, $R_2$, $R_5$, $R_6$ and X are as defined in claim 1.

**4.** A compound according to any of the preceding claims, wherein $R_1$ is a methyl, chlorine, methoxy, ethoxy, phenylthio or 1-pyrrolidinyl group and $R_2$ is a phenyl group or a phenyl group substituted in *para*- position by methyl, halogen, methoxy, nitro or trifluoromethyl.

**5.** A compound according to any of claims 1, 2 and 4, wherein X is CO; $R_3$ is is selected from the group consisting of hydrogen, linear alkyl($C_1$-$C_6$), phenyl optionally substituted by 1 to 5 Z groups, heteroaryl optionally substituted by 1 to 5 Z groups, amino, -NH-alkyl($C_1$-$C_6$), -N-dialkyl($C_1$-$C_6$), 1-pyrrolidinyl, 4-morpholinyl and 1-piperidinyl; and $R_4$ is selected from the group consisting of hydrogen, linear alkyl($C_1$-$C_6$), phenyl optionally substituted by 1 to 5 Z groups and heteroaryl optionally substituted by 1 to 5 Z groups; or both $R_3$ and $R_4$ can form, together with the nitrogen atom to which they are attached, a 5-6 membered heterocyclic ring optionally substituted by 1 to 5 Z groups; and Z is selected from the group consisting of methyl and methoxy.

**6.** A compound according to any of claims 1, 3-4, wherein X is CO; $R_5$ is selected from the group consisting of hydrogen and linear or branched alkyl ($C_1$-$C_6$); and $R_6$ is 5-isoxazolyl, 2-pyrazinyl, 2-quinoxalyl, 4-pyridyl and 2-thienyl.

**7.** A compound according to any of claims 1, 3-4, wherein X is CO, $R_5$ is hydrogen and $R_6$ is -$NR_7R_8$.

**8.** A compound according to claim 7, wherein $R_7$ is selected from the group consisting of hydrogen, methyl, ethyl, n-propyl, i-propyl, cyclopropyl, cyclopentyl, cyclohexyl, 2-propinyl and phenyl optionally substituted by 1 to 5 Z groups; and Z is selected from the group consisting of methyl, fluorine, chlorine, methoxy, thiomethoxy, acetyl and nitro.

**9.** A compound according to any of claims 1, 3-4, wherein X is CO, $R_5$ is hydrogen and $R_6$ is -$OR_7$.

**10.** A compound according to claim 9, wherein $R_7$ is selected from the group consisting of methyl, ethyl, n-propyl, i-propyl, n-butyl,i-butyl and phenyl substituted by 1 to 5 Z groups; and Z is selected from the group consisting of methyl, methoxy and nitro.

**11.** A compound according to any of claims 1,3-4, wherein X is $SO_2$, $R_5$ is hydrogen and $R_6$ is methyl, phenyl substituted by 1 to 5 Z groups, 2-thienyl, 1-methyl-1 H-imidazole-4-yl, 5-bromo-6-chloro-pyridine-3-yl or 3,5-dimethylisoxazole-4-yl; and Z is selected from the group consisting of methyl, chlorine, methoxy and nitro.

**12.** A compound according to claim 5, wherein said compound is selected from the group consisting of:

2-(6-Chloro-2-p-tolyl-imidazo[1,2-b]pyridazin-3-yl)-N,N-diethyl-acetamide;
2-(6-Chloro-2-p-tolyl-imidazo[1,2-b]pyridazin-3-yl)-N,N-dipropyl-acetamide;
N,N-Dibutyl-2-(6-chloro-2-p-tolyl-imidazo[1,2-b]pyridazin-3-yl)-acetamide;
2-(6-Chloro-2-p-tolyl-imidazo[1,2-b]pyridazin-3-yl)-1-piperidin-1-yl-ethanone;
2-(6-Chloro-2-p-tolyl-imidazo[1,2-b]pyridazin-3-yl)-1-morpholin-4-yl-ethanone;
2-(6-Chloro-2-p-tolyl-imidazo[1,2-b]pyridazin-3-yl)-1-pyrrolidin-1-yl-ethanone;
N, N-Diethyl-2-(6-pyrrolidin-1-yl-2-p-tolyl-imidazo[1,2-b]pyridazin-3-yl)-acetamide;
N,N-Diethyl-2-(6-methoxy-2-p-tolyl-imidazo[1,2-b]pyridazin-3-yl)-acetamide;
2-[2-(4-Bromo-phenyl)-6-ethoxy-imidazo[1,2-b]pyridazin-3-yl]-1-morpholin-4-yl-ethanone;
2-[2-(4-Bromo-phenyl)-6-ethoxy-imidazo[1,2-b]pyridazin-3-yl]-1-piperidin-1-yl-ethanone;
2-[2-(4-Bromo-phenyl)-6-ethoxy-imidazo[1,2-b]pyridazin-3-yl]-N,N-dibutylacetamide;

2-[2-(4-Bromo-phenyl)-6-ethoxy-imidazo[1,2-b]pyridazin-3-yl]-N,N-dipropylacetamide;
2-[2-(4-Bromo-phenyl)-6-ethoxy-imidazo[1,2-b]pyridazin-3-yl]-N,N-diethylacetamide;
2-[2-(4-Bromo-phenyl)-6-methyl-imidazo[1,2-b]pyridazin-3-yl]-1-morpholin-4-yl-ethanone;
2-[2-(4-Bromo-phenyl)-6-methyl-imidazo[1,2-b]pyridazin-3-yl]-1-piperidin-1-yl-ethanone;
2-[2-(4-Bromo-phenyl)-6-methyl-imidazo[1,2-b]pyridazin-3-yl]-N,N-dibutylacetamide;
2-[2-(4-Bromo-phenyl)-6-methyl-imidazo[1,2-b]pyridazin-3-yl]-N,N-dipropylacetamide;
2-[2-(4-Bromo-phenyl)-6-methyl-imidazo[1,2-b]pyridazin-3-yl]-N,N-diethylacetamide;
2-[2-(4-Chloro-phenyl)-6-methyl-imidazo[1,2-b]pyridazin-3-yl]-N,N-diethylacetamide;
2-[2-(4-Chloro-phenyl)-6-ethoxy-imidazo[1,2-b]pyridazin-3-yl]-N,N-diethylacetamide;
2-[2-(4-Chloro-phenyl)-6-methyl-imidazo[1,2-b]pyridazin-3-yl]-N,N-dipropylacetamide;
N,N-Dibutyl-2-[2-(4-chloro-phenyl)-6-methyl-imidazo[1,2-b]pyridazin-3-yl]-acetamide;
2-[2-(4-Chloro-phenyl)-6-ethoxy-imidazo[1,2-b]pyridazin-3-yl]-N,N-dipropylacetamide;
N,N-Dibutyl-2-[2-(4-chloro-phenyl)-6-ethoxy-imidazo[1,2-b]pyridazin-3-yl]-acetamide;
N,N-Diethyl-2-(6-methoxy-2-phenyl-imidazo[1,2-b]pyridazin-3-yl)-acetamide;
2-(6-Methoxy-2-phenyl-imidazo[1,2-b]pyridazin-3-yl)-N,N-dipropyl-acetamide;
N,N-Dibutyl-2-(6-methoxy-2-phenyl-imidazo[1,2-b]pyridazin-3-yl)-acetamide;
2-(6-Methoxy-2-phenyl-imidazo[1,2-b]pyridazin-3-yl)-1-morpholin-4-yl-ethanone;
2-(6-Ethoxy-2-phenyl-imidazo[1,2-b]pyridazin-3-yl)-N,N-diethyl-acetamide;
2-(6-Ethoxy-2-phenyl-imidazo[1,2-b]pyridazin-3-yl)-N,N-dipropyl-acetamide;
N,N-Dibutyl-2-(6-ethoxy-2-phenyl-imidazo[1,2-b]pyridazin-3-yl)-acetamide;
2-(6-Ethoxy-2-phenyl-imidazo[1,2-b]pyridazin-3-yl)-1-morpholin-4-yl-ethanone;
2-(6-Ethoxy-2-phenyl-imidazo[1,2-b]pyridazin-3-yl)-1-piperidin-1-yl-ethanone;
N,N-Diethyl-2-(6-methyl-2-phenyl-imidazo[1,2-b]pyridazin-3-yl)-acetamide;
2-(6-Methyl-2-phenyl-imidazo[1,2-b]pyridazin-3-yl)-N,N-dipropyl-acetamide;
N,N-Dibutyl-2-(6-methyl-2-phenyl-imidazo[1,2-b]pyridazin-3-yl)-acetamide;
2-(6-Methyl-2-phenyl-imidazo[1,2-b]pyridazin-3-yl)-1-morpholin-4-yl-ethanone;
2-(6-Methyl-2-phenyl-imidazo[1,2-b]pyridazin-3-yl)-1-pi peridin-1-yl-ethanone;
2-[2-(4-Fluoro-phenyl)-6-methyl-imidazo[1,2-b]pyridazin-3-yl]-N,N-dipropylacetamide;
N,N-Dibutyl-2-[2-(4-fluoro-phenyl)-6-methyl-imidazo[1,2-b]pyridazin-3-yl]-acetamide;
2-[2-(4-Fluoro-phenyl)-6-methoxy-imidazo[1,2-b]pyridazin-3-yl]-N,N-dipropylacetamide;
N,N-Dibutyl-2-[2-(4-fluoro-phenyl)-6-methoxy-imidazo[1,2-b]pyridazin-3-yl]-acetamide;
2-[6-Ethoxy-2-(4-fluoro-phenyl)-imidazo[1,2-b]pyridazin-3-yl]-N,N-dipropylacetamide;
N,N-Dibutyl-2-[6-ethoxy-2-(4-fluoro-phenyl)-imidazo[1,2-b]pyridazin-3-yl]-acetamide;
2-[6-Ethoxy-2-(4-fluoro-phenyl)-imidazo[1,2-b]pyridazin-3-yl]-1-morpholin-4-yl-ethanone;
N,N-Diethyl-2-(6-methoxy-2-p-tolyl-imidazo[1,2-b]pyridazin-3-yl)-acetamide;
2-(6-Methoxy-2-p-tolyl-imidazo[1,2-b]pyridazin-3-yl)-N,N-dipropyl-acetamide;
N,N-Dibutyl-2-(6-methoxy-2-p-tolyl-imidazo[1,2-b]pyridazin-3-yl)-acetamide;
2-(6-Methoxy-2-p-tolyl-imidazo[1,2-b]pyridazin-3-yl)-1-piperidin-1-yl-ethanone;
2-(6-Ethoxy-2-p-tolyl-imidazo[1,2-b]pyridazin-3-yl)-N,N-diethyl-acetamide;
2-(6-Ethoxy-2-p-tolyl-imidazo[1,2-b]pyridazin-3-yl)-N,N-dipropyl-acetamide;
N,N-Dibutyl-2-(6-ethoxy-2-p-tolyl-imidazo[1,2-b]pyridazin-3-yl)-acetamide;
2-(6-Ethoxy-2-p-tolyl-imidazo[1,2-b]pyridazin-3-yl)-1-pyrrolidin-1-yl-ethanone;
2-(6-Ethoxy-2-p-tolyl-imidazo[1,2-b]pyridazin-3-yl)-1-morpholin-4-yl-ethanone;
2-(6-Ethoxy-2-p-thoxy-imidazo[1,2-b]pyridazin-3-yl)-1-piperidin-1-yl-ethanone;
N,N-Diethyl-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-N,N-dipropyl-acetamide;
N,N-Dibutyl-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-1-pyrrolidin-1-yl-ethanone;
2-(6-Methyl-2-p-tolyl-imidazo[1,2-b]pyridazin-3-yl)-1-morpholin-4-yl-ethanone;
2-(6-Methyl-2-p-tolyl-imidazo[1,2-b]pyridazin-3-yl)-1-piperidin-1-yl-ethanone;
N,N-Diethyl-2-[2-(4-fluoro-phenyl)-6-methyl-imidazo[1,2-b]pyridazin-3-yl]-acetamide;
2-[2-(4-Fluoro-phenyl)-6-methyl-imidazo[1,2-b]pyridazin-3-yl]-1-piperidin-1-yl-ethanone;
N,N-Diethyl-2-[2-(4-fluoro-phenyl)-6-methoxy-imidazo[1,2-b]pyridazin-3-yl]-acetamide;
2-[2-(4-Fluoro-phenyl)-6-methoxy-imidazo[1,2-b]pyridazin-3-yl]-1-piperidin-1-yl-ethanone;
2-[6-Ethoxy-2-(4-fluoro-phenyl)-imidazo[1,2-b]pyridazin-3-yl]-N,N-diethylacetamide;
2-[6-Ethoxy-2-(4-fluoro-phenyl)-imidazo[1,2-b]pyridazin-3-yl]-1-piperidin-1-yl-ethanone;
2-[2-(4-Fluoro-phenyl)-6-methyl-imidazo[1,2-b]pyridazin-3-yl]-1-morpholin-4-yl-ethanone;
2-[2-(4-Fluoro-phenyl)-6-methoxy-imidazo[1,2-b]pyridazin-3-yl]-1-morpholin-4-yl-ethanone;

N,N-Diethyl-2-[2-(4-methoxy-phenyl)-6-methyl-imidazo[1,2-b]pyridazin-3-yl]-acetamide;
2-[2-(4-Methoxy-phenyl)-6-methyl-imidazo[1,2-b]pyridazin-3-yl]-N,N-dipropylacetamide;
N,N-Dibutyl-2-[2-(4-methoxy-phenyl)-6-methyl-imidazo[1,2-b]pyridazin-3-yl]-acetamide;
2-[2-(4-Methoxy-phenyl)-6-methyl-imidazo[1 ,2-b]pyridazin-3-yl]-l -piperidin-l - yl-ethanone;
2-[2-(4-Methoxy-phenyl)-6-methyl-imidazo[1,2-b]pyridazin-3-yl]-1-morpholin-4-yl-ethanone;
N,N-Diethyl-2-[6-methoxy-2-(4-methoxy-phenyl)-imidazo[1,2-b]pyridazin-3-yl]-acetamide;
2-[6-Methoxy-2-(4-methoxy-phenyl)-imidazo[1,2-b]pyridazin-3-yl]-N,N-dipropyl-acetamide;
N,N-Dibutyl-2-[6-methoxy-2-(4-methoxy-phenyl)-imidazo[1,2-b]pyridazin-3-yl]-acetamide;
2-[6-Methoxy-2-(4-methoxy-phenyl)-imidazo[1,2-b]pyridazin-3-yl]-1-piperidin-1-yl-ethanone;
2-[6-Methoxy-2-(4-methoxy-phenyl)-imidazo[1,2-b]pyridazin-3-yl]-1-morpholin-4-yl-ethanone;
Acetic acid 2-{[2-(6-methyl-2-p-tolyl-imidazo[1,2-b]pyridazin-3-yl)-acetyl]-propyl-amino}-ethyl ester;
1-(3,5-Dimethyl-piperidin-1-yl)-2-(6-methyl-2-p-tolyl-imidazo[1,2-b]pyridazin-3-yl)-ethanone;
N-Cyclopropylmethyl-2-(6-methyl-2-p-tolyl-imidazo[1,2-b]pyridazin-3-yl)-N-propyl-acetamide;
2-(6-Methyl-2-p-tolyl-imidazo[1,2-b]pyridazin-3-yl)-N-thiazol-2-yl-acetamide;
N,N-Diisopropyl-2-(6-methyl-2-p-tolyl-imidazo[1,2-b]pyridazin-3-yl)-acetamide;
N-Cyclohexyl-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-N-phenyl-acetamide;
2-(6-Methyl-2-p-tolyl-imidazo[1,2-b]pyridazin-3-yl)-N-p-tolyl-acetamide;
2-(6-Methyl-2-p-tolyl-imidazo[1,2-b]pyridazin-3-yl)-N-pyridin-2-yl-acetamide;
2-(6-Methyl-2-p-tolyl-imidazo[1,2-b]pyridazin-3-yl)-N-pyridin-2-ylmethylacetamide;
N-(3,5-Dimethyl-isoxazol-4-yl)-2-(6-methyl-2-p-tolyl-imidazo[1,2-b]pyridazin-3-yl)-acetamide;
N-Cyclopentyl-2-(6-methyl-2-p-tolyl-imidazo[1,2-b]pyridazin-3-yl)-acetamide;
N,N-Diallyl-2-(6-methyl-2-p-tolyl-imidazo[1,2-b]pyridazin-3-yl)-acetamide;
N-Cyclopropyl-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-N-quinolin-2-yl-acetamide;
N-(5-Methyl-isoxazol-3-yl)-2-(6-methyl-2-p-tolyl-imidazo[1,2-b]pyridazin-3-yl)-acetamide;
N-(4-Methoxy-phenyl)-2-(6-methyl-2-p-tolyl-imidazo[1,2-b]pyridazin-3-yl)-acetamide;
N-(3-Methyl-isoxazol-5-yl)-2-(6-methyl-2-p-tolyl-imidazo[1,2-b]pyridazin-3-yl)-acetamide;
2-(6-Methyl-2-p-tolyl-imidazo[1,2-b]pyridazin-3-yl)-N-[1,3,4]thiadiazol-2-yl-acetamide;
[2-(4-Fluoro-phenyl)-6-pyrrolidin-1-yl-imidazo[1,2-b]pyridazin-3-yl]-acetic acid hydrazide;
[2-(4-Bromo-phenyl)-6-methyl-imidazo[1,2-b]pyridazin-3-yl]-acetic acid hydrazide;
[2-(4-Methoxy-phenyl)-6-methyl-imidazo[1,2-b]pyridazin-3-yl]-acetic acid hydrazide;
[2-(4-Chloro-phenyl)-6-methyl-imidazo[1,2-b]pyridazin-3-yl]-acetic acid hydrazide;
[2-(4-Fluoro-phenyl)-6-methyl-imidazo[1,2-b]pyridazin-3-yl]-acetic acid hydrazide;
(6-Methyl-2-phenyl-imidazo[1,2-b]pyridazin-3-yl)-acetic acid hydrazide;
2-(6-Methyl-2-p-tolyl-imidazo[1,2-b]pyridazin-3-yl)-N-morpholin-4-yl-acetamide;
2-(6-Methyl-2-p-tolyl-imidazo[1,2-b]pyridazin-3-yl)-N-piperidin-1-yl-acetamide; and
(6-Methyl-2-p-tolyl-imidazo[1,2-b]pyridazin-3-yl)-acetic acid N',N'-dimethylhydrazide.

**13.** A compound according to claim 6, wherein said compound is selected from the group consisting of:

Thiophene-2-carboxylic acid (6-chloro-2-p-tolyl-imidazo[1,2-b]pyridazin-3-ylmethyl)-amide;
N-(6-Chloro-2-p-tolyl-imidazo[1,2-b]pyridazin-3-ylmethyl)-isonicotinamide;
Quinoxaline-2-carboxylic acid (6-chloro-2-p-tolyl-imidazo[1,2-b]pyridazin-3-ylmethyl)-amide;
Pyrazine-2-carboxylic acid (6-chloro-2-p-tolyl-imidazo[1,2-b]pyridazin-3-ylmethyl)-amide;
Isoxazole-5-carboxylic acid (6-chloro-2-p-tolyl-imidazo[1,2-b]pyridazin-3-ylmethyl)-amide;
Thiophene-2-carboxylic acid (6-phenylsulfanyl-2-p-tolyl-imidazo[1,2-b]pyridazin-3-ylmethyl)-amide;
lsoxazole-5-carboxylic acid (6-phenylsulfanyl-2-p-tolyl-imidazo[1,2-b]pyridazin-3-ylmethyl)-amide;
Pyrazine-2-carboxylic acid (6-phenylsulfanyl-2-p-tolyl-imidazo[1,2-b]pyridazin-3-ylmethyl)-amide;
Quinoxaline-2-carboxylic acid (6-phenylsulfanyl-2-p-tolyl-imidazo[1,2-b]pyridazin-3-ylmethyl)-amide;
N-(6-Phenylsulfanyl-2-p-tolyl-imidazo[1,2-b]pyridazin-3-ylmethyl)-isonicotinamide;
N-(2-p-Tolyl-imidazo[1,2-b]pyridazin-3-ylmethyl)-acetamide;
N-(6-Pyrrolidin-1-yl-2-p-tolyl-imidazo[1,2-b] pyridazin-3-ylmethyl)-acetamide;
N-(6-Piperidin-1-yl-2-p-tolyl-imidazo[1,2-b]pyridazin-3-ylmethyl)-acetamide;
N-(6-Morpholin-4-yl-2-p-tolyl-imidazo[1,2-b]pyridazin-3-ylmethyl)-acetamide;
N-[6-(4-Methyl-piperazin-1-yl)-2-p-tolyl-imidazo[1,2-b]pyridazin-3-ylmethyl]-acetamide;
N-[2-(4-Bromo-phenyl)-6-pyrrolidin-1-yl-imidazo[1,2-b]pyridazin-3-ylmethyl]-acetamide;
N-(2-Phenyl-6-pyrrolidin-1-yl-imidazo[1,2-b]pyridazin-3-ylmethyl)-acetamide;

N-[2-(4-Fluoro-phenyl)-6-pyrrolidin-1-yl-imidazo[1,2-b]pyridazin-3-ylmethyl]-acetamide;
N-(2-Furan-2-yl-6-pyrrolidin-1-yl-imidazo[1,2-b]pyridazin-3-ylmethyl)-acetamide;
N-[2-(4-Methoxy-phenyl)-6-pyrrolidin-1-yl-imidazo[1,2-b]pyridazin-3-ylmethyl]-acetamide;
N-[2-(4-Nitro-phenyl)-6-pyrrolidin-1-yl-imidazo[1,2-b]pyridazin-3-ylmethyl]-acetamide;
N-[6-Pyrrolidin-1-yl-2-(4-trifluoromethyl-phenyl)-imidazo[1,2-b]pyridazin-3-ylmethyl]-acetamide;
Pyrazine-2-carboxylic acid (6-methoxy-2-phenyl-imidazo[1,2-b]pyridazin-3-ylmethyl)-amide;
Isoxazole-5-carboxylic acid (6-methoxy-2-phenyl-imidazo[1,2-b]pyridazin-3-ylmethyl)-amide;
Pyrazine-2-carboxylic acid (2-phenyl-6-pyrrolidin-1-yl-imidazo[1,2-b]pyridazin-3-ylmethyl)-amide;
Isoxazole-5-carboxylic acid (2-phenyl-6-pyrrolidin-1-yl-imidazo[1,2-b]pyridazin-3-ylmethyl)-amide;
N-(6-Phenyl-2-p-tolyl-imidazo[1,2-b]pyridazin-3-ylmethyl)-acetamide;
Pyrazine-2-carboxylic acid [2-(4-fluoro-phenyl)-6-methoxy-imidazo[1,2-b]pyridazin-3-ylmethyl]-amide;
Isoxazole-5-carboxylic acid [2-(4-fluoro-phenyl)-6-methoxy-imidazo[1,2-b]pyridazin-3-ylmethyl]-amide;
Isoxazole-5-carboxylic acid [2-(4-chloro-phenyl)-6-methoxy-imidazo[1,2-b]pyridazin-3-ylmethyl]-amide;
Pyrazine-2-carboxylic acid [2-(4-bromo-phenyl)-6-methoxy-imidazo[1,2-b]pyridazin-3-ylmethyl]-amide;
Pyrazine-2-carboxylic acid [2-(4-bromo-phenyl)-6-ethoxy-imidazo[1,2-b]pyridazin-3-ylmethyl]-amide;
Pyrazine-2-carboxylic acid [2-(4-chloro-phenyl)-6-pyrrolidin-1-yl-imidazo[1,2-b]pyridazin-3-ylmethyl]-amide; and
Pyrazine-2-carboxylic acid [2-(4-chloro-phenyl)-6-methoxy-imidazo[1,2-b]pyridazin-3-ylmethyl]-amide.

**14.** A compound according to claim 8, wherein said compound is selected from the group consisting of:

1-(6-Chloro-2-p-tolyl-imidazo[1,2-b]pyridazin-3-ylmethyl)-3-cyclopentyl-urea;
1-(4-Acetyl-phenyl)-3-(6-chloro-2-p-tolyl-imidazo[1,2-b]pyridazin-3-ylmethyl)-urea;
1-(6-Chloro-2-p-tolyl-imidazo[1,2-b]pyridazin-3-ylmethyl)-3-(4-nitro-phenyl)-urea;
1-(4-Chloro-phenyl)-3-(6-chloro-2-p-tolyl-imidazo[1,2-b]pyridazin-3-ylmethyl)-urea;
1-(6-Chloro-2-p-tolyl-imidazo[1,2-b]pyridazin-3-ylmethyl)-3-(4-fluoro-phenyl)-urea;
1-(6-Chloro-2-p-tolyl-imidazo[1,2-b]pyridazin-3-ylmethyl)-3-p-tolyl-urea;
1-(6-Chloro-2-p-tolyl-imidazo[1,2-b]pyridazin-3-ylmethyl)-3-(4-methylsulfanylphenyl)-urea;
1-(6-Chloro-2-p-tolyl-imidazo[1,2-b]pyridazin-3-ylmethyl)-3-(4-methoxy-phenyl)-urea;
1-(6-Chloro-2-p-tolyl-imidazo[1,2-b]pyridazin-3-ylmethyl)-3-phenyl-urea;
1-(6-Chloro-2-p-tolyl-imidazo[1,2-b]pyridazin-3-ylmethyl)-3-isopropyl-urea;
1-(6-Chloro-2-p-tolyl-imidazo[1,2-b]pyridazin-3-ylmethyl)-3-ethyl-urea;
1-Ethyl-3-(6-phenylsulfanyl-2-p-tolyl-imidazo[1,2-b]pyridazin-3-ylmethyl)-urea;
1-Phenyl-3-(6-phenylsulfanyl-2-p-tolyl-imidazo[1,2-b]pyridazin-3-ylmethyl)-urea;
1-(4-Fluoro-phenyl)-3-(6-phenylsulfanyl-2-p-tolyl-imidazo[1,2-b]pyridazin-3-ylmethyl)-urea;
1-Cyclopentyl-3-(6-phenylsulfanyl-2-p-tolyl-imidazo[1,2-b]pyridazin-3-ylmethyl)-urea;
1-Isopropyl-3-(6-phenylsulfanyl-2-p-tolyl-imidazo[1,2-b]pyridazin-3-ylmethyl)-urea;
1-(4-Acetyl-phenyl)-3-(6-phenylsulfanyl-2-p-tolyl-imidazo[1,2-b]pyridazin-3-ylmethyl)-urea;
1-Ethyl-3-(6-methoxy-2-phenyl-imidazo[1,2-b]pyridazin-3-ylmethyl)-urea;
1-Ethyl-3-[2-(4-fluoro-phenyl)-6-methoxy-imidazo[1,2-b]pyridazin-3-ylmethyl]-urea;
1-[2-(4-Chloro-phenyl)-6-methoxy-imidazo[1,2-b]pyridazin-3-ylmethyl]-3-ethylurea;
1-[2-(4-Bromo-phenyl)-6-methoxy-imidazo[1,2-b]pyridazin-3-ylmethyl]-3-ethylurea;
1-[2-(4-Bromo-phenyl)-6-ethoxy-imidazo[1,2-b]pyridazin-3-ylmethyl]-3-ethylurea; and
1-[2-(4-Chloro-phenyl)-6-pyrrolidin-1-yl-imidazo[1,2-b]pyridazin-3-ylmethyl]-3-ethyl-urea.

**15.** A compound according to claim 10, wherein said compound is selected from the group consisting of:

(6-Chloro-2-p-tolyl-imidazo[1,2-b]pyridazin-3-ylmethyl)-carbamic acid ethyl ester;
(6-Chloro-2-p-tolyl-imidazo[1,2-b]pyridazin-3-ylmethyl)-carbamic acid prop-2-ynyl ester;
(6-Chloro-2-p-tolyl-imidazo[1,2-b]pyridazin-3-ylmethyl)-carbamic acid 2,2,2-trichloro-ethyl ester;
(6-Chloro-2-p-tolyl-imidazo[1,2-b]pyridazin-3-ylmethyl)-carbamic acid phenyl ester;
(6-Chloro-2-p-tolyl-imidazo[1,2-b]pyridazin-3-ylmethyl)-carbamic acid p-tolyl ester;
(6-Chloro-2-p-tolyl-imidazo[1,2-b]pyridazin-3-ylmethyl)-carbamic acid 4-methoxy-phenyl ester;
(6-Chloro-2-p-tolyl-imidazo[1,2-b]pyridazin-3-ylmethyl)-carbamic acid 4-nitro-phenyl ester;
(6-Chloro-2-p-tolyl-imidazo[1,2-b]pyridazin-3-ylmethyl)-carbamic acid benzyl ester;
(6-Chloro-2-p-tolyl-imidazo[1,2-b]pyridazin-3-ylmethyl)-carbamic acid methyl ester;
(6-Chloro-2-p-tolyl-imidazo[1,2-b]pyridazin-3-ylmethyl)-carbamic acid isobutyl ester;
(6-Chloro-2-p-tolyl-imidazo[1,2-b]pyridazin-3-ylmethyl)-carbamic acid butyl ester;

(6-Phenylsulfanyl-2-p-tolyl-imidazo[1,2-b]pyridazin-3-ylmethyl)-carbamic acid ethyl ester;
(6-Phenylsulfanyl-2-p-tolyl-imidazo[1,2-b]pyridazin-3-ylmethyl)-carbamic acid methyl ester;
(6-Phenylsulfanyl-2-p-tolyl-imidazo[1,2-b]pyridazin-3-ylmethyl)-carbamic acid phenyl ester;
(6-Phenylsulfanyl-2-p-tolyl-imidazo[1,2-b]pyridazin-3-ylmethyl)-carbamic acid p-tolyl ester;
(6-Phenylsulfanyl-2-p-tolyl-imidazo[1,2-b]pyridazin-3-ylmethyl)-carbamic acid 4-methoxy-phenyl ester;
(6-Phenylsulfanyl-2-p-tolyl-imidazo[1,2-b]pyridazin-3-ylmethyl)-carbamic acid benzyl ester;
(6-Phenylsulfanyl-2-p-tolyl-imidazo[1,2-b]pyridazin-3-ylmethyl)-carbamic acid isopropyl ester;
(6-Phenylsulfanyl-2-p-tolyl-imidazo[1,2-b]pyridazin-3-ylmethyl)-carbamic acid butyl ester;
(6-Phenylsulfanyl-2-p-tolyl-imidazo[1,2-b]pyridazin-3-ylmethyl)-carbamic acid isobutyl ester;
(6-Methoxy-2-phenyl-imidazo[1,2-b]pyridazin-3-ylmethyl)-carbamic acid isobutyl ester;
(6-Methoxy-2-phenyl-imidazo[1,2-b]pyridazin-3-ylmethyl)-carbamic acid ethyl ester;
(6-Methoxy-2-phenyl-imidazo[1,2-b]pyridazin-3-ylmethyl)-carbamic acid methyl ester;
(2-Phenyl-6-pyrrolidin-1-yl-imidazo[1,2-b]pyridazin-3-ylmethyl)-carbamic acid ethyl ester;
[2-(4-Fluoro-phenyl)-6-methoxy-imidazo[1,2-b]pyridazin-3-ylmethyl]-carbamic acid isobutyl ester;
[2-(4-Fluoro-phenyl)-6-methoxy-imidazo[1,2-b]pyridazin-3-ylmethyl]-carbamic acid ethyl ester;
[2-(4-Fluoro-phenyl)-6-methoxy-imidazo[1,2-b]pyridazin-3-ylmethyl]-carbamic acid methyl ester;
[2-(4-Chloro-phenyl)-6-methoxy-imidazo[1,2-b]pyridazin-3-ylmethyl]-carbamic acid methyl ester;
[2-(4-Chloro-phenyl)-6-methoxy-imidazo[1,2-b]pyridazin-3-ylmethyl]-carbamic acid isobutyl ester;
[2-(4-Chloro-phenyl)-6-methoxy-imidazo[1,2-b]pyridazin-3-ylmethyl]-carbamic acid ethyl ester;
[2-(4-Bromo-phenyl)-6-methoxy-imidazo[1,2-b]pyridazin-3-ylmethyl]-carbamic acid isobutyl ester;
[2-(4-Bromo-phenyl)-6-methoxy-imidazo[1,2-b]pyridazin-3-ylmethyl]-carbamic acid ethyl ester;
[2-(4-Bromo-phenyl)-6-methoxy-imidazo[1,2-b]pyridazin-3-ylmethyl]-carbamic acid methyl ester;
[2-(4-Bromo-phenyl)-6-ethoxy-imidazo[1,2-b]pyridazin-3-ylmethyl]-carbamic acid isobutyl ester;
[2-(4-Bromo-phenyl)-6-ethoxy-imidazo[1,2-b]pyridazin-3-ylmethyl]-carbamic acid ethyl ester;
[2-(4-Bromo-phenyl)-6-ethoxy-imidazo[1,2-b]pyridazin-3-ylmethyl]-carbamic acid methyl ester;
[2-(4-Chloro-phenyl)-6-pyrrolidin-1-yl-imidazo[1,2-b]pyridazin-3-ylmethyl]-carbamic acid methyl ester;
[2-(4-Chloro-phenyl)-6-pyrrolidin-1-yl-imidazo[1,2-b]pyridazin-3-ylmethyl]-carbamic acid ethyl ester; and
[2-(4-Chloro-phenyl)-6-pyrrolidin-1-yl-imidazo[1,2-b]pyridazin-3-ylmethyl]-carbamic acid isobutyl ester.

**16.** A compound according to claim 11, wherein said compound is selected from the group consisting of:

N-(6-Phenylsulfanyl-2-p-tolyl-imidazo[1,2-b]pyridazin-3-ylmethyl)-methanesulfonamide;
N-(6-Phenylsulfanyl-2-p-tolyl-imidazo[1,2-b]pyridazin-3-ylmethyl)-benzenesulfonamide;
4-Methyl-N-(6-phenylsulfanyl-2-p-tolyl-imidazo[1,2-b]pyridazin-3-ylmethyl)-benzenesulfonamide;
4-Methoxy-N-(6-phenylsulfanyl-2-p-tolyl-imidazo[1,2-b]pyridazin-3-ylmethyl)-benzenesulfonamide;
4-Chloro-N-(6-phenylsulfanyl-2-p-tolyl-imidazo[1,2-b]pyridazin-3-ylmethyl)-benzenesulfonamide;
4-Nitro-N-(6-phenylsulfanyl-2-p-tolyl-imidazo[1,2-b]pyridazin-3-ylmethyl)-benzenesulfonamide;
Thiophene-2-sulfonic acid (6-phenylsulfanyl-2-p-tolyl-imidazo[1,2-b]pyridazin-3-ylmethyl)-amide;
1-Methyl-1 H-imidazole-4-sulfonic acid (6-phenylsulfanyl-2-p-tolyl-imidazo[1,2-b]pyridazin-3-ylmethyl)-amide;
5-Bromo-6-chloro-pyridine-3-sulfonic acid (6-phenylsulfanyl-2-p-tolylimidazo[1,2-b]pyridazin-3-ylmethyl)-amide; and
3,5-Dimethyl-isoxazole-4-sulfonic acid (6-phenylsulfanyl-2-p-tolyl-imidazo[1,2-b]pyridazin-3-ylmethyl)-amide.

**17.** A process for preparing a compound of formula (I), according to claim 1, comprising reacting an intermediate (III):

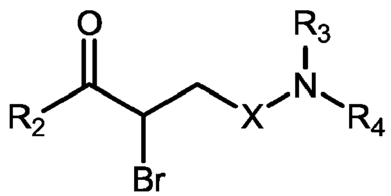

**(III)**

wherein
$R_2$, $R_3$ and $R_4$ are as defined in any of the preceding claims, with a substituted 3-aminopyridazine (XI):

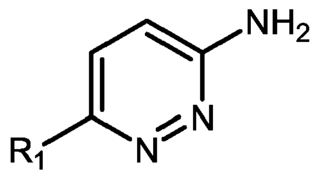

(XI)

wherein $R_1$ is as defined in (I).

**18.** A process for preparing a compound of formula (I) when X is CO, according to claim 1, comprising reacting an intermediate (IV):

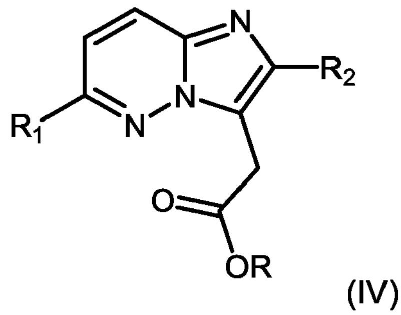

(IV)

wherein R is a linear or branched alkyl($C_1$-$C_6$) group and $R_1$ and $R_2$ are as defined in (I), with a compound $HNR_3R_4$ wherein $R_3$ and $R_4$ are as defined in (I).

**19.** The process of claim 18 wherein R is methyl.

**20.** A process for preparing a compound of formula (IB) according to claim 3 when $R_5$ is hydrogen, $R_6$ is selected from the group consisting of haloalkyl($C_2$-$C_6$), , cycloalkyl($C_3$-$C_6$)alkyl($C_1$-$C_6$), alkynyl($C_2$-$C_6$)alkyl($C_1$-$C_6$), alkyl ($C_1$-$C_6$)-O-alkyl($C_1$-$C_6$), alkyl($C_1$-$C_6$)-NH-alkyl($C_1$-$C_6$), alkyl($C_1$-$C_6$)-N(dialkyl($C_1$-$C_6$)), and X is CO, according to claim 1, comprising reacting an intermediate (V):

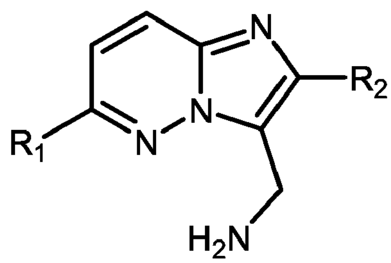

(V)

wherein $R_1$ and $R_2$ are as defined in any of the preceding claims , with an acyl chloride of formula $R_6COCl$, wherein $R_6$ is as defined above.

**21.** A process for preparing a compound of formula (I) according to claim 1 when $R_5$ is hydrogen, $R_6$ is $HNR_7$ and X is CO, according to claim 1, comprising reacting an intermediate (V), according to claim 18, with an isocyanate of formula $R_7NCO$, wherein $R_7$ is as defined above.

**22.** A process for preparing a compound of formula (I) according to claim 1 when $R_5$ is hydrogen, $R_6$ is $OR_7$ and X is CO, according to claim 1, comprising reacting an intermediate (V), according to claim 20, with a chloroformiate of

formula $R_7OCOCl$, wherein $R_7$ is as defined above.

**23.** A process for preparing a compound of formula (I) according to claim 3, when $R_5$ is hydrogen, $R_6$ is selected from the group consisting of haloalkyl($C_2$-$C_6$), cycloalkyl($C_3$-$C_6$), cydoalkyl($C_3$-$C_6$)alkyl($C_1$-$C_6$), alkynyl($C_2$-$C_6$)alkyl ($C_1$-$C_6$), alkyl($C_1$-$C_6$)-O-alkyl($C_1$-$C_6$), alkyl($C_1$-$C_6$)-NH-alkyl($C_1$-$C_6$), alkyl($C_1$-$C_6$)-N(dialkyl($C_1$-$C_6$)), and X is $SO_2$, comprising reacting an intermediate (V), according to claim 20, with a sulfonyl chloride of formula $R_6SO_2Cl$, wherein $R_6$ is as defined above.

**24.** A process for preparing an intermediate (V), according to claim 20, comprising reacting in the adequate acid conditions, an intermediate (VI):

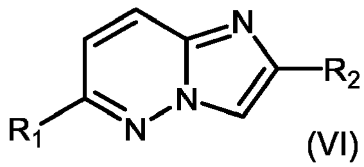

(VI)

wherein $R_1$ and $R_2$ are as defined for (V), with an acetamide intermediate compound (VII):

$CH_3CONHCH_2Q$ (VII)

wherein Q is selected from the group consisting of -OH, -Oalkyl($C_1$-$C_3$), - $N^+$(alkyl($C_1$-$C_3$))$_3Cl^-$, -$N^+$(alkyl($C_1$-$C_3$))$_3Br^-$ and -$N^+$(alkyl($C_1$-$C_3$))$_3I^-$, and then hydrolyzing the obtained intermediate (VIII):

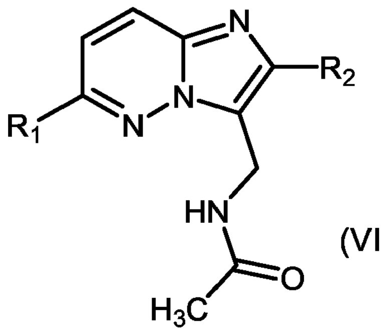

(VIII)

wherein $R_1$ and $R_2$ are as defined above.

**25.** The process of claim 24 wherein Q is -OH.

**26.** An imidazo[1,2-b]pyridazine compound of formula (V):

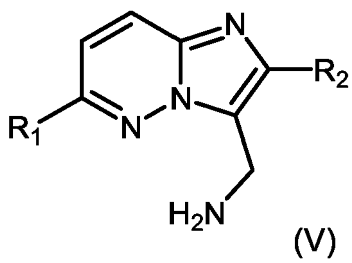

(V)

as well as pharmaceutically acceptable salts thereof, wherein $R_1$ and $R_2$ are as defined in claim 1, and with the proviso that $R_1$ may not be chlorine when $R_2$ is hydrogen.

**27.** The compound according to claim 26, wherein $R_1$ is methyl, chlorine, methoxy, ethoxy, thiophenoxy or 1-pyrrolidinyl group and $R_2$ is a phenyl group or a phenyl group substituted in *para-* position by methyl, halogen, methoxy, nitro or trifluoromethyl.

**28.** An imidazo[1,2-b]pyridazine compound of formula (IV):

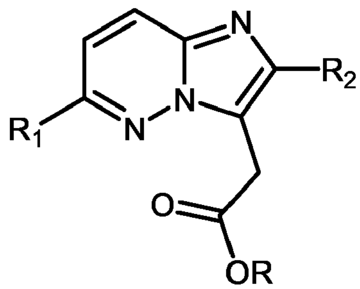

(IV)

as well as pharmaceutically acceptable salts thereof, wherein R is a linear or branched alkyl($C_1$-$C_6$) and $R_1$ and $R_2$ are as defined in claim 1.

**29.** A compound according to claim 28, wherein R is methyl, $R_1$ is methyl, chlorine, methoxy, ethoxy, thiophenoxy or 1-pyrrolidinyl group and $R_2$ is a phenyl group or a phenyl group substituted in *para-* position by methyl, halogen, methoxy, nitro or trifluoromethyl.

**30.** Use of a compound according to claim 1 for the preparation of a medicament for treating or preventing diseases associated with the $GABA_A$ receptor modulation in a human or non-human mammal in need thereof.

**31.** The use of claim 30 wherein the $GABA_A$ receptor is the $\alpha_1$-$GABA_A$ receptor.

**32.** The use of claim 30 wherein the $GABA_A$ receptor is the $\alpha_2$-$GABA_A$ receptor.

**33.** Use of a compound of claim 1 for the preparation of a medicament for treating or preventing anxiety in a human or non-human mammal in need thereof.

**34.** Use of a compound of claim 1 for the preparation of a medicament for treating or preventing epilepsy in a human or non-human mammal in need thereof.

**35.** Use of a compound of claim 1 for the preparation of a medicament for treating or preventing sleep disorders in a human or non-human mammal in need thereof.

**36.** Use of a compound of claim 1 for the preparation of a medicament for treating or preventing insomnia in a human or non-human mammal in need thereof.

**37.** Use of a compound of claim 1 for the preparation of a medicament for inducing sedation-hypnosis in a human or non-human mammal in need thereof.

**38.** Use of a compound of claim 1 for the preparation of a medicament for inducing anesthesia in a human or non-human mammal in need thereof.

**39.** Use of a compound of claim 1 for the preparation of a medicament for modulating the necessary time to induce sleep and its duration in a human or non-human mammal in need thereof.

**40.** Use of a compound of claim 1 for the preparation of a medicament for inducing muscle relaxation in a human or non-human mammal in need thereof.

**41.** A pharmaceutical composition comprising a therapeutically effective amount of a compound as defined in claim 1, together with appropriate amounts of pharmaceutical excipients or carriers.

European Patent Office

# EUROPEAN SEARCH REPORT

Application Number
EP 06 11 1899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BARLIN, G.B. ET AL.: "Imidazo[1,2-b]pyridazines. X. Syntheses and Central Nervous System Activities of Some 3-(Acetamido, benzamido, substituted benzamino or dimethylamino)methyl-2-(phenyl or substituted phenyl)-6-(halogeno, alkylthio, alkoxy, phenylthio, phenoxy, benzylthio or benzyloxy)imidazo[1,2-b]pyridazines." AUSTRALIAN JOURNAL OF CHEMISTRY, vol. 45, 1992, pages 731-749, XP009066843 * page 733; figure 1 * * page 732, paragraph 1 * * pages 734-735; table 1 * ----- | 1,2,4,5, 12-19, 28-41 | INV. C07D487/04 A61K31/5025 A61P25/22 A61P25/08 A61P25/20 |
| Y | WO 89/01333 A (THE AUSTRALIAN NATIONAL UNIVERSITY) 23 February 1989 (1989-02-23) * abstract * * claims 1-15 * * pages 55-59; table 3 * ----- | 1,2,4,5, 12-19, 28-41 | |
| | | | TECHNICAL FIELDS SEARCHED (IPC) |
| Y | TRAPANI G: "Novel 2-Phenylimidazo[1,2-a]pyridine Derivatives as Potent and Selective Ligands for Peripheral Benzodiazepine Receptors: Synthesis, Binding Affinity, and in Vivo Studies" JOURNAL OF MEDICINAL CHEMISTRY, AMERICAN CHEMICAL SOCIETY. WASHINGTON, US, vol. 42, 1999, pages 3934-3941, XP002154069 ISSN: 0022-2623 * abstract * * page 3935; table 1 * ----- -/-- | 1,2,4,5, 12-19, 28-41 | C07D A61K A61P |

~~The present search report has been drawn up for all claims~~

| Place of search | Date of completion of the search | Examiner |
|---|---|---|
| Munich | 7 June 2006 | Marzi, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 11 1899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE BEILSTEIN CROSSFIRE Beilstein Institute of Organic Chemistry; BRN: 9996954 XP002383952 * abstract * | 28 | |
| X | SACCHI A ET AL: "Research on heterocyclic compounds, XLI. 2-Phenylimidazo[1,2-b]pyrida zine-3-acetic derivatives: synthesis and anti-inflammatory activity" EUROPEAN JOURNAL OF MEDICINAL CHEMISTRY, EDITIONS SCIENTIFIQUE ELSEVIER, PARIS, FR, vol. 34, no. 11, November 1999 (1999-11), pages 1003-1008, XP004330438 ISSN: 0223-5234 * page 1004; figure 2; compounds 7A, 7B, 7C * | 28 | |

TECHNICAL FIELDS SEARCHED (IPC)

~~The present search report has been drawn up for all claims~~

| Place of search | Date of completion of the search | Examiner |
|---|---|---|
| Munich | 7 June 2006 | Marzi, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number

EP 06 11 1899

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

see annex

European Patent Office

**LACK OF UNITY OF INVENTION SHEET B**

Application Number
EP 06 11 1899

The Search Division considers that the present European patent application does not comply with the requirements of unity of invention and relates to several inventions or groups of inventions, namely:

1. claims: 1(part), 2, 4(part), 5, 12-16(part), 17-19, 28-29, 30-41(part)

   Compounds characterized in that Y is a group of the formula (IIA), their use, processes of preparation and intermediates thereof

   ---

2. claims: 1(part), 3-4(part), 12-16(part), 20, 23(part), 24-25, 26-27(part), 30-41(part)

   Compounds characterized in that Y is a group of formula (IIB) and R6 is haloalkyl(C2-C6), cycloalkyl(C3-C6)alkyl(C1-C6), alkynyl(C2-C6)alkyl(C1-C6), alkenyl(C2-C6)alkyl(C1-C6), alkyl(C1-C6)-O-alkyl(C1-C6) and alkyl(C1-C6)-NH-alkyl(C1-C6) , alkyl(C1-C6)-N-(dialkyl(C1-C6)), their use, processes and intermediates thereof.

   ---

3. claims: 1(part), 3-4(part), 11-16(part), 23(part), 26-27(part), 30-41(part)

   Compounds characterized in that Y is a group of formula (IIB) and R6 is cycloalkyl(C3-C6) or aryl, their use, process of preparation and intermediates thereof

   ---

4. claims: 1(part), 3-4(part), 7-10, 12-16(part), 21-22, 26-27(part), 30-41(part)

   Compounds characterized in that Y is a group of formula (IIB) and R6 is OR7, NHR7, NR7R8, their use, process of preparation and intermediates thereof.

   ---

5. claims: 1(part), 3-4(part), 6, 11-16(part), 26-27(part), 30-41(part)

   Compounds characterized in that Y is a group of formula (IIB) and R6 is heteroaryl, their use, process of preparation and intermediates thereof.

   ---

## ANNEX TO THE EUROPEAN SEARCH REPORT ON EUROPEAN PATENT APPLICATION NO. EP 06 11 1899

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2006

| Patent document cited in search report | Publication date | Patent family member(s) | Publication date |
|---|---|---|---|
| WO 8901333 A | 23-02-1989 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8901333 A
- US 4382938 A


### Non-patent literature cited in the description


- **H. MÖHLER et al.** *J. Pharmacol. Exp. Ther.,* 2002, vol. 300, 2-8
- **H. MÖHLER et al.** *Curr. Opin. Pharmacol.,* 2001, vol. 1, 22-25
- **U. RUDOLPH et al.** *Nature,* 1999, vol. 401, 796-800
- **D.J. NUTT et al.** *Br. J. Psychiatry,* 2001, vol. 179, 390-396
- **C. F. P. GEORGE.** *The Lancet,* 2001, vol. 358, 1623-1626
- **BARLIN, G. V.** *Australian Journal of Chemistry,* 1992, vol. 45 (4), 731-49
- **J. LAMEH et al.** *Prog. Neuro-Psychopharmacol. Biol. Psychiatry,* 2000, vol. 24, 979-991
- **H. NOGUCHI et al.** *Eur J Pharm,* 2002, vol. 434, 21-28
- **S. ARBILLA et al.** *Eur. J. Pharmacol.,* 1986, vol. 130, 257-263
- **Y. WU et al.** *Eur. J. Pharmacol.,* 1995, vol. 278, 125-132
- **D. J. SANGER et al.** *Eur. J. Pharmacol.,* 1996, vol. 313, 35-42
- **G. GRIEBEL et al.** *Psychopharmacology,* 1999, vol. 146, 205-213
- **KRALIC et al.** *Neuropharmacology,* 2002, vol. 43 (4), 685-689
- **BELELLI et al.** *Neuropharmacology,* 2003, vol. 45, 57-71